# EUROPEAN PATENT SPECIFICATION

(11) **EP 0 528 527 B1**
(45) Date of publication and mention of the grant of the patent: **08.04.1998**
(21) Application number: 92306149.3
(22) Date of filing: 03.07.1992
(51) Int. Cl.: A61K 47/48

(54) **Conjugates**
Konjugate
Conjuguées

(30) Priority: 03.07.1991 GB 9114399
(43) Date of publication of application: 24.02.1993
(73) Proprietor: ZENECA LIMITED, London W1Y 6LN (GB); Pharmacia & Upjohn Aktiebolag, 112 87 Stockholm (SE)
(72) Inventor: Wright, Andrew Firman, Macclesfield, Cheshire, SK10 4TG (GB); Blakey, David Charles, Macclesfield, Cheshire, SK10 4TG (GB); Fitton, John Edward, Macclesfield, Cheshire, SK10 4TG (GB); Lind, Peter, S-751 82 Uppsala (SE); Lindholm, Leif, S-430 41 Kullavik (SE); Holmgren, Jan, S-421 74 Vasta Frolunda (SE)
(74) Representative: Bill, Kevin

(56) References cited:
- EP-A- 0 031 999
- WO-A-91/07941
- WO-A-92/01470
- JOURNAL OF BIOLOGICAL RESPONSE MODIFIERS, vol. 7, 1988, NEW YORK, US, pages 559-567; GRIFFIN ET AL: 'IN VITRO CYTOTOXICITY OF RECOMBINANT RICIN A CHAIN- ANTITRANSFERRIN RECEPTOR IMMUNOTOXIN AGAINST HUMAN ADENOCARCINOMAS OF THE COLON AND PANCREAS'
- ANTI-CANCER DRUG DESIGN, vol. 1, 1986, BASINGSTOKE, UK, pages 179- 188; WORRELL ET AL: 'EFFECT OF LINKAGE VARIATION ON PHARMACOKINETICS OF RICIN A CHAIN-ANTIBODY CONJUGATES IN NORMAL RATS'
- EUROPEAN JOURNAL OF SURGICAL ONCOLGY, vol. 15, 1989, LONDON, UK, pages 367-370; HABIB ET AL: 'EVALUATION OF THE THERAPEUTIC USE OF RADIOACTIVE MONOCLONAL ANTIBODIES C242 AND C215 IN TRANSPLANTED MURINE TUMOURS'
- CLINICAL RESEARCH, vol. 40, no. 2, 1992, THOROFARE, N.J., US, page 211A; DEBINSKI ET AL: 'MONOCLONAL ANTIBODY C242.PSEUDOMONAS EXOTOXIN A:A SPECIFIC AND POTENT IMMUNOTOXIN WITH ANTITUMOR ACTIVITY ON A HUMAN COLON XENOGRAFT IN NUDE MICE'
- ANTICANCER RESEARCH, vol. 11, no. 6, November 1991, ATHENS, GR, pages 2003-2013; SHITARA ET AL: 'APPLICATION OF ANTI-SIALYL LEa MONOCLONAL ANTIBODY, KM231, FOR IMMUNOTHERAPY OF CANCER'
- CANCER RESEARCH, vol. 49, 1989, PHILADELPHIA, US, pages 613-617; RAMAKRISHNAN ET AL: 'RECOMBINANT RICIN A CHAIN CONJUGATED TO MONOCLONAL ANTIBODIES:IMPROVED TUMOR CELL INHIBITION IN THE PRESENCE OF LYSOSOMOTROPIC COMPOUNDS'
- INTERNATIONAL JOURNAL OF CANCER, vol. 47, January 1991, GENEVA, CH, pages 130-135; WAWRZYNCZAK ET AL: 'COMPARATIVE BIOCHEMICAL,CYTOTOXIC AND PHARMACOKINETIC PROPERTIES OF IMMUNOTOXINS MADE WITH NATIVE RICIN A CHAIN,RICIN A1 CHAIN AND RECOMBINANT RICIN A CHAIN'
- JOURNAL OF THE AMERICAN MEDICAL ASSOCIATION, vol. 259, no. 21, 1988, CHICAGO, US, pages 3154-3157; KERNAN ET AL: 'TREATMENT OF STEROID-RESISTANT ACUTE GRAFT-VS- HOST DISEASE BY IN VIVO ADMINISTRATION OF AN ANTI-T-CELL RICIN A CHAIN IMMUNOTOXIN'

SEQUENCE LISTING

(1) GENERAL INFORMATION

(i) APPLICANTS: Imperial Chemical Industries PLC and
Kabi Pharmacia AB
(ii) TITLE OF INVENTION: CONJUGATES
(iii) NUMBER OF SEQUENCES: 22
(iv) CORRESPONDENCE ADDRESS:
(A) ADDRESSEE: Legal Department: Patents
(B) STREET: Bessemer Road
(C) CITY: Welwyn Garden City
(D) STATE: Hertfordshire
(E) COUNTRY: United Kingdom
(F) ZIP: GB-AL7 1HD

(v) COMPUTER READABLE FORM:
(A) MEDIUM TYPE: Diskette, 3.50 inch, 1.2 Mb
storage
(B) COMPUTER: 18 M PS/2
(C) OPERATING SYSTEM: PC-DOS 3.20
(D) SOFTWARE: ASCII from WPS-PLUS

(vi) CURRENT APPLICATION DATA:
(A) APPLICATION NUMBER
(B) FILING DATE:
(C) CLASSIFICATION:

(vii) PRIOR APPLICATION DATA:
(A) APPLICATION NO. 9114399.0
(B) FILING DATE: 03 JUL 1991

(2) INFORMATION FOR SEQ ID NO:1:

(i) SEQUENCE CHARACTERISTICS:
(A) LENGTH: 16 Amino Acids
(B) TYPE: Amino Acid
(C) STRANDEDNESS: Single
(D) TOPOLOGY: Linear
(xi) SEQUENCE DESCRIPTION: SEQ ID NO:1:

Arg Ser Ser Lys Ser Leu Leu His Ser Asn Gly Asn Thr Tyr Leu Tyr 16

(2) INFORMATION FOR SEQ ID NO:2:

(i) SEQUENCE CHARACTERISTICS:
(A) LENGTH: 7 Amino Acids
(B) TYPE: Amino Acid
(C) STRANDEDNESS: Single
(D) TOPOLOGY: Linear
(xi) SEQUENCE DESCRIPTION: SEQ ID NO:2:

Arg Met Ser Asn Leu Val Ser 7

(2) INFORMATION FOR SEQ ID NO:3:

(i) SEQUENCE CHARACTERISTICS:
(A) LENGTH: 9 Amino Acids
(B) TYPE: Amino Acid
(C) STRANDEDNESS: Single
(D) TOPOLOGY: Linear
(xi) SEQUENCE DESCRIPTION: SEQ ID NO:3:

Leu Gln His Leu Glu Tyr Pro Phe Thr 9

(2)    INFORMATION FOR SEQ ID NO:4:

(i)  SEQUENCE CHARACTERISTICS:
(A) LENGTH: 5 Amino Acids
(B) TYPE: Amino Acid
(C) STRANDEDNESS: Single
(D) TOPOLOGY: Linear
(xi) SEQUENCE DESCRIPTION: SEQ ID NO:4:

Tyr Thr Gly Met Asn                                                    5

(2)    INFORMATION FOR SEQ ID NO:5:

(i)  SEQUENCE CHARACTERISTICS:
(A) LENGTH: 17 Amino Acids
(B) TYPE: Amino Acid
(C) STRANDEDNESS: Single
(D) TOPOLOGY: Linear
(xi) SEQUENCE DESCRIPTION: SEQ ID NO:5:

Trp Ile Asp Thr Thr Thr Gly Glu Pro Thr Tyr Ala Glu Asp Phe Lys        16

Gly                                                                    17

(2)    INFORMATION FOR SEQ ID NO:6:

(i)  SEQUENCE CHARACTERISTICS:
(A) LENGTH: 10 Amino Acids
(B) TYPE: Amino Acid
(C) STRANDEDNESS: Single
(D) TOPOLOGY: Linear
(xi) SEQUENCE DESCRIPTION: SEQ ID NO:6:

Arg Gly Pro Tyr Asn Trp Tyr Phe Asp Val                                10

(2)   INFORMATION FOR SEQ ID NO:7:

(i)  SEQUENCE CHARACTERISTICS:
(A) LENGTH: 18 Amino Acids
(B) TYPE: Amino Acid
(C) STRANDEDNESS: Single
(D) TOPOLOGY: Linear
(xi) SEQUENCE DESCRIPTION: SEQ ID NO:7:

Arg Ser Ser Lys Ser Leu Leu His Ser Asn Gly Asn Thr Tyr Leu Tyr      16

Trp Phe                                                              18

(2)   INFORMATION FOR SEQ ID NO:8:

(i)  SEQUENCE CHARACTERISTICS:
(A) LENGTH: 11 Amino Acids
(B) TYPE: Amino Acid
(C) STRANDEDNESS: Single
(D) TOPOLOGY: Linear
(xi) SEQUENCE DESCRIPTION: SEQ ID NO:8:

Ile Tyr Arg Met Ser Asn Leu Val Ser Gly Val                          11

(2) INFORMATION FOR SEQ ID NO:9:

(i) SEQUENCE CHARACTERISTICS:
(A) LENGTH: 11 Amino Acids
(B) TYPE: Amino Acid
(C) STRANDEDNESS: Single
(D) TOPOLOGY: Linear
(xi) SEQUENCE DESCRIPTION: SEQ ID NO:9:

Leu Gln His Leu Glu Tyr Pro Phe Thr Phe Gly 11

(2) INFORMATION FOR SEQ ID NO:10:

(i) SEQUENCE CHARACTERISTICS:
(A) LENGTH: 7 Amino Acids
(B) TYPE: Amino Acid
(C) STRANDEDNESS: Single
(D) TOPOLOGY: Linear
(xi) SEQUENCE DESCRIPTION: SEQ ID NO:10:

Phe Thr Tyr Thr Gly Met Asn 7

(2) INFORMATION FOR SEQ ID NO:11:

(i) SEQUENCE CHARACTERISTICS:
(A) LENGTH: 21 Amino Acids
(B) TYPE: Amino Acid
(C) STRANDEDNESS: Single
(D) TOPOLOGY: Linear
(xi) SEQUENCE DESCRIPTION: SEQ ID NO:11:

Met Gly Trp Ile Asp Thr Thr Thr Gly Glu Pro Thr Tyr Ala Glu Asp 16

Phe Lys Gly Arg Ile 21

(2) INFORMATION FOR SEQ ID NO:12:

(i) SEQUENCE CHARACTERISTICS:
(A) LENGTH: 14 Amino Acids
(B) TYPE: Amino Acid
(C) STRANDEDNESS: Single
(D) TOPOLOGY: Linear
(xi) SEQUENCE DESCRIPTION: SEQ ID NO:12:

Ala Arg Arg Gly Pro Tyr Asn Trp Tyr Phe Asp Val Trp Gly 14

(2) INFORMATION FOR SEQ ID NO:13:

(i) SEQUENCE CHARACTERISTICS:
(A) LENGTH: 25
(B) TYPE: Nucleic Acid
(C) STRANDEDNESS: Single
(D) TOPOLOGY: Linear
(xi) SEQUENCE DESCRIPTION: SEQ ID NO:13:

AATTCGCAT GCGGATCCAT CGATC 25

(2) INFORMATION FOR SEQ ID NO:14:

(i) SEQUENCE CHARACTERISTICS:
(A) LENGTH: 25
(B) TYPE: Nucleic Acid
(C) STRANDEDNESS: Single
(D) TOPOLOGY: Linear
(xi) SEQUENCE DESCRIPTION: SEQ ID NO:14:

GCGTACGCC TAGGTAGCTA GAGCC 25

(2)    INFORMATION FOR SEQ ID NO:15:

(i)   SEQUENCE CHARACTERISTICS:
(A) LENGTH: 21
(B) TYPE: Nucleic Acid
(C) STRANDEDNESS: Single
(D) TOPOLOGY: Linear
(xi) SEQUENCE DESCRIPTION: SEQ ID NO:15:

GATAACAACA TATTCCCCAA A                                                    21

(2)    INFORMATION FOR SEQ ID NO:16:

(i)   SEQUENCE CHARACTERISTICS:
(A) LENGTH: 23
(B) TYPE: Nucleic Acid
(C) STRANDEDNESS: Single
(D) TOPOLOGY: Linear
(xi) SEQUENCE DESCRIPTION: SEQ ID NO:16:

GATAACAAC ATGGTACCC AAA                                                    23

(2)    INFORMATION FOR SEQ ID NO:17:

(i)   SEQUENCE CHARACTERISTICS:
(A) LENGTH: 23
(B) TYPE: Nucleic Acid
(C) STRANDEDNESS: Single
(D) TOPOLOGY: Linear
(xi) SEQUENCE DESCRIPTION: SEQ ID NO:17:

AACAACATG GTACCCAAA CAA                                                    23

(2) INFORMATION FOR SEQ ID NO:18:

(i) SEQUENCE CHARACTERISTICS:
(A) LENGTH: 1140
(B) TYPE: Nucleic Acid
(C) STRANDEDNESS: Single
(D) TOPOLOGY: Linear
(xi) SEQUENCE DESCRIPTION: SEQ ID NO:18:

TTCTGGCAAA TATTCTGAAA TGAGCTGTTG ACAATTAATC ATCGAACTAG TTAACTAGTA 60

CGCAAGTTCA CGTAAAAAGG GTATCGACAA TGGTACCCGG GGATCCACCT CAGGGTGGTC 120

TTTCACATTA GAGGATAACA ACATGGTACC CAAACAATAC CCAATTATAA ACTTTACCAC 180

AGCGGGTGCC ACTGTGCAAA GCTACACAAA CTTTATCAGA GCTGTTCGCG GTCGTTTAAC 240

AACTGGAGCT GATGTGAGAC ATGAAATACC AGTGTTGCCA AACAGAGTTG GTTTGCCTAT 300

AAACCAACGG TTTATTTTAG TTGAACTCTC AAATCATGCA GAGCTTTCTG TTACATTAGC 360

CCTGGATGTC ACCAATGCAT ATGTGGTCGG CTACCGTGCT GGAAATAGCG CATATTTCTT 420

TCATCCTGAC AATCAGGAAG ATGCAGAAGC AATCACTCAT CTTTTCACTG ATGTTCAAAA 480

TCGATATACA TTCGCCTTTG GTGGTAATTA TGATAGACTT GAACAACTTG CTGGTAATCT 540

GAGAGAAAAT ATCGAGTTGG GAAATGGTCC ACTAGAGGAG GCTATCTCAG CGCTTTATTA 600

TTACAGTACT GGTGGCACTC AGCTTCCAAC TCTGGCTCGT TCCTTTATAA TTTGCATCCA 660

AATGATTTCA GAAGCAGCAA GATTCCAATA TATTGAGGGA GAAATGCGCA CGAGAATTAG 720

GTACAACCGG AGATCTGCAC CAGATCCTAG CGTAATTACA CTTGAGAATA GTTGGGGGAG 780

ACTTTCCACT GCAATTCAAG AGTCTAACCA AGGAGCCTTT GCTAGTCCAA TTCAACTGCA 840

AAGACGTAAT GGTTCCAAAT TCAGTGTGTA CGATGTGAGT ATATTAATCC CTATCATAGC 900

TCTCATGGTG TATAGATGCG CACCTCCACC ATCGTCACAG TTTTGATTGC TTATAAGGCC 960

AGTGGTACCC GGGGATCCTC TAGAGTCGAC CTGCAGGCAT GCAAGCTTAG CCCGCCTAAT 1020

GAGCGGGCTT TTTTTTATCG ACCGATGCCC TTGAGAGCCT TCAACCCAGT CAGCTCCTTC 1080

CGGTGGGCGC GGGGCATGAC TATCGTCGCC GCACTTATGA CTGTCTTCTT TATCATGCAA 1140

(2) INFORMATION FOR SEQ ID NO:19:

(i) SEQUENCE CHARACTERISTICS:
(A) LENGTH: 26
(B) TYPE: Nucleic Acid
(C) STRANDEDNESS: Single
(D) TOPOLOGY: Linear
(xi) SEQUENCE DESCRIPTION: SEQ ID NO:19:

ATAACAACAT GGTTCCCAAA CAATAC 26

(2) INFORMATION FOR SEQ ID NO:20:

(i) SEQUENCE CHARACTERISTICS:
(A) LENGTH: 86
(B) TYPE: Nucleic Acid
(C) STRANDEDNESS: Single
(D) TOPOLOGY: Linear
(xi) SEQUENCE DESCRIPTION: SEQ ID NO:20:

AAAAAGGGTA TCGACATGGT ACCCGGGGAT CCACCTCAGG GTGGTCTTTC ACATTAGAGG 60

ATAACAACAT GGTACCCAAA CAATAC 86

(2) INFORMATION FOR SEQ ID NO:21:

(i) SEQUENCE CHARACTERISTICS:
(A) LENGTH: 463
(B) TYPE: Nucleic Acid
(C) STRANDEDNESS: Single
(D) TOPOLOGY: Linear
(xi) SEQUENCE DESCRIPTION: SEQ ID NO:21:

GGAATTCGGC ACGAGGAGTT TTTTTGTATC AAGTTCTCAGA ATG AGG TGC CTA GCT 55
Met Arg Cys Leu Ala

GAG TTC CTG GGG CTG CTT GTG CTC TGG ATC CCT GGA GCC ATT GGG GAT 103
Glu Phe Leu Gly Leu Leu Val Leu Trp Ile Pro Gly Ala Ile Gly Asp

ATT GTG ATG ACT CAG GCT GCA CCC TCT GTA CCT GTC ACT CCT GGA GAG 151
Ile Val Met Thr Gln Ala Ala Pro Ser Val Pro Val Thr Pro Gly Glu

TCA GTA TCC ATC TCC TGC AGG TCT AGT AAG AGT CTC CTG CAT AGT AAT 199
Ser Val Ser Ile Ser Cys Arg Ser Ser Lys Ser Leu Leu His Ser Asn

GGC AAC ACT TAC TTG TAT TGG TTC CTG CAG AGG CCA GGC CAG TCT CCT 247
Gly Asn Thr Tyr Leu Tyr Trp Phe Leu Gln Arg Pro Gly Gln Ser Pro

CAG CTC CTG ATA TAT CGG ATG TCC AAC CTT GTC TCA GGA GTC CCA GAC 295
Gln Leu Leu Ile Tyr Arg Met Ser Asn Leu Val Ser Gly Val Pro Asp

AGG TTC AGT GGC AGT GGG TCA GGA ACT GCT TTC ACA CTG AGA ATC AGT 343
Arg Phe Ser Gly Ser Gly Ser Gly Thr Ala Phe Thr Leu Arg Ile Ser

AGA GTG GAG GCT GAG GAT GTG GGT GTT TAT TAC TGT CTG CAA CAT CTA 391
Arg Val Glu Ala Glu Asp Val Gly Val Tyr Tyr Cys Leu Gln His Leu

GAG TAT CCG TTC ACG TTC GGT CCT GGG ACC AAG CTG GAG CTG AAA CGG 439
Glu Tyr Pro Phe Thr Phe Gly Pro Gly Thr Lys Leu Glu Leu Lys Arg

GCT GAT GCT GCA CCA ACT GTA ACG 463
Ala Asp Ala Ala Pro Thr Val Thr

(2) INFORMATION FOR SEQ ID NO:22:

(i) SEQUENCE CHARACTERISTICS:
(A) LENGTH: 483
(B) TYPE: Nucleic Acid
(C) STRANDEDNESS: Single
(D) TOPOLOGY: Linear
(xi) SEQUENCE DESCRIPTION: SEQ ID NO:22:

GAATTCGGCA CGAGATTGAG CCCAAGTCTT AGACATC ATG GAT TGG CTG CGG 52
Met Asp Trp Leu Arg

AAC TTG CTA TTC CTG ATG GCA GCT GCC CAA AGT ATC CAA GCA CAG GTC 100
Asn Leu Leu Phe Leu Met Ala Ala Ala Gln Ser Ile Gln Ala Gln Val

CAG TTG GTG CAG TCT GGA CCT GAG CTG AAG AAG CCT GGA GAG ACA GTC 148
Gln Leu Val Gln Ser Gly Pro Glu Leu Lys Lys Pro Gly Glu Thr Val

AAG ATC TCC TGC AAG GCT TCT GAT TAT ACC TTC ACA TAC TAT GGA ATG 196
Lys Ile Ser Cys Lys Ala Ser Asp Tyr Thr Phe Thr Tyr Tyr Gly Met

AAC TGG GTG AAG CAG GCT CCG GGA AAG GGT TTA AAG TGG ATG GGC TGG 244
Asn Trp Val Lys Gln Ala Pro Gly Lys Gly Leu Lys Trp Met Gly Trp

ATA GAC ACC ACC ACT GGA GAG CCA ACA TAT GCT GAA GAT TTT AAG GGA 292
Ile Asp Thr Thr Thr Gly Glu Pro Thr Tyr Ala Glu Asp Phe Lys Gly

CGG ATT GCC TTC TCT TTG GAG ACC TCT GCC AGC ACT GCC TAT TTG CAG 340
Arg Ile Ala Phe Ser Leu Glu Thr Ser Ala Ser Thr Ala Tyr Leu Gln

ATC AAA AAC CTC AAA AAT GAG GAC ACG GCT ACA TAT TTC TGT GCA AGA 388
Ile Lys Asn Leu Lys Asn Glu Asp Thr Ala Thr Tyr Phe Cys Ala Arg

CGG GGG CCT TAC AAC TGG TAC TTT GAT GTC TGG GGC GCA GGG ACC ACG 436
Arg Gly Pro Tyr Asn Trp Tyr Phe Asp Val Trp Gly Ala Gly Thr Thr

GTC ACC GTC TCC TCA GCC AAA ACG ACN CCC CCA TCT GTC TAT CC 483
Val Thr Val Ser Ser Ala Lys Thr Thr Pro Pro Ser Val Tyr Pro

## Description

This invention relates to conjugates such as immunotoxins for use in anti-tumour therapy, processes for preparing such conjugates and pharmaceutical compositions containing them.

Ricin and ricin-type molecules, such as abrin, modecin and viscumin are known compounds which are produced by plant cells, and which possess cytotoxic properties. Toxins of this type consist of two polypeptide chains which are linked via a disulphide bridge. One of the polypeptide chains (the "A chain") is primarily responsible for the cytotoxic properties of the toxin molecule; whilst the other polypeptide chain (the "B chain") enables the toxin molecule to bind to cell surfaces.

The toxicity of ricin-type molecules operates in three phases:-
(a) binding of the toxin to the cell surface through interaction of galactose binding sites on the B chain with glycoproteins or glyolipids exposed at the cell surface;
(b) penetration of at least the A chain into the cytosol of the cell; and
(c) inhibition of protein synthesis through the A chain destroying the activity of ribosomal 60S subunits.

It is also believed that the B chain has an important secondary function, apart from its primary function of binding the toxin molecule to the cell surface, in that it facilitates uptake of the toxin into the cell. Thus, separated A and B chains are essentially non-toxic since the A chain lacks the ability to bind to cell surfaces and penetrate into the cytosol of the cell in the absence of the B chain; whilst the B chain does not possess cytotoxic properties.

As mentioned above ricin and ricin-type toxin molecules are produced by plants. In the case of ricin itself, production occurs in the Ricinus communis otherwise known as the castor oil plant. It is thought that a precursor polypeptide (known as preproricin) which comprises a leader sequence, the A chain, a connecting sequence and the B chain is produced first. During transport of this precursor (preproricin) within the plant cell, the leader sequence is thought to be eliminated to yield proricin. Mature ricin is then produced from proricin by formation of a disulphide bridge between the A and B chains and elimination of the connecting sequence.

It has already been suggested that the toxicity of the A chain of toxins such as ricin might be useful in anti-tumour therapy if the indiscriminately-binding B chain could be replaced by a different carrier which has the ability to bind to tumour cells in preference to normal cells. Thus various conjugates have been suggested and prepared which contain mature ricin or the A chain of ricin and a tumour-specific antibody. However, such conjugates, immunoconjugates or immunotoxins, possess a number of disadvantages.

One problem with known conjugates arises from a structural feature of the A chain of natural ricin. It is believed that during the synthesis of ricin in plant cells, N-glycosylation takes place and that the resulting sugar moieties are capable of non-specific interactions with cell surfaces.

Loss of selectivity with plant ricin-immunoconjugates is also thought to occur as a result of either:-
(a) the galactose binding sites on the B chain interacting in a non-specific manner with cell surfaces in the case of whole ricin-immunoconjugates; or
(b) through binding of the glycan side side chains expressed on plant ricin A with galactose and mannose receptors on cell surfaces, in the case of plant ricin A-immunoconjugates.

A further problem is that, as mentioned above, the B chain is believed to facilitate uptake of the toxin molecule into the cell. Thus, although conjugates in which the B chain is absent, that is those consisting of A chain and an antibody, tend to be more specific than native toxins they are reported to lack the potency of the native toxin (Moolten, F.L, et al, Immun Rev, 62, 47-72, 1982). This loss of potency is believed to be due to a decrease in the effective uptake of the toxin into the cell. Thus relatively few of the antibodies which have been reported facilitate effective internalisation of the toxin, and so give rise to immunotoxins which lack potency.

Thus far, conjugates which are of potential use in treating tumours in a number of different tissues have been prepared. For example, PCT Patent Application No WO85/003508 describes the preparation of conjugates which comprise ricin A or diphtheria toxin A chain linked via a spacer molecule to antibodies specific for breast tumours.

Antibodies which recognise colorectal tumour cells have also been reported, but conjugates which include these antibodies tend to suffer from unacceptable binding to normal tissue and/or low potency. Thus known antibodies which recognise colorectal tumour cells have not found utility in the preparation of conjugates of real therapeutic interest. There is thus a need for a conjugate which is of therapeutic value in treating colorectal tumours.

Thus, although various conjugates have been suggested and prepared, there is a need for improved conjugates. Such improved conjugates may ameliorate one or more of the disadvantages associated with known conjugates. There is also a need for conjugates which are selective for gastrointestinal tumours.

According to the present invention there is provided a conjugate which comprises a toxin moiety and a target cell binding moiety which is selective for gastrointestinal tumours and wherein the target cell binding moiety comprises C242 antibody or a target cell binding moiety which has substantially the same cell binding properties.

In particular, the target cell binding moiety (and hence the conjugate) is selective for colorectal and pancreatic tumour cells, more particularly colorectal tumour cells.

The target cell binding moiety will, for example, in general comprise an antibody, an antibody fragment or a derivative of an antibody or antibody fragment.

The target cell binding moiety is selective for gastrointestinal tumour cells (especially colorectal tumour cells) in that it binds to such cells in preference to normal cells. Thus the target cell binding moiety will bind to gastrointestinal tumour cells, but will show no or only minimal binding affinity towards normal cells. The target cell binding moiety will exhibit a selectivity towards gastrointestinal tumours substantially the same as that displayed by C242 antibody. The selectivity of C242 antiboty towards gastrointestinal tumours, such as colorectal tumours, is exemplified by the immunohistochemical data given below. Thus a target cell binding moiety, such as C242 antibody, will bind to an antigen associated with gastrointestinal tumours but which is absent or only weakly expressed in normal cells.

As mentioned herein, the conjugate is capable of killing gastrointestinal tumour cells. Thus the conjugate (or "immunotoxin") is able to deliver the toxin moiety to the tumour cell such that the toxin moiety may exert its cytotoxic properties. The conjugate will therefore, in general, be able to bind to the tumour cells (through the target cell binding moiety binding to the tumour cells) and allow the toxin moiety to pass into the cell, that is allow the toxin moiety to "internalise". Particularly effective conjugates will, in general, have the following properties:-
1. The target cell binding moiety should be capable of binding to a tumour cell surface antigen.
2. The cell surface antigen should be present in high copy number on tumour cells, for example, at least ten thousand per cell.
3. The antigen should not be expressed in high copy number on normal cells.
4. The antibody:antigen complex should be internalised efficiently such that the toxin moiety can exert its cytotoxicity intracellularly.
5. The conjugate should preferably be constructed such that it is sufficiently stable to remain intact in the blood for long enough to deliver the toxin moiety to the tumour cells as well as being sufficiently cleavable to allow the release of the toxin moiety once the toxin moety is inside the cell.

The antibody C242 is a murine antibody of the IgG class which is produced when culturing in an appropriate medium a hybridoma cell line obtained by fusing spleen cells from a mouse, which has been immunised with a human colonic adenocarcinoma cell line, with the murine myeloma cell line Sp 2/0. A hybridoma cell line (242.II) which produces the C242:II antibody (also referred to herein simply on C242 antibody) was deposited on January 26, 1990 in accordance with the Budapest Treaty at the European Collection of Animal Cell Cultures (ECACC), PHLS Centre for Applied Microbiology and Research, Porton Down, Salisbury, Wiltshire, United Kingdom, under depository accession no. 90012601.

The term "a target cell binding moiety which has substantially the same cell binding properties" includes target cell binding moieties which have substantially the same immunological specificity as that produced by the deposited ECACC cell line 90012601, that is bind to the same antigenic determinant or the epitope, and compete with the C242 antibody for binding at that site.

The expression "target cell binding moiety" also includes antibody fragments, and in particular, fragments of the antibody C242 as well as intact antibody. Antibody fragments will retain the binding capability and specificity of the unfragmented immunoglobulin and, include, for example those obtained by degrading the antibody with a proteolytic enzyme, such as pepsin. Particular examples of fragments include F(ab′) and F(ab′)₂ fragments.

It will be appreciated that the target cell binding moiety may be prepared by genetic engineering and accordingly the term target cell binding fragment inculdes antibodies and antibody fragments generated by such techniques. In particular the term target cell binding moiety includes C242 antibody or a fragment thereof which is the product of genetic engineering.

The preparation of antibody fragments and in particular humanised antibody fragments is described, for example, by Carter et al, Biotechnology, Vol. 10, February 1992.

The target cell binding moiety includes derivatised forms of antibodies or antibody fragments, and in particular humanised forms of antibodies or antibody fragments derived from non-human sources, for example humanised forms of murine antibodies.

The antibody C242 is directed against a tumour associated antigen, designated herein as CA-242. Thus, in particular the target cell binding moiety includes an antibody or antibody fragment which is capable of binding to the antigen CA-242. A specific example of such an antibody is the antibody C242 itself. The tumour-associated antigen CA-242 is expressed in the majority of colo-rectal tumours, and is only weakly expressed or is absent in normal colonic tissue.

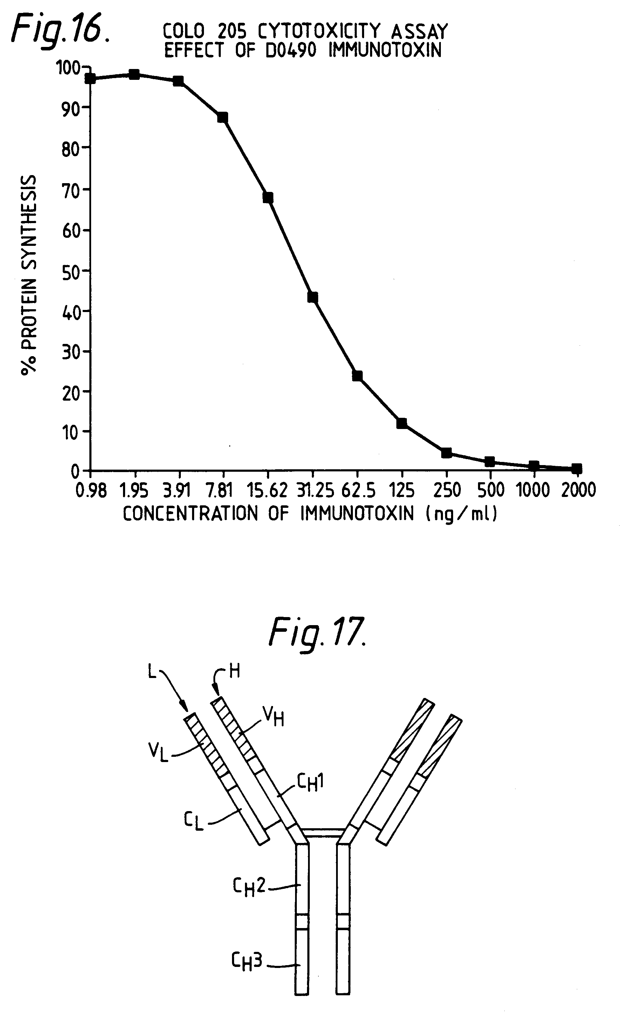

With respect to genetic engineering as mentioned above, the cDNA's of the variable regions of the light and heavy chains of the C242:II monoclonal antibody were cloned as will be described below. Before discussing this in any more detail it may not be out of place to give a brief general description of the basic immunoglobulin structural unit, reference being made to Fig. 17 of the accompanyings drawings which describes the general structure of an antibody, i.e. immunoglobulin, or class G.

Referring to Fig. 17, the immunoglobulin consists of two identical light (L) polypeptide chains and two identical heavy polypeptide chains (H), the four chains being joined by disulphide bonds and various non-covalent forces in a symmetric "Y"-configuration. Each heavy chain has a variable domain (V_{H}) at each end followed by a number of constant domains (C_{H}), and each light chain has a variable domain (V_{L}) at one end and a constant domain (C_{L}) at the other end. There are two types of light chains, designated kappa and lambda, respectively. The variable domain of the light chain is aligned with the variable domain of the heavy chain, and the costant domain of the light chain is aligned with the first constant domain of the heavy chain, the variable domains of the light and heavy chains forming the antigen binding site.

The variable domains of the light and heavy chains have the same general structure, each comprising three hypervariable regions, called complementarity determining regions, or CDR's, intervening between four framework regions, or FR's. The CDR's of each variable domain are kept in close proximity by the framework regions, and the two sets of CDR's together provide for the specificity of the antibody.

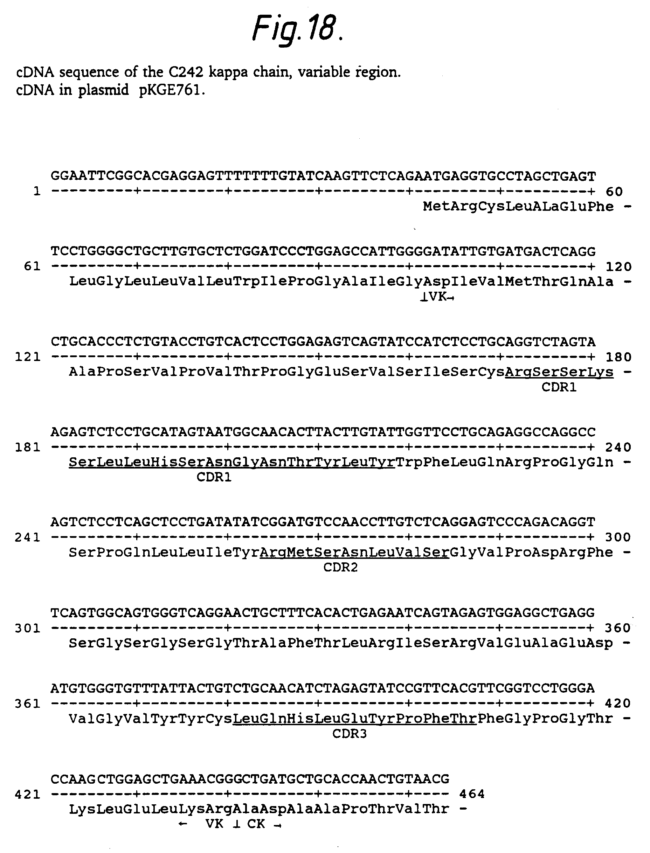

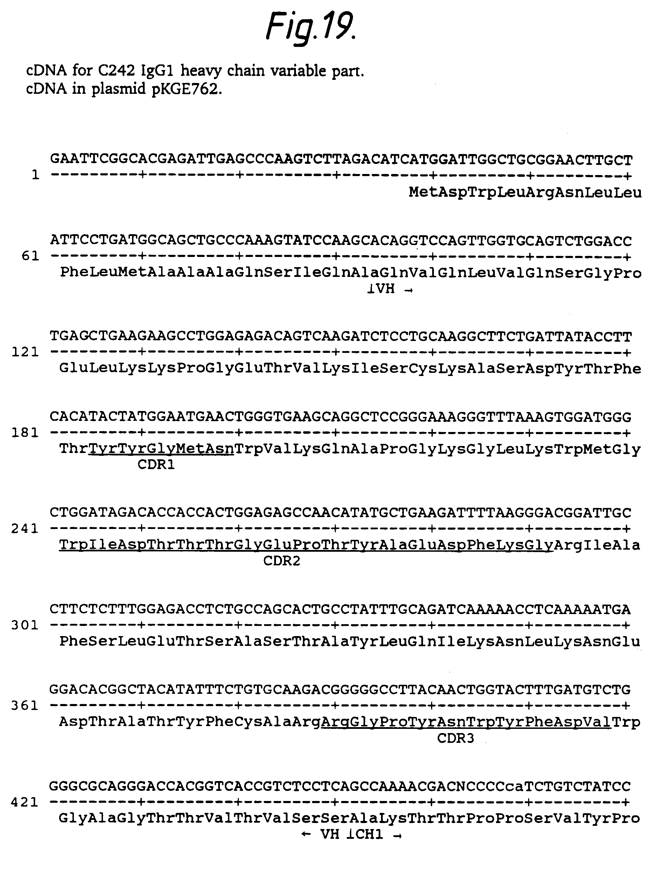

In order to clone the cDNA's of the variable portions of the light and heavy chains of the C242:II antibody, a cDNA phage library was first prepared from the C242:II hybridoma by per se known methods. Hybridization probes covering constant parts of the heavy chain and the light chain (kappa), respectively, were then prepared from mouse genomic DNA using the polymerase chain reaction (PCR) and suitable primers. The resulting probes were used to screen the cDNA library for cDNA clones containing DNA sequences encoding the heavy and kappa chains of the C242:II antibody. Positively hybridizing phage clones were expanded and the cDNA was excised in the form of plasmids. The latter were characterized by restriction enzyme mapping, and plasmids containing inserts of the expected sizes were sequenced. In order to determine the CDR regions, the amino acid sequences amino acid sequences encoded by the sequences inserts were compared with those of previously known mouse kappa and heavy chains, considering the basic locations of CDR regins as defined by, e.g., Kabat E.A., et al. (1987), Sequence of Proteins of Immunological Interest, 4th Ed., U.S. Department of Health and Human Services, National Institutes of Health. It was found that the CDR's of the respective chains had the following amino acid sequences (as are also shown in Fig. 18 and 19 of the accompanying drawings - see also SEQ. ID. No's 21 and 22):

### Light chain

CDR1 (SEQ. ID. NO 1): ArgSerSerLysSerLeuLeuHisSerAsnGlyAsnThrTyrLeuTyr
CDR2 (SEQ. ID. NO 2): ArgMetSerAsnLeuValSer
CDR3 (SEQ. ID. NO 3): LeuGlnHisLeuGluTyrProPheThr

### Heavy Chain

CDR1 (SEQ. ID. NO 4): TyrThrGlyMetAsn
CDR2 (SEQ. ID. NO 5): TrpIleAspThrThrThrGlyGluProThrTyrAlaGluAspPheLysGly
CDR3 (SEQ. ID. NO 6): ArgGlyProTyrAsnTrpTyrPheAspVal

With the knowledge of these CDR amino acid and/or DNA sequences the skilled person may introduce the CDR regions of the C242:II antibody into the cloned variable parts of another antibody or antibody fragment by means of site-directed mutagenesis as is per se known in the art. Such procedure, generally known as "CDR grafting" (described, for example, in published European Patent Application Number, EP 239,400), involves substitution at DNA level of the CDR encoding sequences of the C242:II antibody for the CDR encoding sequences of the recipient antibody or fragment and will result in an antibody or antibody fragment having a corresponding specificity as the C242:II monoclonal antibody. Included within the scope of the target cell binding moiety is therefore, of course, any such moiety which comprises an antibody or antibody fragment having at least one of the above defined CDR's (or CDR's substantially as defined above), the number of CDR's and their arrangement on the antibody or antibody fragment being such that the target cell binding moiety has substantially the same cell binding properties as C242 antibody. It is generally preferred that the target cell binding moiety includes all of the CDR's (that is CDR1, CDR2 and CDR3 of the light chain and CDR1, 2 and 3 of the heavy chain) defined above (or substantially as defined above). CDR's substantially as defined above included extended sequences such as:

### Light chain

CDR1 (SEQ. ID. NO 7):
ArgSerSerLysSerLeuLeuHisSerAsnGlyAsnThrTyrLeuTyrTrpPhe
CDR2 (SEQ. ID. NO 8): IleTyrArgMetSerAsnLeuValSerGly Val
CDR3 (SEQ. ID. NO 9): LeuGlnHisLeuGluTyrProPheThrPheGly

### Heavy Chain

CDR1 (SEQ. ID. NO 10): PheThrTyrThrGlyMetAsn
CDR2 (SEQ. ID. NO 11):
MetGlyTrpIleAspThrThrThrGlyGluProThrTyrAlaGluAspPheLysGlyArgIle
CDR3 (SEQ. ID. NO 12): AlaArgArgGlyProTyrAsnTrpTyrPheAspValTrpGly

Antibodies or antibody fragments prepared by CDR grafting will preferably be prepared by grafting the C242 CDR's onto a framework which is selected to be close to C242 in both homology and CDR size.

It will be appreciated that the conjugate of the present invention does not necessarily consist of one toxin molecule and one antibody molecule. For example the conjugate may comprise more than one toxin molecule per antibody molecule.

The toxin moiety generally comprises a component which possesses cytotoxic properties and hence is capable of killing cells following internalisation.

The target cell binding moiety generally comprises a target cell binding moiety which is capable of recognising a specific antigenic determinant or target cell.

The toxin moiety and the target cell binding moiety may be coupled directly to one another, or they may be coupled indirectly. The toxin moiety and the target cell binding moiety are, in general, coupled such that the geometry of the conjugate permits the target cell binding moiety to bind to its target cell. Advantageously, the toxin moiety and the target cell binding moiety are coupled such that the conjugate is extracellularly stable, and intracellularly unstable so that the toxin moiety and the target cell binding moiety remain coupled outside the target cell, but following internalisation, the toxin moiety is released. Thus, advantageously the conjugate has an intracellularly cleavable/extracellularly stable site.

Examples of conjugates in which the toxin moiety is directly coupled to the target cell binding moiety include those in which the toxin moiety and the target cell binding moiety are coupled by a disulphide bridge formed between a thiol group on the toxin moiety and a thiol group on the target cell binding moiety.

The target cell binding moiety may include a portion which recognises the toxin moiety and which hence couples the toxin moiety and target cell binding moiety. An example of the latter is where the target cell binding moiety includes an antibody or antibody fragment which binds to the toxin moiety.

The target cell binding moiety and the toxin moiety may comprise a single polypeptide, which preferably includes an intracellularly cleavable site so that the toxin moiety is released in the cell. In such a polypeptide, the toxin and target cell binding moieties may be connected by way of a polypeptide linker.

Examples of conjugates in which the toxin moiety is indirectly coupled to the target cell binding moiety include those in which the toxin moiety is coupled to the target cell binding moiety through a bifunctional linker, especially a heterobi-functional linker.

Suitable linkers include, for example, linkers which comprise a polypeptide, such as those described in PCT Patent Application WO 85/003508 and linkers which comprise chemical moieties such as those described in Published European Patent Application No. 169,111.

Conveniently, the toxin moiety and the target cell binding moiety are coupled by way of a disulphide bond, or a thioether bond. It is, in general, preferred that the toxin moiety and target cell binding moeity are coupled by a linker which couples said moieties by way of an intracellularly cleavable disulphide or thioether bond. Examples of such linkers and their use in preparing immunotoxins are described in Published European Patent Application No 169,111; in Methods in Enzymology 112, 207-225, 1985, (Cumber, A. J., Forrester, J. A., Foxweel, B. M. J., Ross, W. C. J., Thorpe, P. E. - Preparation of antibody-toxin conjugates); and in Vogel, Immunoconjugates: Antibody conjugates in radioimaging and therapy of cancer, pp 28-55, (Wawrzynczak, E. J. and Thorpe, P. E. - Methods for preparing immunotoxins: effects of the linkage on activity and stability).

Particular linkers include those which are able to form a disulphide bond with a thiol group on one of the toxin moiety and the target cell binding moiety and an amide bond with an amino group on the other of the toxin moiety and the target cell binding moiety.

Examples of particular linkers are those of formula:

-S-X-C0-

wherein X is a spacer group.

X may comprise a straight-chain alkyl group, optionally substituted by one or two substituents selected from (1-6C)alkyl, phenyl and (1-4C)alkylphenyl; or an (1-4C)alkylphenyl group, in which the alkyl moiety is optionally substituted by one or two substituents selcted from (1-6C)alkyl, phenyl and (1-4C)alkylphenyl; and wherein the phenyl ring may bear a substituent selected from, for example, halogen, (1-4C)alkyl and (1-4C)alkoxy.

It is generally preferred, for example, that in X the alkyl group bears a substituent selected from those mentioned above.

For example, X may comprise a (1-6C)alkyl chain, optionally substituted by one or two substituents selected from (1-4C)alkyl and phenyl. Particular values of X include, for example, a methylene, ethylene, propylene, butylene group optionally substituted by one or two groups independenly selected from methyl, ethyl, propyl and butyl (especially when substituted by methyl, ethyl, propyl and butyl).

Specific values of X of interest are those of in which X comprises a methylene or ethylene group optionally substituted by one or two groups as defined above.

Preferably X comprises an ethylene group which is unsubstituted or substituted by one or two methyl groups, especially an ethylene group substituted by one methyl group.

Preferred conjugates of the present invention include those of the formula: 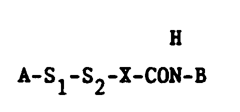
- wherein: A comprises one of the toxin moiety and the target cell binding moiety, and B comprises the other of the toxin moiety and the target cell binding moiety; the target cell binding moiety may carry one or more toxin moieties;
S₁ is a sulphur atom present on A;
NH is on B; and
S₂-X-CO is the linker, in which S₂ is a sulphur atom and X is as defined above.

It will be appreciated that S, is derived from a thiol group on A.

It will be appreciated that when the linker contains a chiral centre, it may exist in and be isolated in, optically active or racemic form. The invention encompases the use of any optically active or racemic form of the linker. The synthesis of optically active forms may be carried out by standard techniques of organic chemistry well known in the art.

Preferably A comprises the toxin moiety and B comprises the target cell binding moiety.

As stated above, the toxin moiety may comprise any component which possesses cytotoxic properties. Examples of such components include polypeptides which are capable of inactivating ribosomes and hence inhibiting protein synthesis. Particular examples include polypeptides which are found in nature and components of such polypeptides. Where such a polypeptide comprise a toxic component and an essentially non-toxic component, the toxin moiety conveniently comprises the toxic component. For example the toxin moiety may comprise ricin, but conveniently the toxin moiety comprises the A-chain of ricin. The toxin moiety may also comprise portions or analogues of polypeptides found in nature, which possess cytotoxic properties. Suitable polypeptides for the toxin moiety and their preparation are described in "Ribosome inactivating proteins up to date" - Stripe F and Barbieri. L, FEBS 3269 and the references therein. Particularly suitable polypeptides for the toxin moiety include the A-chain of ricin (ricin A), restrictocin and shiga toxin. Other suitable polypeptides for the toxin moiety include abrin, viscumin, modeccin, volkensin, gelonin, pokeweed antiviral protein, saporin, luffin, trichosanthin, barley ribosome inactivating protein, dianthins, byodin, momordin, tritin, dodecandrin, α-sarcin, mitogellin, diphtheria toxin, pseudomonas exotoxin A and the "shiga-like" toxins, VT1 and VT2.

Analogues of polypeptides found in nature include those polypeptides which have a primary structure which is related to the polypeptide found in nature in that it differs by one or more amino acid alterations (deletions, additions, substitutions) which do not result in loss of cytotoxic activity. Particular examples of such analogues include those in which the alteration or alterations facilitate coupling to the target cell binding moiety.

Where the toxin moiety comprises a polypeptide found in nature or a portion thereof the toxin moiety may be prepared by isolating the polypeptide from a natural source and where a portion is required, modifying the isolated polypeptide by chemical means, for example enzymatically. The polypeptide or portion thereof may also be prepared by genetic engineering techniques. Such techniques include those of recombinant DNA technology where a DNA sequence coding for the desired polypeptide is usually incorporated into a suitable vector or plasmid. Host cells transformed with the vector or plasmid may be cultured under appropriate conditions to obtain expression of the DNA sequence and production of the polypeptide.

It is preferred that the toxin moiety comprises ricin A, a portion or an analogue thereof. Where the toxin moiety comprises ricin A, the ricin A may be obtained from the seeds of the Ricinis Communis or the "castor bean" plant. However, it is preferred that the A chain or ricin is prepared by genetic engineering techniques. Thus recombinant ricin A or ricin is preferred.

Ricin A obtained by means of recombinant DNA technology is generally preferred to that obtained from natural sources since recombinant DNA technology permits the preparation of ricin A which is not contaminated with the B-chain of ricin, and since ricin A prepared in this way is not glycosylated. Thus the use of recombinant ricin A tends to lead to a more selective conjugate due to the absence of the indiscrimately-binding B-chain and to the absence of sugar moieties which are capable of non-specific interaction with cell surfaces.

In a preferred embodiment a conjugate of the present invention comprises a target cell binding moiety which is selective for colorectal tumour cells.

The target cell binding moiety preferably comprises C242 antibody or a fragment thereof, especially C242 antibody.

The toxin moiety and target cell binding moiety are preferably coupled through the 3-mercaptobutyric radical, -C0.CH₂.CH(CH₃)-S-. This radical is conveniently linked to the target cell binding moiety by a covalent bond between an NH group on the target cell binding moiety, for example the NH of a lysyl residue, and a disulphide bridge with a thiol group on the toxin moiety, for example the thiol group on the cysteine residue in ricin A.

Thus, in particular, the present invention provides an immunotoxin which comprises C242 antibody and recombinant ricin A, linked from an amino residue on C242 to a thiol of cysteine residue in ricin A by the 3-mercaptobutyric radical, -COCH₂CH(CH₃)-S-.

A preferred immunotoxin is one which comprises C242 antibody and recombinant ricin A, linked from the terminal amino group of a lysyl residue in C242 to the terminal thiol of a cysteine residue in ricin A by a 3-mercaptobutyric acid diradical.

Each antibody unit will, in general, bear at least one ricin A unit. It will be appreciated that an antibody unit may carry more than one recombinant ricin A moiety via respective linker moieties. It will be appreciated that the antibody may carry one or more linker moieties in addition to those linked to ricin A. These additional linker moieties may bear a non-toxic molecule, for example, cysteine. Such linkers may therefore be "capped" by the non-toxic molecule.

The immunotoxins of the present invention are of potential use in treating gastrointestinal tumours, and in particular colorectal or pancreatic tumours. Thus according to the present invention there is also provided a method of treating gastrointestinal tumours, comprising administering an effective amount of a conjugate (as defined herein) to a warm-blooded mammal, such as man.

It will be appreciated that the dose and dosage regimen will depend upon the particular toxin moiety employed, the population of the target cell and the patient's history. The dose of the conjugate administered will typically be in the range 0.1 to 1mg/kg of patient weight.

The conjugates of the present invention will normally be administered in the form of a pharmaceutical composition. Thus according to the present invention there is also provided a pharmaceutical compostion which comprises a conjugate (as defined herein) in association with a pharmaceutically-acceptable diluent or carrier.

Pharmaceutical compositions of the present invention may be formulated in a variety of dosage forms. Generally, the conjugates of the present invention will be administered parenterally, preferably intravenously. A particular parenteral pharmaceutical composition is one which is formulated in a unit dosage form which is suitable for administration by injection. Thus, particularly suitable compositions comprise a solution, emulsion or suspension of the immunotoxin in association with a pharmaceutically acceptable parenteral carrier or diluent. Suitable carriers or diluents include aqueous vehicles, for example water or saline, and non-aqueous vehicles, for example fixed oils or liposomes. The compositions may include agents which enhance the stability of the conjugate in the composition. For example, the composition may include a buffer, for example Tween. The concentration of the conjugate will vary, but in general, the conjugate will be formulated at concentrations of about 1 to 10 mg/dose.

The antibody C242 is selective for colorectal tumour cells and conjugates incorporating this antibody have been found to be potent immunotoxins which are selective for colorectal tumour cells.

In particular, the preferred conjugate of the present invention has been found to be a surprisingly efficient immunotoxin in that it is both selective for colorectal tumour cells and potent. This conjugate has also been found to have a relatively low rate of blood clearance and extracellular cleavage and destruction, leading to improved plasma persistence. This has the potential advantage of allowing the immunotoxin sufficient time to interact with its target cells before clearance or destruction takes place.

The present invention also provides processes for the preparation of an conjugate as defined above:-
(a) For those conjugates in which the target cell binding moiety is directly coupled to the toxin moiety, reacting the toxin moiety with the target cell binding moiety.

For example, where the target cell binding moiety is coupled to the toxin moiety through a disulphide bridge, one of the target cell binding moiety and toxin moiety may be reduced using, for example, dithiothreitol before reaction with the other of the target binding moiety and the toxin moiety.

For those conjugates in which the target cell binding moiety is coupled to the toxin moiety by a linker, reacting target cell binding moiety derived with linker with the toxin moiety, or reacting toxin moiety derivatised with linker with the target cell binding moiety.

The appropriate moiety is derivatised by reaction with a linking agent so as to bind the linker to that moiety. Conveniently, derivatised target cell binding moiety is reacted with the toxin moiety.

The derivatised target cell binding moiety or derivated toxin moiety may be prepared, for example, by dissolving that moiety in an appropriate buffer (such as an acetate-, phosphate- or borate buffer) and adjusting the pH to a value which is compatible with the linking agent. The linking agent may then be added to the reaction mixture. In cases where the linking agent is insoluble in the reaction mixture, a solution of the target cell binding moiety or toxin moiety as appropriate may be added to a solution of the lining agent in a small amount of an organic solvent such as dimethylsulphoxide or dimethylformamide. Following reaction with the linking agent the derivatised product may be separated by standard techniques such as gel filtration, dialysis or column chromatography. The derivatised product (derivated target cell binding moiety or derivatised toxin moiety) may then be reacted with the other moiety to produce the final conjugate.

It will be approached that the exact nature of the linking agent employed will be dependent on the nature of the groups (for example, thiol, carboxy or amino) on the target cell binding moiety and toxin moiety which are available forconjugation.

The toxin moiety may be prepared from natural sources. It is preferred that the toxin moiety is prepared by recombinant DNA technology. This technology uses techniques well known to those skilled in the art and includes techniques for extracting mRNA, preparing cDNA libaries, constructing vectors, transforming cells and culturing transformed host cells to obtain expression of the toxin polypeptide. Suitable hosts for expression of the desired toxin moieties include procaryotes, for example E. coli, and eucaryotes, for example yeast. These procedues are illusrated in the preparation of the conjugate described below.

The target cell binding moiety, in general, comprises an antibody or antibody fragment or derivative. Preferably the antibody comprises a monoclonal antibody. Antibodies may be prepared by methods well known in the art. They may be isolated from serum, from spleen and from antibody secreting hybridomas.

The preparation of antibodies is described, for example, in Methods in Enzymology; Volume 178; Antibodies, Antigens and Molecular Mimicry, Edited by J Langone, Academic Press Inc (see, in particular, Section II: Engineered Antibodies); and Methods in Enzymology; Volume 73; Immunochemical Techniques Part B; edited by J Langone and H Van Vunakis; Academic Press Inc (see, in particular, Section I: Production of Antibodies).

Where the linker comprises the group -S-X-CO-, the antibody (or toxin moiety) may be derivatised by reaction with a compound of formula L₁-S-X-CO-L₂, wherein L₁ and L₂ are displaceable groups. For example CO-L₂ may comprise a carboxylic acid group or, preferably, an activated caboxylic acid group. For example, the group CO-L₂ may comprise an ester or anhydride group. Particular examples for L₂ include an imidazolyl group or a succinimidyl ester group. L₁ may comprise a group which a group which is displaced on oxidation of the thio group, for example it may comprise a pyridine group. The linker is generally reacted with a reduced thiol group on the antibody or toxin moiety (preferably the toxin moiety).

Where other linkers are employed they are reacted with the antibody and toxin moiety by methods known in the art. Generally, the linker is reacted with available respective functional groups on the target cell binding moiety and toxin moiety. However, the target cell binding and toxin moieties may be coupled directly, by formation of a covalent link, for example a disulphide bond.

Where the toxin moiety has a sulphydryl group, such as that group of a cysteine residue, this may be utilised in conjugation. For example it may be utilised to form a di-sulphide bond with a sulphydryl group on the target cell binding moiety or on a linker. Where sulphydryl group is not present on the toxin moiety, it may be derivatised so as to provide such a group and hence facilitate conjungation. This derivatisation may be carried out using, for example iminothiolane.

Where polypeptide linkers are employed the linker may be reacted with free carboxy or amino groups on the toxin and target cell bonding moieties.

Where the toxin moiety comprises the A-chain of ricin and the linker comprises the diradical -COCH₂CH(CH₃)-S-, a suitable linking agent is N-succinimidyl-3-(2-pyridyldithio)butyrate. A preferred process for the preparation of such an immunotoxin comprises reacting derivatised target cell binding moiety with reduced ricin A.

The target cell binding moiety is derivatised by reaction with a coupling agent, such as N-succinimidyl-3-(2-pyridyldithio)- butyrate, and the A-chain of ricin reduced by treatment with a suitable reducing agent to reduce the thiol group on the free cysteine group, using for example dithiothreitol.

Conveniently an excess of the linking agent is employed, so that the derivatised target cell binding moiety has more than one linker bound to it. Following reaction with ricin A any linking groups which are not bound to ricin A are conveniently capped by reaction with a blocking group, for example cysteine.

The conjugates of the present invention are potentially useful in treating tumours. This may be demonstrated by the conjugate's ability to inhibit protein synthesis in tumour cells in vitro, and/or by the conjugate's ability to reduce tumour size or growth in vivo. Details of appropriate test protocols are given below.

### Brief description of drawings:-

Figure 1 illustrates the construction of pICI 0020;
Figure 2 illustrates the construction of pTB344;
Figure 3 illustrates the construction of pICI 0042;
Figure 4 illustrates the construction of pICI 1079;
Figure 5 illustrates the construction of pICI 1187;
Figure 6 illustrates a Coomassie blue-stained SDS gel of E.coli lysates in which track A is pICI 1102; B is pICI 0020, and C is molecular weight markers;
Figure 7 illustrates a gel profile of pICI 1102 in which peak R represents ricin A;
Figure 8 is a western blot of ricin A produced by pICI 1102 and in which track 1 is molecular weight markers; 2 and 3 are non-ricin producing clones; 4 is pICI 1102, and 5 is pICI 0020 (control plasmid-non ricin A sequence);
Figure 9 is a partial sequence of pICI 1102;
Figure 10 illustrates the construction of pICI 1102;
Figure 11 describes a fragment used in the preparation of plasmids;
Figure 12 is a plasmid map of pICI 0042;
Figure 13 illustrates the sequence of a transcription terminator;
Figure 14 is a plasmid map of pICI 1079;
Figure 15 illustrates endocytosis of ¹²⁵I-C242 by COLO 205 cells;
Figure 16 illustrates in the vitro cyctotoxicity of the C242/ricin A immunotoxin against COLO 205 cells;
Figure 17 illustrates an antibody;
Figure 18 illustatrates the cDNA sequence of the C242 kappa chain, varaible reion (cDNA inplasmid pKGE761); and
Figure 19 illustatrates the cDNA sequence of the C242 heavy chain, varaible reion (cDNA inplasmid pKGE762).

The invention will now be illustrated by the following non-limiting Examples in which, unless otherwise stated:-
(i) evaporations were carried out by rotary evaporation in vacuo;
(ii) operations were carried out at room temperature, that is in the range 18-26°C;
(iii) yields are given for illustration only and are not necessarily the maximum attainable by diligent process development;
(v) proton NMR spectra were normally determined at 200 MHz in deuterated dimethyl sulphoxide as solvent, using tetramethylsilane (TMS) as an internal standard, and are expressed as chemical shifts (delta values) in parts per million relative to TMS using conventional abbreviations for designation of major peaks: s, singlet; m, multiplet; t, triplet; br, broad; d,doublet; and
(vi) the following abreviations are used:
   BSA - bovine serum albumin
   PBS - phosphate buffered saline
   IPTG - isopropylthio-β-galactoside
   EtOH - ethanol
   SDS-PAGE - sodium dodecyl sulphate (SDS) polyacrylamide gel electrophoresis (PAGE)

### PREPARATION OF RICIN A/C242 IMMUNOTOXIN

A solution of the monoclonal antibody C242 (200mg) in phosphate buffered saline (sodium phosphate/150 mM sodium chloride pH7.2) at 4.4 mg/ml was concentrated to 12 mg/ml by membrane filtration (Amicon YM10 membrane) at 4^{o}C. The concentrate was diluted with 0.5 vols of borate buffer (100 mM sodium borate pH9.1). The protein concentration was determined by monitoring absorbance at 280 nm and the pH of the mixed solution was noted to be 8.8 +/- 0.1.

N-succinimidyl-3-(2-pyridyldithio) butyrate (the "linker") was dissolved in dry, redistilled dimethylformamide or acetonitrile at a concentration of 10 mg/ml. An aliquot of this solution (0.352 ml), containing 3.52 mg of N-succinimidyl-3-(2-pyridyldithio) butyrate, was added immediately to the concentrated antibody solution. The resulting solution was mixed and then allowed to stand at 15°C for one hour.

The solution was then applied to a desalting column (G25 Sephadex - Pharmacia, 2.6 x 58 cm, flow rate 2 ml/min, equilibrated with 50 mM sodium phosphate/150 mM sodium chloride/1 mM EDTA pH8.0) in order to remove excess reagents and buffer exchange the derivatised antibody. Alternatively, the reaction products can be removed by crossflow filtration. The desalted derivatised antibody was pooled and the protein concentration determined by monitoring the absorbance at 280 nm. The extent of derivatisation with the linker was determined by the addition of excess dithiothreitol and monitoring the release of free thiopyridyl groups at 343 nm. The extent of derivatisation was found to be 4 to 6 linker groups per mole of antibody.

Recombinant ricin A was reduced by treating a solution of ricin A in phosphate buffer at pH 8 with excess dithiothreitol and concentrated by crossflow filtration. Excess reagents were removed by column chromatography (G25 Sephadex - Pharmacia, 2.6 x 58 cm, flow rate 2 ml/min, in 50 mM sodium phosphate/150 mM sodium chloride/1 mM EDTA pH 8.0).

Recombinant ricin A was conjugated to derivatised antibody by mixing prepared recombinant Ricin A (190 mg) and derivatised antibody (190 mg) solutions at a 1:1 w/w Ricin A/derivatised antibody ratio. Glycerol was added to 20 % v/v and the vessel purged with argon. The resulting solution was maintained at 15°C for 40 to 65 hours.

Cysteine was then added to a final concentration of 0.2 mM (and at least 10 fold molar excess over linker groups on the antibody) in order to cap excess linker groups on the antibody. The solution was mixed and maintained at 20°C for 2-3 hours. Following capping with cysteine, a sample of the resulting immunotoxin was analysed by treatment with excess dithiothreitol and monitored at 343 nm to quantitate completion of the reaction.

The solution containing the conjugate was then concentrated by membrane filtration (Amicon YM10 membrane) and applied to a chromatographic column (HR300 Sephacryl - Pharmacia, 2.6 x 50 cm, flow rate 3 ml/min, buffer 50 mM sodium phosphate/25 mM sodium chloride/1 mM EDTA) which results in the separation of immunotoxin and unconjugated antibody from unconjugated Ricin A. The peak containing the mixture of immunotoxin and antibody was pooled and the protein concentration determined by monitoring absorbance at 280 nm.

The resulting solution containing both unconjugated antibody and immunotoxin was adjusted to pH 6.3 by the addition of 1 M HCl and applied to a column containing triazine dye matrix (Mimetic A6XL, ACL plc, Cambridge, UK., 8 x 26 cm column, flow rate 1.5 ml/min). The column was washed with 100 ml starting buffer which elutes unconjugated antibody and the immunotoxin eluted with starting buffer containing 0.5 M sodium chloride. The immunotoxin solution was dialysed against phosphate buffered saline, filtered through a 0.22 micron filter and stored at 4°C.

Purity of the immunotoxin was determined by SDS polyacrylamide electrophoresis and contained a total of 45 mg immunotoxin with the composition : 50 to 60 % mono Ricin A derivative, 10 to 30 % di Ricin A derivative, 5 to 15 % tri Ricin A derivative and <10 % underivatised antibody.

Alternatively the gel filtration step (removal of residual r-ricin A) and the dye affinity chromatography step (removal of residual C-242 antibody) may be transposed in the purification sequence. In this variation of the method the conjugation mixture, immediately after blocking the unreacted linker moieties with cysteine (as described above) is diluted to a conductivity of less than 5mS with distilled water. The pH is then adjusted to 6.0 with 1M acetic acid and the solution clarified by membrane filtration. The solution is then applied to the dye ligand column (2.5ml gel/mg r-ricin A used in the conjugation mixture). The column is then washed with 0.68% sodium acetate pH 6.0 until the unconjugated C242 antibody has been washed through the column. The immunotoxin and residual r-ricin A may then be eluted from the column in 20mM sodium phosphate buffer at pH 7.0, 0.5M with respect to the sodium chloride concentration. This eluate is then concentrated by crossflow filtration using a spiral cartridge ultrafiltration system and applied to a column of Sephacryl S300HR (2.5ml gel for every mg of antibody used in the original conjugation reaction and in a volume <5% of the total volume of the column). The column may be equilibrated in any suitable formulation buffer such as phosphate buffered saline pH 7.2.

### PREPARATION OF THE LINKER MOIETY

### N-Succinimidyl (R)-3-(2-Pyridyldithio)butyrate used above was prepared using the following procedure in which, unless otherwise stated:-

(i) evaporations were carried out by rotary evaporation in vacuo;
(ii) operations were carried out at room temperature, that is in the range 18-26°C;
(iii) yields are given for illustration only and are not necessarily the maximum attainable by diligent process development;
(v) proton NMR spectra were normally determined at 270 MHz in deuterated chloroform as solvent, using tetramethylsilane (TMS) as an internal standard, and are expressed as chemical shifts (delta values) in parts per million relative to TMS using conventional abbreviations for designation of major peaks: s, singlet; m, multiplet; t, triplet; br, broad; d,doublet.

### N-Succinimidyl (R)-3-(2-Pyridyldithio)butyrate

(R)-3-(2-Pyridyldithio)butyric acid (90.0 g, 0.393 mol) was dissolved in dichloromethane (630 ml) with stirring at room temperature. N-Hydroxysuccinimide (45.2 g, 0.393 mol, 1.0 mol equivalents) was added and washed into the mixture with dichloromethane (90 ml). The mixture was stirred for 30 minutes whilst cooling to -2°C. A solution of dicyclohexylcarbodiimide (81.08 g, 0.393 mol, 1.0 mol equivalents) in dichloromethane (540 ml) was added over a period of 35 minutes, whilst maintaining the reaction temperature at 0°C ± 2°C, and washed into the mixture with dichloromethane (90 ml). The solution was stirred at a temperature of 0 - 10°C for 3 hours 15 minutes and then allowed to warm up to room temperature. The solution was filtered to remove solid dicyclohexylurea which was washed with dichloromethane (180 ml x 2). The filtrate was evaporated under reduced pressure at 20 - 22°C to give a brown oil (150 g). This oil was purified by chromatography on a column of Kieselgel 60 silica gel 230 - 400 mesh (1180 g) prepared in toluene/ethyl acetate 80 : 20 v/v. Elution with toluene/ethyl acetate (80:20 v/v) gave N-succinimidyl (R)-3-(2-pyridyldithio)butyrate (52 g) as a pale yellow gum after evaporation under vacuum (0.25 mbar) at 28°C.
NMR:
   1.5 (d,3H), 2.85 (m,4H), 2.8 (dd,1H), 3.2 (dd,1H), 3.5 (m,1H), 7.1 (m,1H), 7.7 (m,2H), 8.5 (d,1H).

Electron impact mass spectroscopy showed the compound to have a molecular weight of 326, with fragmentation consistent with the structure of N-succinimidyl-(R)-3-(2-pyridyldithio)butyrate.

The starting material was prepared as follows:-

### a. (R)-3-Hydroxybutyric Acid

Methyl (R)-3-hydroxybutyrate (1.670 Kg, 14.152 mol) was cooled in an ice/ethanol bath. Water (4698 ml) was added, followed by 47% w/w sodium hydroxide (965 ml, 16.98 mol, 1.2 mol equivalents) at such a rate as to maintain the temperature at 0°C ± 2°C. The reaction mixture was stirred at 0°C for a further 1 hour 30 minutes. Concentrated hydrochloric acid (1490 ml, 17.28 mol, 1.22 mol equivalents) was added to the reaction mixture over a period of 35 minutes, whilst maintaining the temperature at or less than 5°C, to give a final pH of 1.5. Sodium chloride (1061 g) was then added to the mixture and the aqueous mixture was extracted with methyl tert-butyl ether (10 x 3.5 l). The extracts were combined and the solvent was removed by distillation under vacuum at room temperature to give a residue. Toluene (7.5 l) was mixed with the residue and the volatile material removed by distillation under vacuum to give (R)-3-hydroxybutyric acid (1189 g, 81%) as an oil. This oil was cooled to 4°C and seeded to afford a solid.

### b. (S)-β-Butyrolactone

(R)-3-Hydroxybutyric acid (530 g, 5.096 mol) was mixed with triethyl orthoacetate (1322.8 g, 1488 ml, 8.154 mol, 1.6 mol equivalents) at 25-30°C to give a hazy solution (the haziness was believedto be due to residual sodium chloride from the previous stage).
The solution was evaporated under high vacuum (1.0 mm) at 30°C to remove volatile material. The residual liquid (1001 g), which contained (2S,6R)-2-ethoxy-2,6-dimethyl-1,3- dioxan-4-one, was heated at 80°C for 2 hours and then cooled to 40°C. The crude product was distilled through a glass column (50 cm x 3.5 cm diameter) packed with glass Raschig rings to give (S)-β-butyrolactone (57.3g), boiling point 59-62^{o}C at 10-12 mbar.

### c. (R)-3-Mercaptobutyric Acid

(S)-β-Butyrolactone (91.12 g, 1.058 mol) was cooled to 5°C with stirring under nitrogen. Thiolacetic acid (80.57 g, 75.65 ml, 1.058 mol, 1.0 mol equivalents) was added, whilst maintaining the temperature at 5°C. Triethylamine (146.7 ml, 1.058 mol, 1.0 mol equivalents) was then added over a period of 45 minutes, whilst maintaining the reaction temperature at -5 to +5°C. The cooling bath was removed and the temperature rose to 65°C, giving a yellow/orange solution. The reaction mixture was cooled to 10°C and stirred at room temperature overnight. The solution was cooled to 0°C and water (93 ml) was added. The mixture was further cooled to -10°C, and then treated with sodium hydroxide solution (185 ml of 47% w/w sodium hydroxide and 92 ml of water), the rate of addition being such that the temperature was maintained at -10 to +10°C. The mixture was then stirred for 1½ hours at -5°C and then allowed to warm to room temperature. The mixture was washed with methyl tert-butyl ether (265ml). The aqueous layer was diluted with water (185 ml), cooled to 0°C and treated with concentrated hydrochloric acid (270 ml) at 0-10°C until the pH was 1-2. The aqueous mixture was then extracted with methyl tert-butyl ether (2 x 265 ml). The organic extracts were combined, washed with water (265 ml), dried over anhydrous magnesium sulphate and evaporated under reduced pressure at 30°C to give an orange oil (132.8 g). This oil was purified by vacuum distillation to give (R)-3-mercaptobutyric acid (86.6 g), boiling point 96 - 100°C at 2 mbar.

### d. Pyridine-2-Sulphenyl Chloride

2,2′-Dipyridyldisulphide (110.0 g, 0.5 mol) was dissolved in dichloromethane (800 ml) at room temperature to give a pale yellow solution which was cooled to 0°C. Chlorine gas was bubbled into the solution which was maintained at 0 - 5°C. After 3 hours 25 minutes the solution was saturated with chlorine and was golden brown in colour. The solution was concentrated under reduced pressure at 0 - 5°C to remove excess chlorine and precipitate pyridine-2-sulphenyl chloride as a yellow solid. This solid (11.8 g) was collected by filtration and washed with dichloromethane. The dichloromethane filtrate (1624 g) contained a further 133 g of pyridine-2-sulphenyl chloride.

### e. (R)-3-(2-Pyridyldithio)butyric Acid

Pyridine-2-sulphenyl chloride (11.8 g of the yellow solid and 1035 g of the solution in dichloromethane prepared above, 0.667 mol total, 1.0 mol equivalents) was diluted with dichloromethane (50 ml). The mixture was stirred at room temperature for 5 - 10 minutes and then cooled to -5°C. A solution of (R)-3-mercaptobutyric acid (80.0 g, 0.667 mol) in dichloromethane (400 ml) was added, whilst maintaining the temperature at -5 to 0°C. The mixture was stirred overnight at room temperature to give a brown oil and a yellow upper layer. Water (485 ml) was added to the mixture which was then cooled to 5°C and treated with 2N sodium hydroxide solution (385 ml) until the pH was 4.55. The dichloromethane layer was separated and the remaining aqueous phase extracted with dichloromethane (200 ml). The dichloromethane extracts were combined, dried over anhydrous magnesium sulphate and evaporated under reduced pressure to give a light brown oil. The oil was stirred vigorously with hexane (325 ml) and cooled in an ice bath until a solid formed. The mixture was stirred for 3 hours, and then the brown crystalline solid was collected by filtration, washed with hexane (2 x 162 ml) and dried overnight at room temperature in vacuo to give (R)-3-(2-pyridyldithio)butyric acid (140g).
NMR:
   1.45 (d,3H), 2.6 (dd,1H), 2.8(dd,1H), 3.4(m,1H), 7.1(m,1H), 7.7(m,2H), 8.5 (m,1H).

### PREPARATION OF C242 ANTIBODY (C242:II)

### Establishment of hybridoma cell line and production of C242:II

### Preparation of established spleen cells

A human colorectal carcinoma cell line, COLO 205, commercially obtainable from the American Type Culture Collection (ATCC), Rockville, Md., USA, under accession No. CCL 222, was routinely cultured in Iscove's MEM complemented with 10% fetal calf serum (FCS). Cells were harvested prior to confluence, normally 2 to 3 days after subcultivation, and washed 3 times with phosphate buffered saline (PBS) for immunization.

BALB/c mice, 4 to 6 weeks old, were immunized intraperitoneally with a priming dose of 3 x 10⁷ COLO 205 cells suspended in 0.1ml of phosphate buffered saline solution. The animals were then boosted with another 0.1ml suspension of 3 x 10⁷ COLO 205 cells and sacrificed four days later and the spleens were removed. The spleens were then dissociated into a single cell suspension.

### Preparation of hybridoma

1.2 x 10⁸ spleen cells from the above described cell suspension were distributed among two tubes. To each tube were additionally added 10⁸ myeloma cells from the mouse myeloma cell line Sp2/0 (available from the collection of American Type Culture Collection (ATCC), Rockville, Maryland, U.S.A. under the accession No. CRL 1581). The two tubes were centrifuged, and all liquid was decanted. To each tube were then slowly added 2 ml of 37°C PEG solution (10 g of PEG M_{w} 4000, 1 ml of DMSO and 10 ml of monoclonal saline) over 1 minute and with constant stirring. The tubes were transformed to a water-bath at 37°C for 90 seconds with gentle stirring. The fusion was interrupted by adding to each test tube a physiological buffer solution according to the following scheme: 2 ml during the first 30 seconds, 6 ml during the following 30 seconds and another 32 ml over 1 minute. After washing of the cell suspension in culture medium it was suspended in totally 100 ml of hypoxanthine/aminopterin/thymidine (HAT) supplemented culture medium. The culture medium was Iscove's MEM supplemented with 10% FCS, L-asparagine (36 mg/l), L-arginine-HCl (116 mg/l), folic acid (10 mg/l), L-glutamine (292.3 mg/l), sodium pyruvate (110.1 mg/l) and mercaptoethanol (3.49 µl/l). Aliquots of 50 µl were distributed to the wells of 96-well tissue culture dishes. The wells had been precoated with 250 µl of macrophage suspension from BALB/c mice (2 x 10⁴ cells/ml) in HAT supplemented culture medium. Medium was changed 6 days after fusion.

### Screening of antibody hybridomas

The spent medium from day 6 above was tested for COLO 205 positive antibodies as described by Kennett R.H., "Enzyme-linked antibody assay with cells attached to polyvinyl chloride plates". Monoclonal Antibodies, Hybridomas, A new dimension in biological analyses, Eds. H.R. Kennett, T.J. McKelvin, K.B. Bechtol, Plenum Press, New York 1980. In brief, the wells of Nunc immunoplates type IIF were coated with 2 x 10⁵ cells/well of COLO 205 cells (1 mg/100 ml PBS); coating volume 50 µl. The above mentioned spent culture medium from day 6 was then added to the coated wells, 50 µl/well. After incubation over night at room temperature peroxidase conjugated anti-mouse Ig (Dakopatts P260, Dakopatts A/S, Cophenhagen, Denmark) diluted (1/500) in 1% BSA-PBS was added at 100 µg/well and incubated over night at room temperature. Substrate, in the form of 4 OPD-tablets Dako S2000 dissolved in 12 ml of citric acid buffer, pH 5.0, plus 5µl of 30% hydrogen peroxide, was then added at 100 µl/well and the absorbance was measured at 450 nm following incubation.

Roughly 600 hybridoma clones were tested. Initially, 190 of these reacted with COLO 250 cells. Of these, 177 reacted also with normal human lymphocytes prepared on Lymphoprep from Nyegaard A/S, Norway, and were discarded. A clone established from one of the remaining clones was designated hybridoma C242 and was isotyped as IgGl class. The C242 hybridoma was then subjected to a number of subcloning steps to eventually produce a final, stable monoclonal antibody producing a clone designated as C242:II.

Thus, the C242 hybridoma was first cloned resulting in a clone called C242:5. This clone was in turn cloned to form clone C242:5:2. In both these clonings, hybridoma suspension was diluted in hypoxanthine/thymidine (HT) supplemented culture medium to a density of 4 cells/ml. 50 µl (0.2 cells) were distributed to each well in a 96-well culture dish pre-coated with 250 µl of Balb/c mice macrophage suspension (5 x 10³ macrophages/well) in HT supplemented medium. After 2-5 days, single cell clones were detected by visual inspection in a microscope. On day 6, medium was changed and aliquotes of spent media analysed for quantity of antibodies binding to COLO 205 cells but not to normal human cells by ELISA as described above. The best clones in terms of positive reaction in the COLO 205 ELISA with retained negative reaction in the normal cell ELISA were selected and frozen in vials in liquid nitrogen for further development.

For performing a third cloning, the cells were thawed and cultured in DMEM (Dulbecco's Modified Eagle's Medium) (5% FCS). Cells were then seeded into the wells of a 96-well tissue culture plate at a mean density of three cells per well. The cloning was performed in DMEM with 5% FCS and mouse macrophages used as feeder cells. On this plate, 30 clones occured of which 16 were tested for IgG production by nephelometry and specificity of the antibodies by ELISA as described above. Twelve of these clones were found to produce IgG. Ten clones were then expanded on a 24-well tissue culture plate from which spent medium was tested for IgG production and specificity. This selection resulted in the saving of 4 clones with relatively high productivity. A final productivity test of these four clones was performed in duplicate tissue culture flasks. The clone showing the highest productivity was selected and designated as C242/CL 510 A1. It was frozen in liquid nitrogen for further use.

A first cloning of the clone C242/CL 510 A1 indicated that one third of the cells did not produce IgG as judged by an absorbance of less than 0.1 at a dilution of 1:1000 in a general mouse IgG ELISA. Five positive subclones were pooled to create a clone called C242:I. The productivity from this clone was quantified by a general HPLC-based assay to be 150 µg of mouse IgG per ml.

Clone C242:I was subsequently cloned into a 96-well dish yielding 33 clones, all positive for mouse IgG production. Five of these, all yielding above 150 µg/ml, were pooled to create a final clone, designated as C242:II, having a stable productivity of IgG. HPLC assay indicated the productivity of C242:II to be 196 µg of mouse IgG per ml. The cells were frozen and stored in vials in liquid nitrogen. A sample of the C242:II hybridoma produced was deposited at the ECACC under accession number 90012601 as stated above.

### Production of C242 monoclonal antibody

An inital cell suspension of the above prepared hybridoma was obtained from a frozen vial. Cells were seeded into tissue culture chambers with a total area of 6000 cm² from Nunc A/S at a density of 2 x 10⁴ cells/ml (C242:II hybridoma). The cells were then cultured in DMEM modified according to Iscove (Iscove N.N., and Melchers F., J. Exp. Med. Vol. 147, p 923, 1978) and supplemented with 1% gentamycin, 1% amino-acid supplement and 5% fetal calf serum. The cells were then incubated for 4 or 5 days at 37°C in a humidified atmosphere containing 8% CO₂. At the end of incubation cells were counted and samples were taken for determination of monoclonal antibody concentration. The cell culture supernatant was decanted and filtered through a cellulose filter in order to remove suspended cells. The supernatant was then concentrated 20-30 times by ultrafiltration in a Millipore Pellican Ultrafiltration cell using a 30,000 molecular weight cut-off membrane. A sample from the concentrate was taken for analysis of C242:II monoclonal antibody concentration and antigen reactivity, whereupon the monoclonal antibody concentrate was stored at -70°C until purification.

### Purification of C242 monoclonal antibody

Protein-A-Sepharose 4B (trademark of Pharmacia AB, Uppsala, Sweden) was prepared according to the manufacturer's instructions concerning swelling and washing of the gel, whereupon the above produced C242:II (also referred to herein simply as C242 antibody) concentrate was bound thereto using 1.5 glycine-NaOH, 3M NaCl, pH8.9, as binding buffer. After an initial washing using the same buffer, the affinity column was eluted with 0.1M citric acid-NaOH, pH 5.0, and the C242 monoclonal antibody was collected in tubes containing 1 mole/l of Tris-HCl, pH 8.0. The antibody obtained was then dialyzed against 0.02 M phosphate buffer, 0.15 M NaCl, pH 7.2, 0.2 g/l NaN₃. The dialyzed C242 antibody was then concentrated by ultrafiltration using a 30,000 molecular weight cut-off membrane. After taking of samples for concentration and quality control analysis the C242 monoclonal was stored at -20°C.

### PREPARATION OF RICIN A

Ricin A may be prepared by means of recombinant DNA technology using DNA sequences which code for ricin A. Such sequences are described in EP 145,111; O'Hare et al, FEBS Letts, 216, p73-78, 1987; and Lord et al, Eur.J.Biochem, 148, p265-270, 1985. A DNA sequence coding for ricin A will be placed under the control of appropriate control sequences such as a promoter (for example a trp promoter), ribosome binding site, and transcription terminator. The preparation and purification of recombinant ricin A is described in published PCT patent application WO 85/03508 and published European application no. 237,676.

The following illustrates the construction of a recombinant plasmid in which the coding sequence of ricin-A chain is placed under the transcriptional and translational control of a prokaryotic promoter element. Transcription is terminated by the incorporation of a natural termination element at the 3′ end of the message. Translation initiation is controlled by the DNA sequence at the 5′ end of the message, the ribosome binding site (RBS). The method of construction necessitates the substitution of the two N-terminal amino-acid residues of the natural, mature ricin A protein.

Several intermediate stages in the derivation of the vector used to prepare recombinant ricin A are described.

### EXPERIMENTAL PROCEDURES

### 1. Synthetic oligonucleotides

Synthetic oligonucleotides were used to introduce specific DNA sequence alterations into the ricin gene. All oligonucleotides subsequently described were prepared on an Applied Biosystems 380A DNA synthesiser from 5′-dimethoxytrityl base-protected nucleoside-2-cyanoethyl-N,N-diisopropylphosphoramidites and protected nucleosides linked to controlled-pore glass supports on a 0.2 micro mol scale, according to protocols supplied by Applied Biosystems Inc.

Each oligonucleotide, after cleavage from the solid support and removal of all protecting groups, was dissolved in water (1ml) and a measurement of absorbance at 260nm used to determine concentration.

### 2. Enzymes and host strains

A variety of restriction endonucleases and DNA modifying enzymes were used in the manipulations described below. These were purchased from one of a number of suppliers (Amersham International, Bethesda Research Laboratories, Boehringer Mannheim or New England Biolabs) and used in accordance with the manufacturers instructions with respect to reaction conditions.

The host strains referred to herein are generally available from numerous sources. For example, E.coli C600 is freely available from numberous sources including many culture collections such as the E.coli Genetic Stock Centre, Yale University, USA under accession number GCSC 3004. The genotype of E.coli C600 is K12 thr-1 leuB6 thi-1 lacY1 tonA21 λ⁻ supE44; E.coli HB101 and DH5α are available from Bethesda Research Laboratories (BRL); and E.coli TG1 and K19 are available from Anglia Biotechnology.

### 3. Geneclean (TM)

The kit contains 1) 6M sodium iodide 2) a concentrated solution of sodium chloride, Tris and EDTA for making a sodium chloride/ethanol/water wash; 3) Glassmilk (TM)- a 1.5 ml vial containing 1.25 ml of a suspension of silica matrix in water.

This is a technique for DNA purification based on the method of Vogelstein and Gillespie published in Proceedings of the National Academy of Sciences USA (1979) Vol 76, p 615.

Alternatively any of the methods described in "Molecular Cloning - a laboratory manual" Second Edition, Sambrook, Fritsch and Maniatis Cold Spring Harbor Laboratory, 1989 (referred to hereinafter as "Maniatis") can be used.

### 4. Sequenase (TM)

### Chemically modified T7 DNA polymerase

Based on the procedure of Tabor and Richardson published in "Proceedings of the National Academy of Sciences USA (1987) vol 84 pp 4767-4771.

### 5. Construction of the pICI expression vectors

### 5.a) pICI0020

Plasmid vector pICI0020 is a pAT153 based plasmid in which the 651 bp EcoRI-AccI region is replaced by a 167 bp EcoRI - ClaI fragment consisting of:-
(1) a synthetic E. coli trp promoter and trp leader ribosome binding site
(2) a translation initiation codon
(3) a multiple restriction enzyme recognition sequence derived from M13mp18, containing sites for KpnI, BamHI, XbaI, SalI, PstI, SphI and HindIII
(4) a synthetic transcription termination sequence

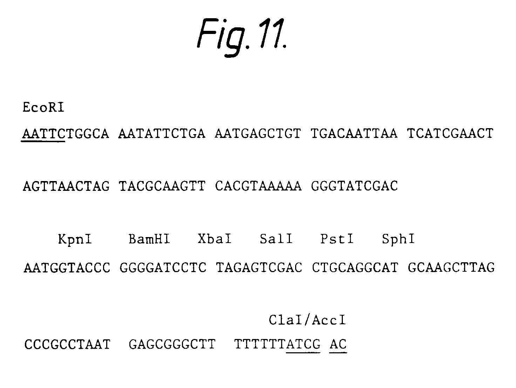

The DNA sequence of this region is shown in Figure 11.

The construction of a plasmid vector containing a synthetic trp promoter sequence is published (Windass et al Nuc.Acids Res. 10 p6639-6657, 1982). A promoter fragment was isolated from such a vector after digestion with the enzymes EcoRI and HpaI and purification of the appropriate band from an agarose gel by electro-elution (in "Molecular Cloning - A Laboratory Manual", Maniatis, Fritsch and Sambrook, published by CSH laboratory, second edition 1989).

A pair of complementary synthetic oligonucleotides were prepared which would ligate to the HpaI end of the promoter fragment providing the natural trp leader ribosome binding site, a translation initiation codon and a 3′ KpnI cloning site. These oligonuleotides were mixed in equimolar concentrations and allowed to anneal by heating to 100°C followed by slowly cooling to room temperature.

The promoter fragment and annealed oligonucleotides were then ligated and the appropriate band isolated from a polyacrylamide gel by electroelution. This fragment was then ligated with an M13mp18 vector derivative containing the trp attenuator sequence (generated from synthetic oligonucleotides) cloned into the HindIII site and introducing an additional ClaI restriction site 3′ to the attenuator. The ligated DNA was transfected into E.coli strain JM109 (Yanisch-Perron et al Gene, 33, p103, 1985) made competent by the CaCl₂ method (Maniatis, chapter 1, p82). After plating out and incubation of the plates, plaques were screened by the method of Benton and Davies (Maniatis, chapter 4, p41) using a ³²P labelled probe generated by nick translation of the EcoRI-HpaI promoter fragment isolated previously. Single stranded DNA was prepared from positively hybridising plaques by a standard method (Maniatis, chapter 4 p29) and sequenced using the M13 universal primer and the Sanger dideoxy chain termination method as provided in kit form by a number of suppliers eg. Sequenase (United States Bioscience).

RF DNA was prepared from one isolate in which the promoter/ribosome binding site/attenuator sequence had been confirmed. This DNA was digested with EcoRI and ClaI and the appropriate fragment isolated from a polyacrylamide gel as above. Plasmid pAT153 was digested with the enzymes EcoRI and AccI and ligated with the isolated promoter fragment. Ligated DNA was transformed into competent E.coli HB101 (Boyer, H.W. and Roulland-Dussoix, D; 1969, J. Mol. Biol., 44, p459) (Bethesda Research Laboratories) and ampicillin resistant colonies selected.

Plasmid DNA from several clones was prepared and DNA sequence derived from the region between the EcoRI and ClaI sites. One clone confirmed as containing the correct promoter/attenuator region was named pICI0020.

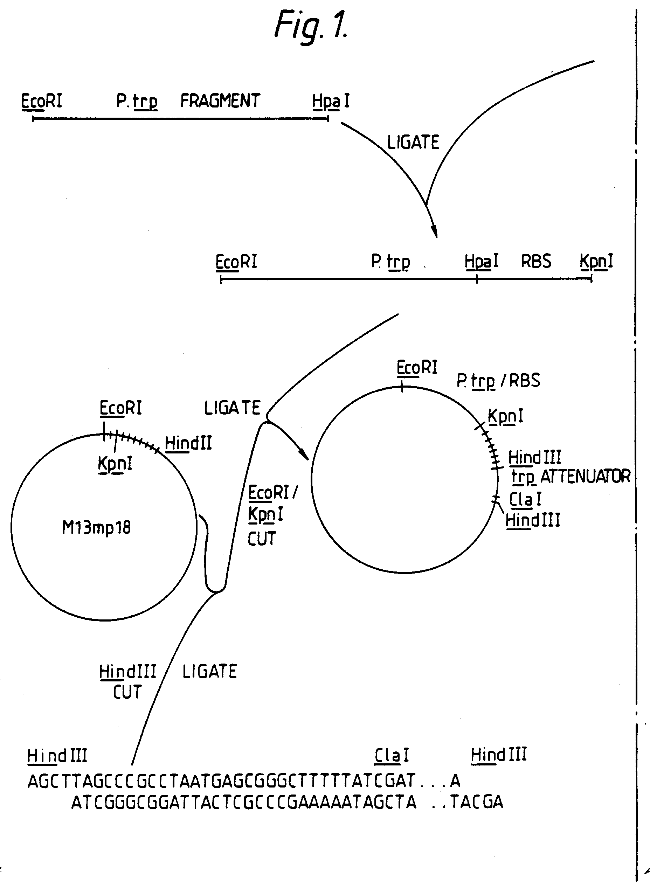

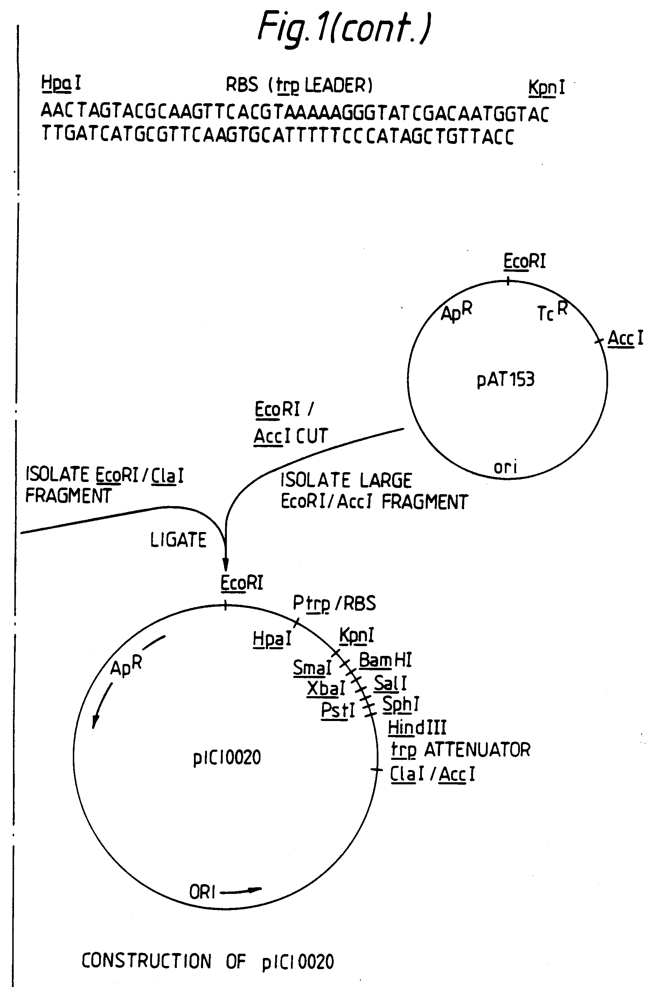

This construction is outlined in fig.1.

### 3.b) pICI0042

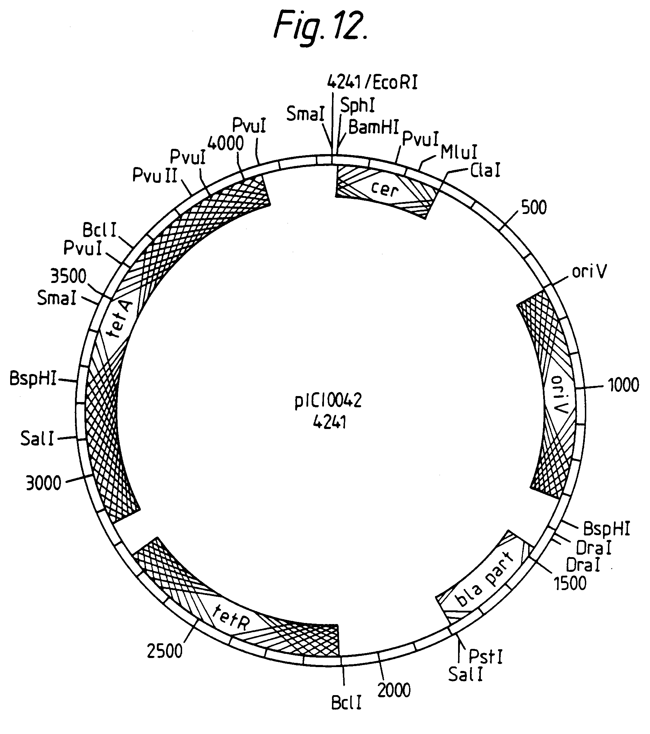

pICI0042 (Figure 12) is a plasmid in which the antibiotic resistance markers of pAT153 have been replaced by a single, inducible tetracycline resistance gene from the plasmid RP4 (encoded by the gene tetA and regulated by the product of the tetR gene). These genes have been characterised by Klock et al (J.Bacteriol., 161, p326-332, 1985). Because the new resistance marker is only expressed in the presence of antibiotic, the tetA gene product will not be a potential contaminant of recombinant ricin A in cultures where the plasmid is stabily maintained in the absence of tetracycline. A plasmid stability function (cer) has also been incorporated.

The initial stage in the generation of this vector was to produce a derivative of pAT153 from which the gene encoding tetracycline resistance had been completely removed. A complementary pair of synthetic oligonucleotides were designed to replace the EcoRI-AvaI fragment from pAT153 with a short sequence containing several unique restriction endonuclease sites for subsequent cloning.

pAT153 plasmid DNA was digested with the enzymes EcoRI and AvaI and the 2.175Kbp plasmid DNA fragment isolated from a 0.7% agarose gel using Geneclean (Bio 101, California) in accordance with the manufacturers instructions. The 1.425Kbp fragment containing the tetracycline resistance gene was thus removed.

The oligonucleotides (SEQ ID's 13 and 14) were phosphorylated using T4 polynucleotide kinase and equimolar amounts annealed together. A sample of the annealed oligonucleotides was then ligated with the plasmid fragment from pAT153. Ligated DNA was transformed into E.coli HB101 (BRL) and ampicillin resistant colonies selected.

Several colonies were picked for small-scale plasmid DNA preparation (method of Birnboim and Doly as specified in Maniatis, chapter 1, p25) and the desired construction identified by restriction analysis with suitable enzymes eg. EcoRI AvaI and BamHI. The structure of 3 isolates identified as having the correct restriction pattern was confirmed by DNA sequence analysis using a pBR322 EcoRI site clockwise primer (New England Biolabs). One isolate was named pICI0019.

RP4 plasmid DNA was isolated from extant stocks by the method of Holmes and Quigley (Maniatis, chapter 1p29). This DNA was cut to completion with BglII and then partially with XmaI (at 25°C for up to 35min) taking samples at various timepoints until a 2.45 Kbp fragment containing the tetR and tetA was clearly identifiable. A sample of pUC8 DNA (Amersham International) was digested to completion with BamHI and XmaI. Ligations were performed to insert the tetracycline resistance genes into the pUC8. Ligated DNA was transformed into E.coli C600 (Appleyard, R.K. Genetics 39 p440, 1954) made competent by the CaCl₂ method (Maniatis, chapter 1, p82) and tetracycline resistant colonies selected. Plasmid DNA was prepared from 8 clones (Holmes and Quigley) and the presence of the RP4 tetR and A genes confirmed by restriction analysis. One of these isolates was named pTB344.

The tetracycline resistance genes were then inserted into pICI0019 (described above) by replacement of an EcoRI/PstI fragment from pICI0019 with the corresponding fragment from pTB344. This results in replacement of the majority of the ampicillin resistance gene in pICI0019 with the tetracycline resistance genes. After digestion and ligation of the plasmid DNAs, followed by transformation into E.coli C600, colonies were selected on the basis of phenotype ie. Tc^{R} and Ap^{S}. Plasmid DNA was prepared from 4 such clones and digested with a combination of enzymes eg. BamHI/PstI/SstI EcoRI/SalI SmaI, StyI/SalI and AvaI/PstI. All 4 clones produced restriction patterns consistent with the desired construct. One of these was designated pTB351.

Summers and Sherratt (Cell 36 p1097-1103, 1984) have shown that the instability of plasmids derived from ColEI (eg. pAT153) is due to the loss of a 283bp sequence, cer, present in the parent plasmid. This sequence helps prevent the formation of plasmid oligomers, the latter appearing to disrupt plasmid partitioning in some as yet undefined way. The cer sequence (Summers, D. et al; Mol. and Gen. Genetics 201, p334-338, 1985; and Cell, 36, 1097-1103, 1984) was isolated from a fragment cloned into pUC18 (pKS492). pKS492 plasmid DNA was digested with BamHI and TaqI to release a 289bp cer-containing fragment. Plasmid pTB351 DNA (isolated from the Dam⁻ host E.coli GM48 - Arraj, J.A. and Marinus, M.G. J.Bact. 153 p562-565, 1983) was digested to completion with BamHI and ClaI and ligated with the digested pKS492 DNA. After transformation of ligated DNA into competent E.coli C600, tetracycline resistant colonies were selected. Restriction analysis of plasmid DNA from putative clones with the enzymes AvaI MluI and PvuI was used to confirm the presence of cer. One isolate with the correct structure was named pICI0042.

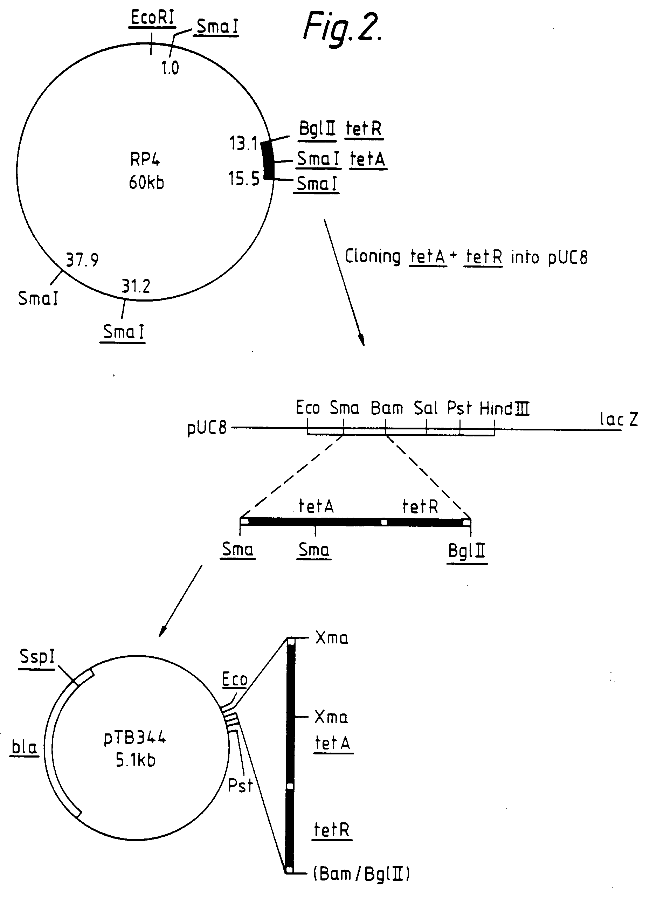

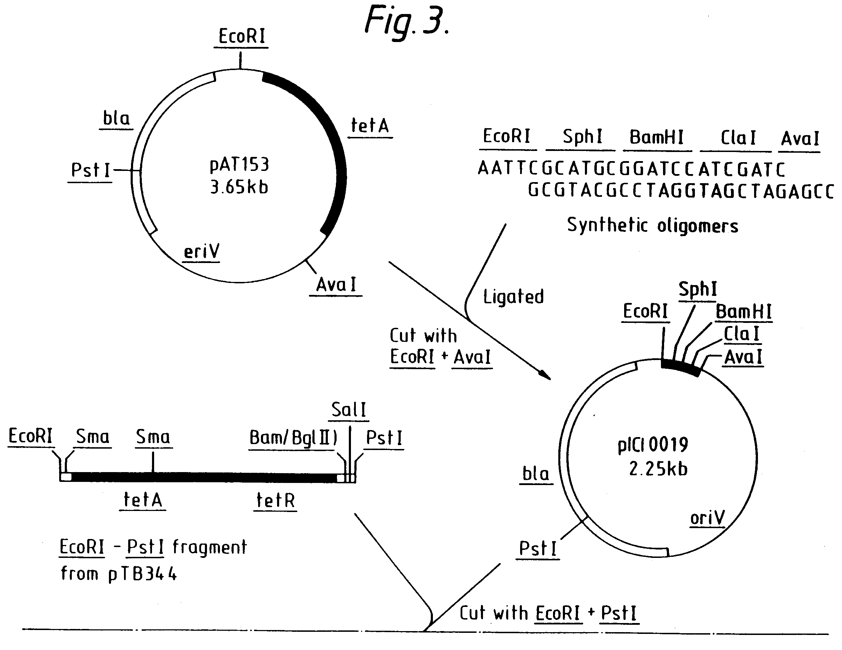

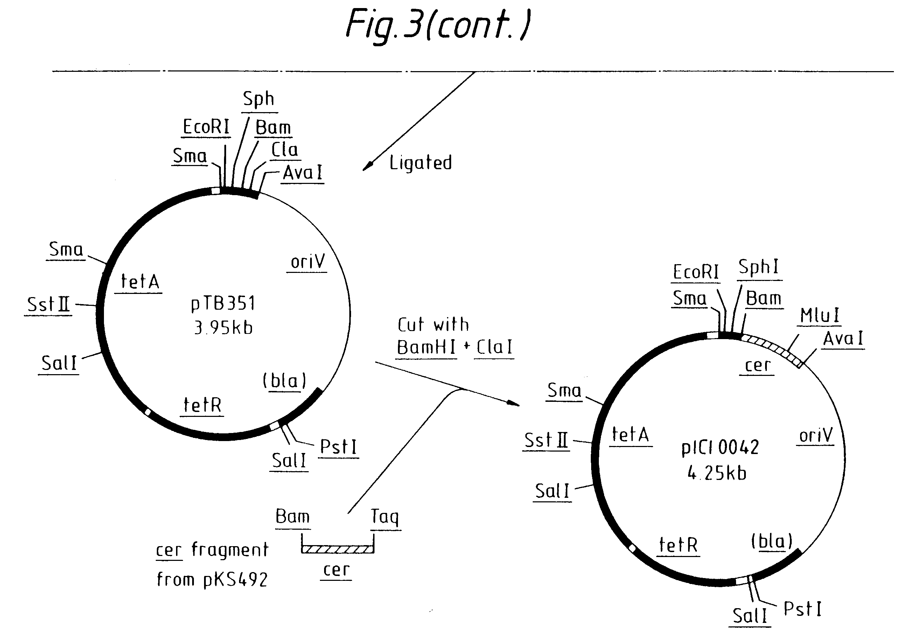

The construction of these plasmids is outlined in figs.2 and 3.

### 5.c) pICI1079

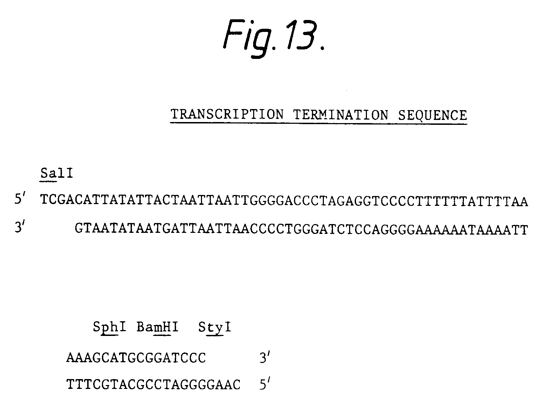

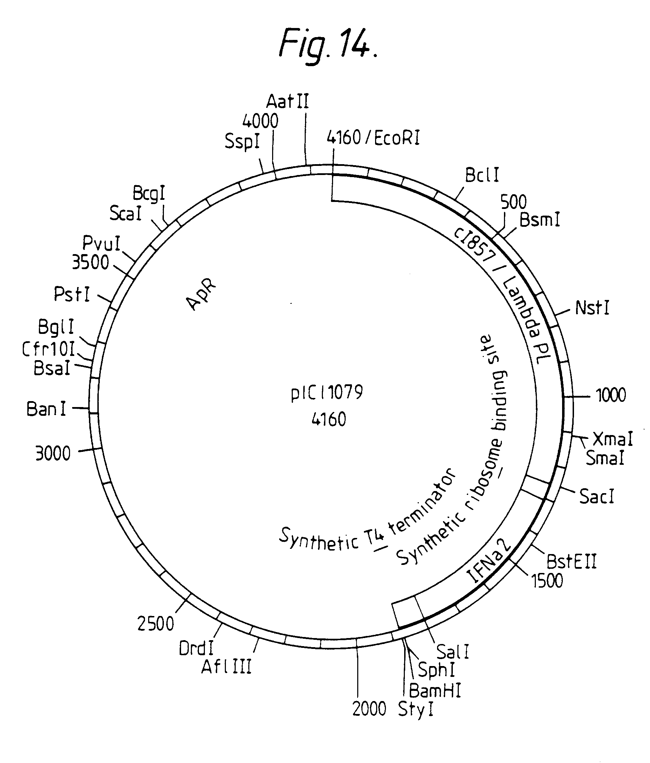

Plasmid vector pICI1079 is an ampicillin resistant, pAT153-derived plasmid containing the following elements between the EcoRI and StyI restriction sites:-
(i) a CI857 gene from phage λ;
(ii) a λP_{L} promoter;
(iii) a synthetic ribosome binding site;
(iv) a synthetic interferon α2 gene sequence;
(v) a synthetic transcription terminator sequence, derived from phage T4, between the SalI and StyI restriction sites. The DNA sequence of this transcription terminator is shown in Figure 13. pICI1079 is illustrated in Figure 14.
   pICI1079 was deposited under the Budapest Treaty on 19 February 1991 at the NCIMB, 23 St. Machaer Drive, Aberdeen, Scotland; and has been allotted the NCIMB Accession no. 40370.

Plasmid pICI1079 was used to provide a source of the T4 transcription terminator for the generation of the ricin A expressing clone pICI1185 (see 7.d below). The starting point for the generation of plasmid pICI1079 was pICI1043. pICI1043 is a plasmid based on pICI0020 (see 3.a above) in which an expression cassette containing a λP_{L} promoter and interferon α2 gene (Edge et al Nuc.Acids Res. 11 p6419-6435, 1983) is present between the EcoRI and SalI sites.

A complementary pair of oligonucleotides was synthesised to generate the transcription terminator from gene 32 of bacteriophage T4 with 5′ SalI and 3′SphI cohesive ends. This fragment was ligated with a plasmid fragment isolated from pICI1043 which had been digested to completion with SalI and SphI. The intermediate plasmid thus produced (pICI1078) contained both the T4 terminator and trp attenuator sequences in tandem.

A second pair of complemetary oligonucleotides was then used to replace the trp attenuator sequence (and remaining part of the tetracycline resistance gene) by insertion between the SphI and StyI sites of pICI1078. A unique BamHI site was introduced within this synthetic fragment.

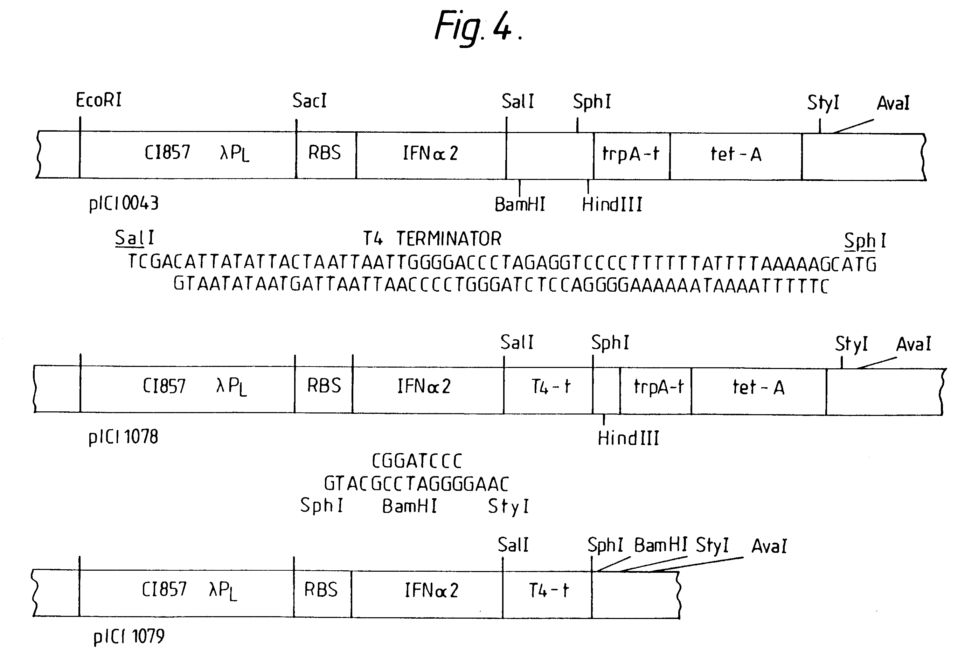

These manipulations are outlined in fig.4.

### 6. Generation of a ricin A expressing clone

### 6.a) Preparation of pUC8RA plasmid DNA

A clone (pUC8RA) was generated which contained cDNA for ricin A. This clone contains A-chain cDNA from base number -74 in the leader sequence through to the BamHI site within the B-chain (base number 857) according to the published cDNA sequence (Lamb,I.F., Roberts,L.M., Lord,J.M. Eur.J.Biochem , 1985, 148, p265-270) in plasmid pUC8 (Vieira,J and Messing,J. Gene, 19, p259, 1982). In addition, site-directed mutagenesis had been used to generate a translation termination codon immediately 3′ to final codon of mature ricin A (as reported in O'Hare, M et al FEBS Letts, 1987, 216, p73-78). The entire A-chain coding region is included in a BamHI fragment from this clone.

A small quantity of pUC8RA plasmid DNA was obtained as described above. For future stocks, a dilution of this DNA was transformed into E.coli DH5α competent cells (Hanahan, D.; 1983, J. Mol. Biol., 166, p557) (Bethesda Research Laboratories) and an ampicillin resistant transformant selected. Plasmid DNA from this clone was prepared by a modified Birnboim-Doly procedure (Maniatis, chapter 1, p25). Samples of this DNA were digested with BamHI and BanI separately and compared to corresponding digests of the original DNA reported by Lord et al after electrophoresis on an agarose gel. No differences in restriction pattern were observed and, on this basis, the two DNA samples were assumed to be identical.

### 6.b) Sub-cloning into M13

BamHI digests of pUC8RA plasmid DNA and RF (replicative form) DNA from the phage M13 strain K19 (Anglian Biotechnology) were "shotgun" ligated using standard conditions (Maniatis, chapter 1, p68). Control ligations were also performed. The ligated DNAs were transformed into E.coli strain TG1 (Gibson, 1984/Anglian) made competent by the CaC1₂ method (Maniatis, chapter 1, p82).

The transformation frequences indicated efficient ligation and recombinant phage were expected in the progeny. Recombinant phage were predicted to produce clear plaques on IPTG + X-gal (BRL) containing plates due to disruption of the lacZ (β-galactosidase) gene. Wild type phage produce blue plaques due to hydrolysis of the X-gal by β-galactosidase.

Several clear plaques were picked for single strand DNA preparation. Direct gel electrophoresis of lysed phage suspensions indicated that one phage clone contained a sizeable insert which was confirmed by sequencing to be the ricin A-chain coding sequence. Only 182 bases of the mature ricin A coding sequence were confirmed but this was taken as sufficient evidence for the presence of the entire ricin A gene. This clone was named M13K19RA

### 6.c) Mutagenesis of M13K19RA

To generate a KpnI site compatible with pICI expression vectors at the start of mature ricin A, the following changes (underlined) are necessary:- 
SEQ. ID. 15

5′....GATAACAACATATTCCCCAAA...... 3′
.....Ricin leader sequence ....|---Mature ricin A-->

Changed to:

SEQ. ID. 16

5′....GATAACAACATGGTACCCAAA.......3′
| KpnI
|
Translation initiation
 and result in an ATG codon overlapping a KpnI site. A KpnI fragment containing ricin A can be excised from the mutant and inserted into the ICI expression vector series . Two N-terminal amino acid modifications are made (ile-phe to met-val).

The single stranded DNA prepared from M13K19RA was the template for the mutagenesis step for each mutation strategy. A single oligonucleotide, DTR16 introducing all the mutational changes for this strategy was synthesised.
DTR16 (SEQ. ID. 17) 5' AACAACATGGTACCCAAACAA 3'

Several protocols exist for the introduction of specific DNA sequence changes by site directed mutagenesis. The procedures outlined below were achieved using the method of Eckstein et al (Nuc. Acid Res., 1985, 13, p8749-8764 and 1986, 14, p9679-9698) as provided in kit form (Amersham International) and used in accordance with the manufacturers instructions.

The principle of this method is to prime the single-stranded DNA template with the mutagenic oligonucleotide and synthesise the complementary strand incorporating dATPαS in place of dATP. Using this nucleotide results in the formation of phosphorothioate bonds which are not cleaved by certain restriction enzymes (eg. NciI). After synthesis of the second strand, NciI is used to nick the parent strand and exonuclease III added to digest back past the mutation point. DNA polymerase I then allows resynthesis of the parent strand. Consequently, the mutagenic oligonucleotide acts as a template for resynthesis and the mutation is introduced into both strands prior to transformation. Mutation frequencies up to 96% of the total progeny are claimed and screening is performed simply by picking plaques at random for sequence analysis.

In our experiments 4 out of 4 plaques picked were correctly mutated.

Having chosen one mutant (MRA16), RF DNA was prepared and checked for the presence of the newly generated restriction fragment ie KpnI.

### 6.d) Cloning, Expression and Initial Characterisation

The pICI series of expression vectors (see section 3) can accept DNA fragments cloned into a unique KpnI restriction site adjacent to the Trp promoter. The KpnI site overlaps the translation initiation codon (ATG) which is situated 8bp downstream from the Shine-Dalgarno site (AGGA) of the promoter.

Having verified the sequence of MRA16, a large scale (∼5µg RF DNA) KpnI digest was performed and the relevent ricin A coding DNA fragment isolated from an agarose gel (Nu-Sieve GTG agarose, FMC Bio-products) by phenol extraction of an excised gel slice according to the manufacturer's protocol.

pICI0020 (see 3a) was digested with KpnI and then dephosphorylated using calf intestinal alkaline phosphatase (CIP - Boehringer Mannheim). The latter treatment prevents recircularisation of the vector upon ligation which would lead to a high proportion of parentals in the transformation progeny.

Ligations were set up with ratios of plasmid vector to isolated fragment from 8:1 (w/w) to 1:3 for the various strategies. Control ligations to test the effectiveness of phosphatase treatment, ligase activity etc., were included. The ligation conditions were appropriate for the source of T4 DNA ligase used (New England Biolabs or Amersham). Reactions were generally incubated at 15°C overnight.

Fifty percent of each ligation (5µl) reaction was diluted to 100µl with 1 x TNE (50mM Tris, 50mM NaC1, 1mM EDTA) and 200µl of competent E.coli DS410 added. After a standard transformation protocol (Maniatis, chapter 1, p74), the cells were plated onto L agar plus streptomycin (25µg/ml) and ampicillin (100µg/ml) and incubated at 37°C overnight.

The transformation plates were examined after incubation. In general, 5 to 10 times more colonies were seen in ligations compared to controls without ligase. In some cases, little difference in the number of colonies produced in the presence or absence of ligase occurred indicating incomplete digestion of the vector or poor ligase activity.

Transformants, plus the relevant controls were picked onto nitrocellulose filters placed on L agar plates for hybridisation screening (based on the method of Grunstein and Hogness as described in Maniatis, chapter 1, p98). After incubation, the colonies were lysed in situ using 10% SDS and 1M NaOH, neutralised using 1M Tris (pH 7.5) and dried under vacuum at 80°C for 2 hours.

Hybridisation probes were generated by ³²P labelling of the mutational oligonucleotides using T4 polynucleotide kinase. The filters were probed at room temperature and then washed in stages up to 55-65°C to remove non-specifically bound counts before autoradiography. Specific hybridisation indicated putative clones containing ricin A DNA.

Small scale DNA preparations (by the methods of Holmes and Quigley or Birnboim-Doly as specified in Maniatis, chapter 1, p25) were made from positively hybridising clones. The DNAs were digested with the relevant restriction enzymes eg. KpnI and EcoRI/BglII, and analysed by electrophoresis on agarose gels. Vector DNAs and mutated RF DNAs were cut with the same enzymes to demonstrate the fragment sizes expected for the correct clones.

Larger scale plasmid DNA preparations (Birnboim-Doly) of each clone were used for more detailed restriction analysis, eg. ClaI, HindIII, BamHI, EcoRI/BglII, KpnI, and ScaI. On agarose gels, these digests showed the size of fragment inserted, an indication of its orientation and the gain of restriction enzyme sites unique to the ricin A chain gene.

### 6.e) Expression studies

The clones positively identified by hybridisation and restriction screening were tested for expression of ricin A by SDS-PAGE analysis of total cell lysates. The standard conditions for expression studies were:-
1) Inoculate 10ml of L-broth + antibiotic(s) with a single colony and grow at 37°C overnight with gentle shaking.
2) Take 750µl of the L-broth and pellet the cells in a microfuge (1 min at 6500 rpm).
3) Resuspend pellet in 300µl M9 medium (Maniatis, appendix A.3) + 0.02% casein hydrolysate + 0.2% glucose + 50µg/ml thiamine and inoculate into 10ml of same.
4) Incubate for 7 hours or overnight at 37°C with gentle shaking.
5) After incubation, measure OD₅₄₀, pellet the cells and resuspend in a volume of Laemmli sample buffer equivalent to that which would give an OD₅₄₀ of 10 if suspended in water (Maniatis, chapter 18, p53). Boil for 15 minutes.
6) Load 20µl of total cell lysate on an SDS polyacrylamide gel, electrophorese, stain with Coomassie blue, destain and visualise.

Of the clones studied by SDS-PAGE, only 1 showed an additional band with an approximate equivalent molecular weight of ∼29KD (equivalent to that estimated for unglycosylated, mature ricin A). Gel scans indicated the accumulation level to be in the range of 5-10% of total cell protein. The plasmid in this clone was named pICI1102.

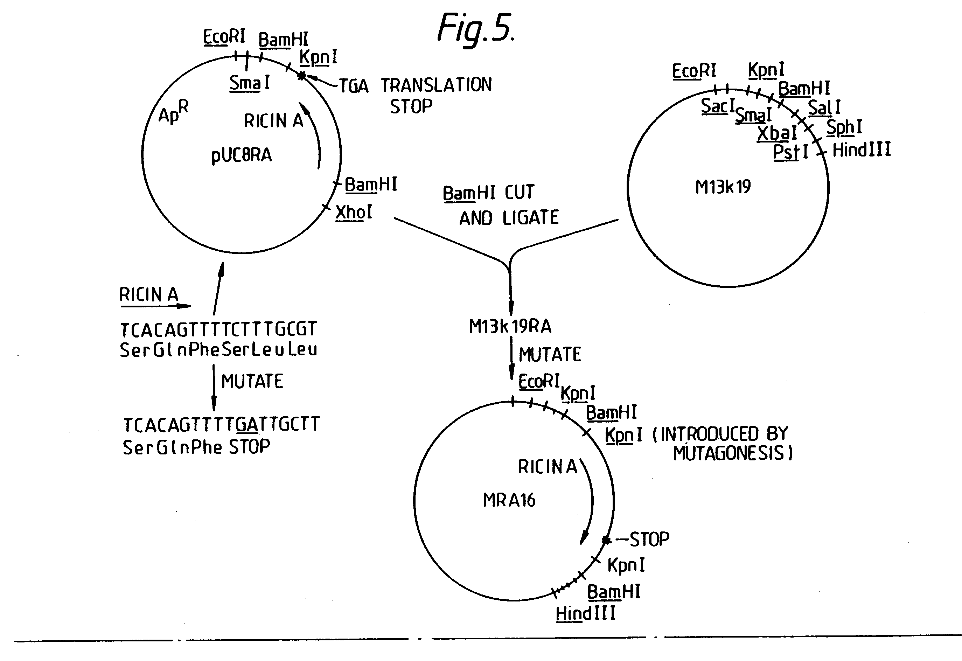

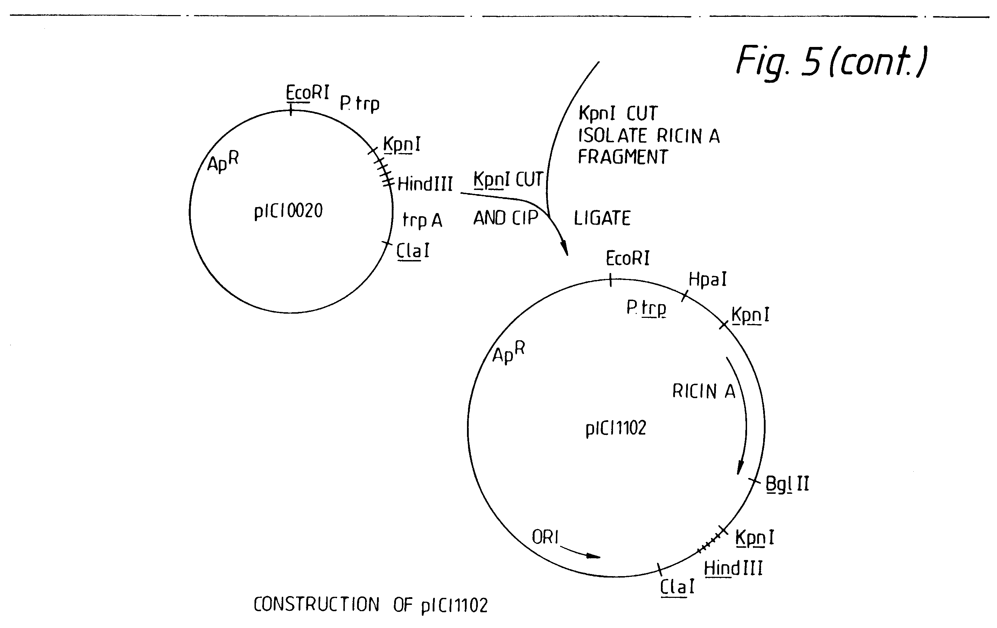

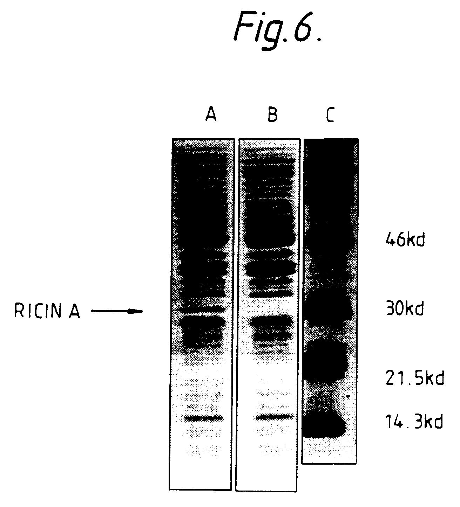

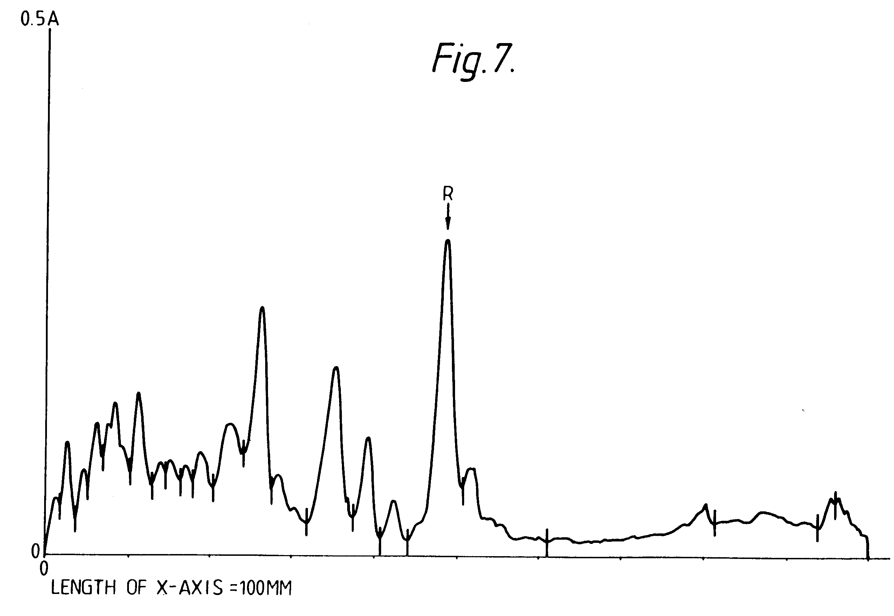

The construction of pICI1102 is outlined in fig.5. Results of expression studies are shown in figs.6 and 7.

### 6.f) Western transfers and immunodetection of recombinant ricin A

Authenticity of recombinant ricin A-chain protein, initially observed by Coomassie blue staining of SDS-polyacrylamide gels, was confirmed by Western blotting. The protein bands were transferred to nitrocellulose filters and detected using a ricin A specific antibody followed by peroxidase labelled antiglobulins.

15% SDS-PAGE gels were run overnight at 8mA then equilibrated for at least 30 minutes in transfer buffer.

Protein bands on the gels were then transferred to nitrocellulose membranes (Hybond-C, Amersham) electrophoretically in a Bio-Rad Trans Blot apparatus at 70V for 3 hours. The filters could be stored, after drying, in sealed plastic bags at -20°C.

Ricin A.1 was a polyclonal antibody raised in rabbits against a synthetic peptide fragment of ricin A. Preliminary studies showed good affinity for ricin A but considerable cross-reactivity with many E.coli proteins. To overcome the high background caused by this cross-reactivity the antibody was pre-incubated with an E.coli lysate.

Thus, a 10ml L-broth overnight culture of E.coli strain DS410 was centrifuged at 4000 rpm for 10 minutes to pellet the cells. The pellet was resuspended in 5ml of bacterial buffer and sonicated at 4-6µ for 6 x 10 second bursts with 30 seconds cooling intervals on ice.

0.5ml of sonicate was then mixed with 0.5ml of ricin A.1 antiserum and incubated at room temperature for 90 minutes. Cell debris was spun down at 13000 rpm for 5 minutes and the supernate stored at -20°C.

The nitrocellulose filters from Western transfers were blocked by incubation overnight at room temperature in 5% BSA-PBS/Tween. (PBS Tween = 5ml Tween 20 per 1 litre of PBS).
Washed 3 x 3 minutes in PBS/Tween.
Incubated 2 hours (or overnight) at room temperature with a 1/4000 dilution of "blocked" Ricin A.1 antibody in 0.5% BSA-PBS/Tween.
Washed 3 x 3 minutes in PBS/Tween.
Incubated 1 hour with a 1/1000 dilution of goat anti rabbit antiserum in 0.5% BSA-PBS/Tween at room temperature.
Washed 3 x 3 minutes in PBS/Tween.
Incubated 1 hour with a 1/5000 dilution of rabbit peroxidase anti-peroxidase antiserum in 0.5% BSA/PBS/Tween at room temperature.
Washed 3 x 3 minutes in PBS/Tween.
Developed by immersion in a solution of 4-chloronaphthol (60mg) in 20ml methanol made to 120ml with PBS and containing 12µl hydrogen peroxide. The membrane was removed from the solution as soon as bands were visible, dried and photographed.

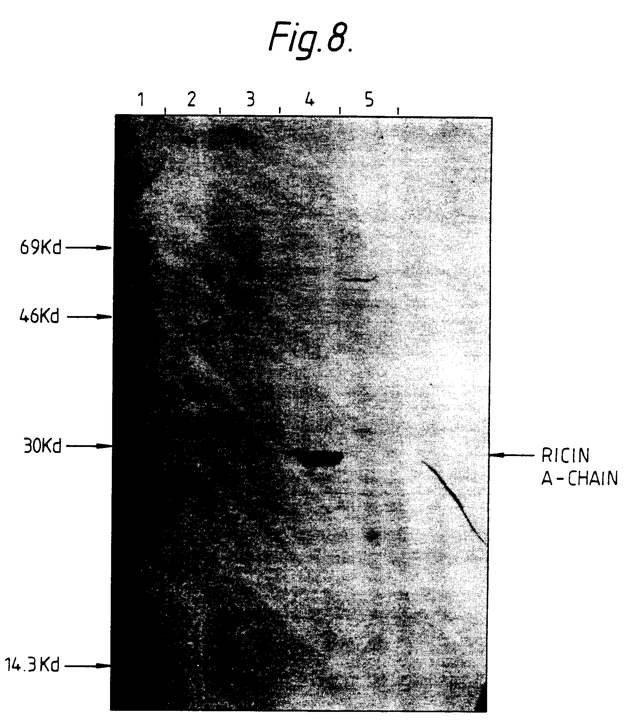

A typical Western blot analysis is shown in fig.8.

### 6.g) Biological assay for recombinant ricin A protein

The aim was to establish an experimental system to test r-ricin A for biological activity in a cell-free in vitro protein synthesis assay.

Rabbit reticulocyte lysates were prepared according to the method of Allen and Schweet (J Biol Chem (1962), 237, 760-767). The assay demonstrates inhibition of protein synthesis in a cell-free system by a lack of incorporation of ¹⁴C-labelled leucine into newly synthesised protein.

### 6.g.i) The assay protocol

Stock solution: 1mM amino acid mix minus leucine.
A solution containing all L-amino acids at 1mM except leucine (adjusted to pH7.4 with NaOH and stored at -70°C).

Soln. A: 40mM Magnesium acetate; 2M Ammonium acetate; 0.2M Tris; (pH 7.4 with HC1, stored 4°C).

Soln. B: ATP (Sigma A5394) 246mg/ml; GTP (Sigma G8752) 24.4mg/ml.

Assay mix: 1ml Amino acid mixture; 1ml Soln. A; 0.1ml Soln. B; 103mg Creatine phosphate; 1mg Creatine kinase; 510µl H₂O; 600µl (60µCi) L-¹⁴C-leucine (New England Nuclear, NEC-279E).

Reaction mix: Test sample 25µl; Assay mix 12.5µl; Rabbit reticulocyte lysate 25µl.

Blank solution was 2mg/ml BSA in PBS

All assays were done in duplicate
12.5µl of assay mix placed in sterile glass tubes
25µl of BSA in PBS added to each of first four tubes for blanks
25µl of test samples added to rest of tubes
1ml 0.1M KOH added to first two tubes (background blank)
Tubes equilibrated to 28°C in a water bath
25µl of rabbit reticulocyte lysate (allowed to thaw from liquid nitrogen temperature) were added to each tube at 20 second intervals.
When first tube had incubated for 12 minutes, 1ml 0.1M KOH was added to each tube again at 20 second intervals to allow all tubes to have 12 minutes incubation. Two drops of 20% hydrogen peroxide were added to each tube followed by 1ml of 20% TCA.
Tubes were mixed and allowed to stand for at least 1 hour, or overnight, at 4°C. The precipitates were filtered on to 2.5 cm GFC discs, washed with 3 x 4 ml of 5% TCA, transferred to scintillation vials and 10ml scintillant (Ready-Solv. MP, Beckman) added. After 1 hour the vials were shaken and counted.

### 6.g.ii)Establishment of technique for use with E.coli lysates

10ml L-broth cultures were grown overnight at 37°C. 400µl aliquots were pelleted at 13000 rpm for 30 seconds and most of the supernate decanted.

The pellets were subjected to 2 rounds of rapid freezing in dry ice/EtOH followed by thawing at 37°C. 12µl of 25% sucrose in 50mM Tris HC1 pH 8.0 were added followed by 4µl of a 10mg/ml solution of lysozyme.

After incubation on ice for 15 minutes, 8µl of 0.25M EDTA were added and incubation continued for 15 minutes. Lysis was brought about osmotically by diluting the samples to 400µl with water. This procedure produced viable cell counts of 80-100 per ml.

When a 25µl aliquot of this lysate was added into the assay reaction mix, the level of incorporation of ¹⁴C-leucine into newly synthesised protein was ∼10% of the blank without lysate. This was a similar level of inhibition to that produced by 8ng/ml ricin A. Dilutions of the E.coli lysate were then prepared and the assay repeated. The result clearly showed that a minimum 16-fold dilution was necessary to reduce the effect of the lysate to equal that of the blank.

In order to be as confident as possible that lysis of E.coli and E.coli lysates would not compromise ricin A toxicity, 2 control assays were performed. The first added plant-derived ricin A to a 16X diluted E.coli cell pellet so as to give a final concentration of 8ng/ml in the assay mix after cell lysis. Both these controls showed no deleterious affect from the lysates or the lysis procedure on the inhibitory action of ricin A.

These techniques were used to verify the synthesis of biologically active, recombinant ricin A from pICI1102 and the clones described below.

### 6.h) DNA sequence analysis

Plasmid DNA sequencing was used to analyse pICI1102. The protocol chosen was modified from Zagursky et al (Gene Analysis Techniques Vol 2, N° 5) and involves alkaline denaturation of double stranded plasmid DNA prior to primer annealing and sequencing by a standard procedure such as that provided in kit form by several suppliers, eg. Sequenase (United States Bioscience). By using an oligonucleotide to prime at the 3′ end of β-lactamase and several A-chain internal primers, sequencing both strands of the promoter and ricin A gene was possible.

The initial sequencing data revealed an unexpected result in that an additional KpnI fragment was present between the promoter and ricin A coding sequence, ie (SEQ. ID. NO 20):

KpnI
------
5′ AAAAAGGGTATCGACATGGTACCCGGGGATCCACCTCAGGGTGG

KpnI
------
TCTTTCACATTAGAGGATAACAACATGGTACCCAAACAATAC 3′

The additional KpnI fragment has come from M13K19RA and contains restriction enzyme sites plus the part of the ricin leader sequence cloned from pUC8RA. The 5′ region of the ricin A chain contains the base changes induced during mutagenesis.

Study of this sequence reveals that the first translation initiation codon (ATG) is out of frame with that the ricin A coding region. Also, there is an in-frame termination codon (TAG) prior to the ricin A initiation codon and a putative Shine-Dalgarno sequence (AGGA) which could re-initiate translation from the second ATG.

Subsequent studies revealed that, surprisingly, this additional DNA fragment conferred a beneficial advantage with respect to the accumulation level of ricin A-chain in E.coli when compared to clones from which it had been excised.

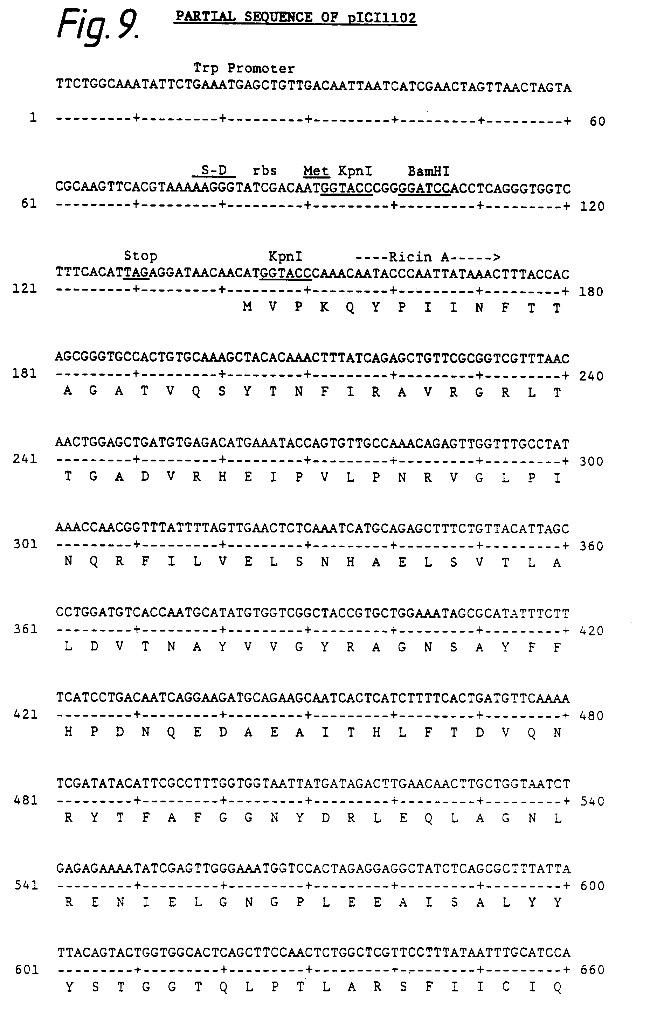

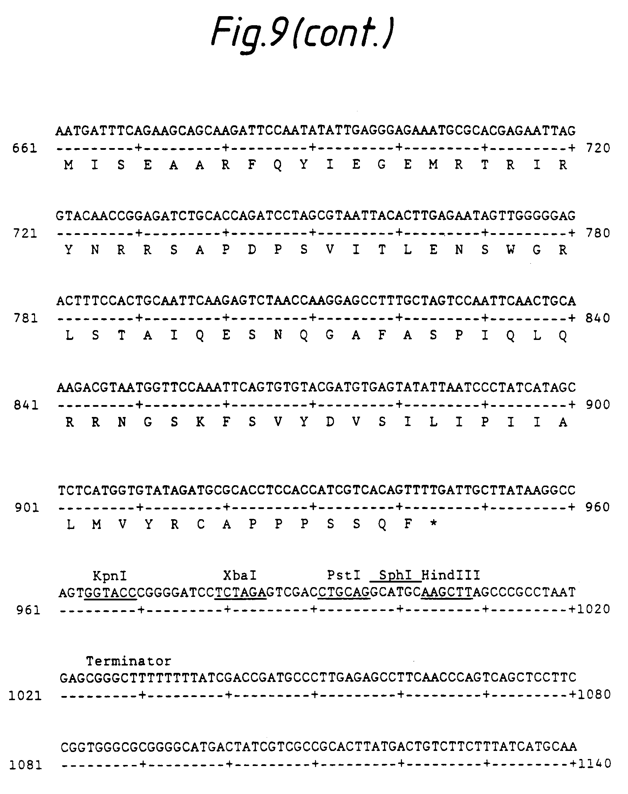

The complete DNA sequence of the ricin A gene contained in pICI1102 is given in fig.9 (SEQ. ID. NO 18).

### 7. Generation of subsequent ricin A expressing clones

### 7.a) Mutation of Ricin-A clone pICI1102 to allow subcloning

To subclone the two KpnI fragments from the fortuitously generated pICI 1120 in the correct orientation for ricin-A expression would be difficult. Consequently, we planned to alter the internal KpnI recognition site by a single base substitution (A to T). This would prevent KpnI cleavage at this site and allow the subcloning of a single KpnI fragment into a range of pICI expression vectors. By substituting the adenine of the KpnI recognition site (GGTACC) with thymine (ie GGTTCC) the first residue of ricin-A is unaltered (GTA/GTT = Val).
ie:

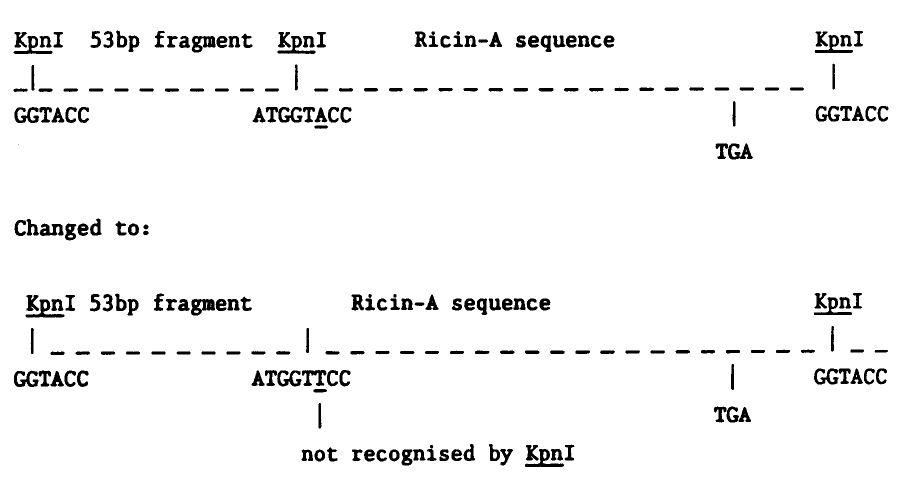
KpnI 53bp fragment KpnI Ricin-A sequence KpnI


GGTACC ATGGTACC GGTACC

TGA

Changed to:

KpnI 53bp fragment Ricin-A sequence KpnI


GGTACC ATGGTTCC GGTACC

TGA

not recognised by KpnI

The oligonucleotide synthesised to produce this change has the sequence (SEQ. ID. NO 19):

5′ A T A A C A A C A T G G T T C C C A A A C A A T A C 3′

Where the underlined base represents the mutational change.

We planned to clone the mutated ricin-A fragment into a range of trp expression vectors for comparative expression studies. Cloning into pICI0020 provides a comparison with pICI 1102 to determine the effects on expression, if any, of the single base substitution.

### 7.b) Mutagenesis

The template for mutagenesis was MRA16 which is the M13 clone containing the two KpnI fragments present in pICI 1102. After mutagenesis, isolates carrying the desired mutations were identified by random sampling and DNA sequence determination over the region to which the mutagenic oligonucleotide binds specifically.

One mutated template was named MRA22. This was analysed further by DNA sequence determination of the entire ricin-A coding sequence to verify the absence of non-specific mutations.

### 7.c) Sub-cloning

The mutated, single-stranded DNAs were transformed into competent E.coli TG1 cells to produce single plaques. Individual plaques were then picked and replicative form (RF, double-stranded) DNA purified by banding on caesium chloride/ethidium bromide buoyant density gradients. The purified RF DNA was digested to completion with KpnI. Cloning was achieved by "shotgun" ligation of the digested RF DNA with the appropriate KpnI cut and phosphatased expression vector or by specific ligation of the ricin-A fragment after its purification from an agarose gel. Ligated DNA was transformed into E.coli TG1 or HB101.

Ricin-A containing clones were identified by hybridisation screening using a ³²P labelled ricin-A probe produced by random hexanucleotide priming of a KpnI fragment isolated from another ricin A containing clone (pICI 1121). Colonies showing positive hybridisation were screened further by restriction analysis of plasmid DNA using a KpnI single digest and an EcoRI/BglII double digest. KpnI identifies the size of the inserted fragment and EcoRI/BglII determines the orientation of the fragment.

Clones confirmed as having the ricin-A fragment in the correct orientation for expression were analysed by SDS-PAGE followed by Coomassie staining and Western blotting of duplicate gels. The level of ricin A accumulation in these clones was equivalent to that detected from pICI1102.

One isolate was selected and the plasmid named pICI1131.

### 7.d) Use of an alternative transcription terminator element.

A transcription terminator element produces a secondary structure at the 3′ end of mRNA which is involved in halting RNA polymerase activity. The stability of this secondary structure can improve the accumulation of protein by protecting the mRNA from exonuclease attack at the 3′ end. The T₄ transcription terminator is regarded as having a stronger secondary structure than that of the trp attenuator present in all previous ricin-A constructs.

In these experiments, the trp promoter and ricin-A fragment from pICI 1131 was excised by digestion with the enzymes EcoRI and SalI. The latter enzyme cleaves between the 3′ terminus of the ricin-A coding sequence and the trpA transcription terminator. The resulting fragment was excised from an agarose gel (2% NuSieve GTG Agarose, FMC Bioproducts) and purified by phenol and chloroform extractions followed by ethanol precipitation. The purified fragment was ligated with pICI 1079 cut with EcoRI and SalI. This latter plasmid contains the T₄ terminator between unique SalI and SphI sites (see 5.c)

Ligated DNA was transformed into competent E.coli HB101 (BRL) and hybridisation screening used to detect the presence of ricin-A DNA as in previous experiments. Positively hybridising clones were chosen for plasmid DNA preparation followed by restriction analysis with EcoRI and SalI together to show the presence of an appropriately sized fragment.

One isolate with the correct construction was identified and named pICI1185.

### 7.e) The use of alternative plasmid background.

Plasmid pICI1185 , was used to produce a further construct by subcloning the expression cassette into pICI 0042. Plasmid DNA was prepared from pICI1185 and digested with EcoRI and SphI together to excise an expression cassette containing the trp promoter/RBS1/ricin-A (MRA22) fragment/T₄ terminator. This fragment was isolated by the method outlined in 4.d and ligated with pICI 0042 cut with EcoRI and SphI.

Ligated DNA was transformed into E.coli HB101 and incubated at 37°C overnight. HB101 transformations were plated on L agar + tetracycline and colonies screened by hybridisation with a ³²P labelled ricin-A DNA probe.

In both cases, positively identified colonies were confirmed by restriction analysis of plasmid DNA using EcoRI/SphI and EcoRI/BglII digests. Three isolates were identified ie pICI1187.1-3.

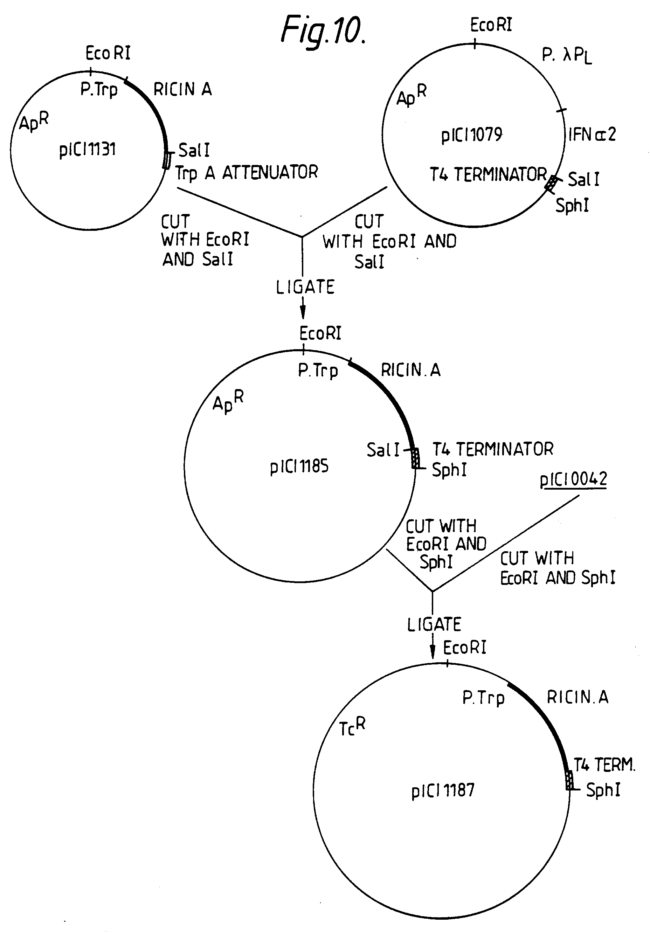

Fig.10 outlines the construction of pICI1185 and pICI1187.

### 7.f) Clone selection

The plasmids isolated were transformed into E.coli 71.18 (Gronenborn, B.; 1976, Mol. Gen. Genet. 148, 243-250) and single colonies picked for clone selection studies. The resulting whole cell lysates were electrophoresed on duplicate SDS-PAGE gels, one of which was stained with Coomassie blue and the other used for Western blot analysis.

The stained gel provided minimal data on ricin-A expression due to the presence of a co-migrating protein from E.coli 71.18. Western blotting clearly indicated ricin-A expression in comparison to positive and negative control samples. One isolate was provided to the Fermentation Research Group for process scale-up.

### r-Ricin A Fermentation

(a) Plasmid pICI 1187 was transformed into E. coli strain DS410 (also referred to herein as MSD68) and the resultant recombinant (MSD1051) purified and maintained on glycerol stocks at -80°C.
An aliquot of the culture was removed from stock and streaked onto agar plates of L-tetracycline to separate single colonies after overnight growth at 37°C. A single colony of MSD 1051 was removed and resuspended in 10ml L- tetracycline broth and 100µl immediately inoculated into each of 10 250ml Erlenmeyer flasks containing 75ml L-tetracycline broth. After growth for 16 hours at 37°C on a reciprocating shaker the contents of the flasks were pooled and used to inoculate a fermenter containing 20L of a modified LCM50 growth medium having the following composition:

| | Made up of distilled water g/l |
|---|---|
| KH₂PO₄ | 3.0 |
| Na₂HPO₄ | 6.0 |
| NaCl | 0.5 |
| Casein hydrolysate (Oxoid L41) | 2.0 |
| (NH₄)₂SO₄ | 10.00 |
| Yeast Extract (Difco) | 20.00 |
| Glycerol | 35.00 |
| MgSO₄. 7H₂O | 0.5 |
| CaCl₂. 2H₂O | 0.03 |
| Thiamine | 0.008 |
| FeSO₄/Citric Acid | 0.04/0.02 |
| Trace element solution (TES) | 0.5ml e⁻¹ |

Fermentations were then carried out at a temperature of 37°C and pH, controlled by automatic addition of 6M sodium hydroxide solution, of pH 6.7. The dissolved oxygen tension (dOT) set point was 50% air-saturation and was controlled by automatic adjustment of the fermenter stirrer speed. Air flow to the fermenter, initially 20L/min, corresponding to 1 volume per volume per minute (VVM) was increased to 45L/min when the fermenter stirrer speed approached 80-90% of its maximum.
Throughout the fermentation samples were taken for measurement of optical density (OD₅₅₀), cell dry weight and accumulation of ricin A within the cells. Ricin A accumulation was measured by scanning Coomassie blue stained SDS-PAGE gels of whole cell lysates of the sampled bacteria as is well known in the art.
From 4½ hours after inoculation, yeast extract (Difco) solution (225g/L) was pumped into the fermenters at a rate of 1.7g/L/hour.
After 12 hours when OD₅₅₀ reached approximately 50, and before the fermentation became oxygen-limited bacteria were harvested on a Sorval RC3B centrifuge (7000g, 30 min, 4°) and accumulated protein recovered from the bacteria.
(b) Plasmid pICI 1187 was transformed into E.Coli strain DS410 (also referred to as MSD68 herein) and the resulting recombinant (MSD 1051) purified and maintained on glyercol stocks at -80°C.

An aliquot (100 µl) of the culture was removed and immediately inoculated into a 2 litre Erlemeyer flask containing 600 ml of L-tetracycline broth. After growth for 16 hours at 37°C on a reciprocating shaker, the contents of the flask were used to inoculate a fermenter containing a growth medium with the following composition:

| Component: | made up of distilled water (g/l) |
|---|---|
| KH₂PO₄ | 3.0 |
| Na₂HPO₄ | 6.0 |
| NaCl | 0.5 |
| Casein Hydrolysate (oxoid L41) | 2.0 |
| (NH₄)₂ SO₄ | 10.0 |
| Yeast extract (Difco) | 20.0 |
| Glycerol | 35.0 |
| MgSO₄.7H₂O | 0.5 |
| CaCl₂.2H₂O | 0.03 |
| Thiamine | 0.008 |
| FeSO₄/Citric acid | 0.04/0.02 |
| Trace element solution | (0.5 ml l⁻¹) |
| Tetracycline | (10 mg l⁻¹) |

The fermentation was carried out at a temperature of 37°C. The pH was controlled by automatic addition of 2M sulphuric acid and 6M sodium hydroxide solution so that the pH was controlled at pH 6.7 up to 10 hours post inoculation and thereafter at pH 6.0.

The dissolved oxygen tension (dOT) set point was 50% air saturation and was initially controlled by automatic adjustment of the fermenter stirrer speed. Air flow to the fermenter was initially 20L/minute corresponding to 1 volume volume per minute (VVM) and was increased to 45L/minute manually when the fermenter stirrer speed reached its maximum (1000 revolutions per minute). The air flow to the fermenter was manually decreased back to 20L/minute when towards the later stages of the fermentation the stirrer speed had automatically decreased to approximately 500 revolutions per minute. The fermentation was performed for 23 hours and during that time samples were taken for measurment of optical density (OD₅₅₀), cell dry weight, accumulation and partitioning of ricin A within the bacterial cells. Ricin A accumulation was measured by scanning Coomassie blue stained SDS-PAGE gels of whole cell lysates of the sampled bacteria as is well known in the art. Partitioning of ricin A in the cytoplasmic (soluble) and inclusion body (insoluble) fractions of the cells was determined by subjecting sampled bacteria to sonication lysis as is well known in the art.

A solution of yeast extract (225 gl⁻¹) was pumped into the fermenter from 4.5 hours post inoculation at 1.7 gl⁻¹hour⁻¹.

When the carbon source in the fermentation became exhausted (leading to a rapid rise in dOT from 50% air saturation) a feed containing glycerol (714 gl⁻¹) and ammonium sulphate (143 gl⁻¹) was pumped into the fermenter at a rate sufficient to meet the maximum carbon demand of the bacteria. The feed rate of the carbon source and ammonium sulphate was then left unchanged during the rest of the fermentation.

After 23 hours the fermentation yielded approximately 1500 mg of soluble ricin A per litre of fermentation broth.

### NOTES:

1. E. coli DS410 (also referred to as MSD68 herein) is well known (Dougan and Sherratt, Molecular and General Genetics, Vol 151, p151-160, 1977). This strain is freely available to the public, and moreover was deposited by the Applicants on 7 June 1985, under the Budapest Treaty, with the National Collections Of Industrial & Marine Bacteria Ltd, Aberdeen, Scotland under deposition number 12100.
2. Trace Element Solution (TES)

TES has the following composition:-

| | mg/10ml (deionized water) |
|---|---|
| AlCl₃.6H₂O | 2.0 |
| CoCl₂.6H₂O | 0.8 |
| KCr(SO₄)₂.12H₂O | 0.2 |
| CuCl₂.2H₂O | 0.2 |
| H₃BO₃ | 0.1 |
| KI | 2.0 |
| MnSO₄.H₂O | 2.0 |
| NiSO₄.6H₂O | 0.09 |
| Na₂MoO₄.2H₂O | 0.4 |
| ZnSO₄.7H₂O | 0.4 |

### PURIFICATION OF r-RICIN A FROM E.COLI

Under optimal fermentation conditions, r-ricin A accumulates as a soluble cytosolic protein. This protein was recovered by breakage of the cells (homogenization) in a buffer which promotes the stability of r-ricin A. This unit operation was performed on live cells at harvest to ensure solution stability of the product. r-Ricin A was recovered from the homogenate by removal of solids (cell debris) by centrifugation. In order for this procedure to be scaled-up the debris was flocculated with an agent (polythene imine) which also precipitates the bulk of the nucleic acid present in the extract. The centrifuge supernatant was then sterile filtered, concentrated by cross flow filtration and the protein precipitated with ammonium sulphate. The ammonium sulphate precipitate was stored frozen at -70^{o}C.

r-Ricin A has an isoelectric point of 7.3 well above the isoelectric point of many other E. coli proteins. The product may therefore be conveniently purified by ion-exchange chromatography. All the recovery and chromatography steps were performed under conditions which promote r-ricin A stability: temperature <15°C, presence of dithiothreitol to maintain the free thiol in a reduced state and EDTA to reduce air oxidation and proleolysis.

### Recovery of r-Ricin A

The cells were collected from the fermentation broth using a continuous disc stack intermittent discharge separator. The broth (50l from 2 x 25l fermentation) was initially transferred from the fermenters to a 50l trundle tank and transported to a contained system consisting of a number of holding tanks connected to the separator and high pressure homogenizer.

The trundle tank was connected to this system and the broth pumped through the centrifugal separater at a flow rate of 40l/hour. The discharge rate was adjusted so that the centrifuge supernatant was clear by visual inspection of an eyeglass in the supernatant discharge line. The solid discharge (Cells) from the separator were collected in a suitable vessel and were resuspended in 40l of Buffer A (50mM sodium dihydrogen orthophosphate; 25mM ethylene diamine tetra acetic acid; 5mM benzamidine; 2mM dithiothreitol; pH 6.3 with 5N sodium hydroxide.) and prechilled to 8^{o}C in the solids receiver vessel. The suspended cells were then transferred back to the trundle tank via the homogenizer adjusted to a working pressure of 600 bar. The resulting homogenate (60l) was chilled to <20^{o}C and made 0.5% with respect to polythenemine by the addition of 2.5l of a 10% (v/v) solution. The suspension was allowed to flocculate for 10 minutes before transfer to the Holding Tank via the centrifugal separator. The clear supernatant was then collected and sterilized by purifying through a depth filter and a positively charged 0.2µ membrane filter.

### Ammonium sulphate Precipitation

The sterile clarified supernatant was concentrated to a volume of 12l using a spiral cartridge cross flow filtration device and the solution brought to 40% saturation by the addition of 2.9kg of solid ammonium sulphate crystals. The solution was allowed to flocculate by gentle stirring overnight at 15^{o}C and then centrifuged. The discharged slurry was collected and stored at -70^{o}C until required for further processing.

### Resolubilization and Desalting

The ammonium sulphate precipitate was thawed in the presence of 14l of Buffer B (50mM sodium dihydrogen orthophosphate; 25mM elthylene diamine tetracetic acid; 2mM dithiothreitol; pH 6.3 with 5N sodium hydroxide;). After 30 minutes the suspension was clarified by centrifugation and desalted by diafiltration against 70l of Buffer B and the conductivity checked that it had been reduced to below 3mS/cm. The desalted solution was clarified further by centrifugation and processed immediately.

### Anion exchange chromatography

The desalted solution was slowly added to a batch chromatography tank containing 2kg of DEAE-cellulose which had been equilibrated with 60l of Buffer B. After stirring for 6.5h the unbound r-ricin A solution was pumped from the bottom of the tank through an 11.3cm diam x 10cm column of packed and equilibrated DEAE-cellulose at a flow rate of 80ml/min. The bulk of the r-ricin A did not bind and was collected in a stainless steel vessel.

### Cation exchange Chromatography

The r-ricin A solution was adjusted to pH 5.5 with 1M orthophosphoric acid and applied to a 10cm diameter x 10cm column of carboxymethyl agarose equilibrated with 10l of Buffer C (25mM sodium dihydrogen orthophosphate; 5mM ethylene diamine tetra acetic acid; 2mM dithiothreitol; pH 5.5 with 5N sodium hydroxide.) The r-ricin A bound to this column and after washing with 10l of Buffer C was eluted with Buffer D (25mm sodium dihydrogen orthophosphate; 5mM ethyl diamine tetracetic acid; 2mM dithiothreitol; 100mM sodium chloride; pH 5.5 with 5N sodium hydroxide). The pure r-ricin A eluted as a single peak which was collected and stored at -70°C as a frozen sterile solution until required for further processing. The r-ricin A is stable under these conditions for up to a year.

The following equipement was used:
Anion exchanger : DE-52, DEAE Cellulose (Whatman Biochemicals)
Cation exchanger : CM/Sepharose (Pharmacia)
Centrifuge : Westphalia CSA-1 disk stack centrifuge (Westphalia)
Homogenizer : APV-Schroeder Lab 60/60 homogenizer (APV)
Filters : AMI 0057P Depth filter, ABI NFZP-Posidyne membrane filter (Pall)
Batching Tank : 701 Pharmacia Batch Chromatography Tank (Pharmacia)
DE-Column : Bioprocess 113 (Pharmacia)
DM-Column : K100/50 (Pharmacia).

### BIOLOGICAL PROPERTIES OF C242 ANTIBODY AND IMMUNOTOXIN

### Immunohistochemical evaluation of C242 (C242.II) in colorectal cancer and normal tissues

Specimens from primary colo-rectal carcinoma and various normal tissues were obtained from patients undergoing surgical resection. The tissues were kept on ice and were frozen within 1 hour after resection in iso-pentane pre-chilled with liquid nitrogen. The tissues were stored at -70°C until sectioned. The frozen biopsies were cut into 5µm sections and fixed in 50% acetone for 30 seconds at +4°C followed by 100% acetone for 5 minutes at +4°C. The sections were air-dried and rinsed in PBS for 10 minutes. All sections were then incubated in 0.3% hydrogen peroxide in PBS for 5 minutes to block endogenous peroxidase and rinsed twice in PBS. Thereafter the sections were treated with normal swine serum and diluted 1:10 in PBS-4% BSA for 5 minutes at +4°C to block non-specific binding of antibodies.

All incubations with antibodies were carried out in a humid atmosphere for 30 minutes at room temperature. The sections were first incubated with monoclonal antibody C242 (C242:II), obtained in Example 1 above, in PSB-4% BSA, thereafter with biotinylated horse anti-mouse IgG (Vectastain - trademark, Vector Laboratories, Burlingame, CA, USA) diluted 1:400 in PBS-4% BSA with 2% swine anti-rabbit immunoglobulin and finally with Avidin DH/biotinylated horseradish peoxidase H complex (Dakopatts A/S, Copenhagen, Denmark). After each incubation the slides were rinsed in PBS for 15 minutes. The sections were then treated with substrate for 15 minutes, rinsed in PBS, counterstained with haematoxylin and mounted with glycerolgelatin (Merck, Darmstadt, Germany). The substrate used was 10mg of 3-amino-9-ethylcarbazole (Sigma, St. Louis, Mo., SA) dissolved in 6ml of dimethylsulfoxide and diluted with 50ml of 0.02 M sodium acetate, pH 5.5, containing 4µl of 30% H₂O₂. The substrate solution was filtered prior to use. The sections were then examined and the results are presented in Table 1 below.

**TABLE 1**

| **Staining of colo-rectal tumours and various normal tissues with C242:II** | |
|---|---|
| TISSUE | STAINED/TOTAL |
| Colo-rectal carcinoma | 26/41 |
| normal colon | 8/16 |
| mammary gland | 2/3 |
| parotid gland | 2/2 |
| skin | 0/1 slight staining of sweat glands |
| liver | 0/2 weak staining of bile ducts |
| kidney | 0/1 |
| pancreas | 2/2 ducts |
| stomach | 0/1 |
| small intestine | 0/1 |

As appears from Table 1, 63% of the examined biopsies from colo-rectal tumours showed positive staining with monoclonal C242:II. The expression of the antigen CA242 in normal colonic tissue was found to be generally weaker than in tumour tissue, the staining being entirely localized to columnar epithelium and goblet cells. Non-colon normal tissue was almost devoid of reactivity with monoclonal C242:II, whereas positivity was found in normal breast ducts, pancreatic ducts, bile ducts, and sweat glands, but the intensity of staining was weak.

### Immunohistochemical evaluation of C242 (242:II) in normal human tissues taken at post mortem

An extensive range of normal human tissues were collected from a total of 21 individuals at post mortem. The best 5 sets of tissue specimens were selected for evaluating C242:II immunoreactivity.

At Post Moretem, the tissues were removed and placed immediately into prechilled tubes containing a basic medium with added antibiotic. The tissue containing tubes were then transported to the laboratory (on ice), the culture fluid removed and the tissue trimmed into 0.5cm³ cubes. The tissue cubes were placed on filter paper strips and snap frozen in liquid N₂. The frozen tissues were stored at -80°C until sectioned. At sectioning, the frozen samples were cut into 6µm sections which were adhered to glass microscope slides. The sections were fixed by immersing the slides in 100% acetone at room temperature for 2 minutes. The slides were then removed from the acetone, allowed to air dry and stored at -20°C prior to use.

All incubations were carried out in a humid atmosphere for 30 minutes at room temperature. The sections were first incubated with monoclonal antibody C242:II in Tris Buffered Saline (TBS), thereafter with horseradish peroxidase conjugated rabbit anti-mouse IgG (Dako Ltd, High Wycombe, Bucks, UK) diluted 1:50 in TBS containing 20% human serum (Sigman Chemical Company Ltd, Poole, Dorset, UK) and finally with horseradish peroxidase conjugated swine anti-rabbit IgG (Dako Ltd), diluted 1:50 in TBS containing 20% human serum. After each incubation the slides were rinsed 2x in TBS. The sections were then treated with substrate for 3-5 minutes, rinsed in TBS, counterstained with Mayers haematoxylin and mounted with Synthetic Mounting Medium (Shandon Scientific Ltd, Runcorn, Cheshire, UK).
The substrate used was 10 mg of diaminobenzidine (Sigma) dissolved in 17 mls of TBS containing 17µl of 30% H₂O₂. The substrate solution was filtered prior to use. The sections were examined and the results are presented in table 2 below.

**TABLE 2**

| **Staining of post mortem normal tissues with C242:II** | |
|---|---|
| TISSUE | STAINED/TOTAL |
| Cerebellum | 0/3 |
| Cerebrum | 0/3 |
| Mid-brain | 0/3 |
| Heart | 1/5 faint, diffuse reactivity |
| Lung | 0/5 |
| Peripheral Nerve | 0/3 |
| Kidney | 1/5 reactivity to proximal/distal tubules |
| Liver | 1/5 faint reactivity to ducts |
| Pancreas | 2/2 ductal and acinar |
| Colon | 2/3 faint reactivity to epithelium |
| Small Intestine | 3/3 faint reactivity to epithelium |
| Adrenal | 2/5 faint reacity to capsule |
| Bladder | 1/5 diffuse |
| Thyroid | 1/5 diffuse |
| Parathyroid | 0/2 |
| Skin | 2/4 squamous epithelium and sweat glands |
| Striated Muscle | 1/5 diffuse |
| Spleen | 0/5 |
| Lymph Node | 0/5 |
| Stomach | 3/3 faint reactivity to epithelium |
| Testes | 0/3 |
| Parotid | 1/1 faint reactivity to ducts |
| Tonsil | 4/4 reactivity to squamous epithelium |

As appears in Table 2, 72% of the normal P.M tissues screened had no C242:II reactivity. Of the 28% scored positively, the majority of the staining was either diffuse, without definition or focal, predominantly to ductal structures within the tissues. More heterogeneous, but still minimal binding was observed to the gastrointestinal epithelium. 2/4 skin specimens stained positive with reactivity to squamous epithelium and sweat glands. Squamous epithelium also stained positively on the 4 tonsil specimens screened with C242:II.

### Endocytosis of C242:II binding to COLO 205 human colon carcinoma

An endocytosis assay was conducted as follows:
Single cell suspensions were prepared from COLO 205 cells (see prepartion of C242 antibody described above) grown as a monolayer culture. The cells were trypsinized, extensively washed and resuspended in culture medium. Iodine labelled C242:II antibody from above (200 000 cpm corresponding to 50ng of monoclonal antibody) was added to a single cell suspension of COLO 205 cells (10⁶ cells) in 5ml test tubes. The final volume was 200 µl/tube.

Cells were incubated in the presence of ¹²⁵I-C-242 for 1 hour on ice (0°C). The suspension was then centrifuged and the cells were washed free from unbound monoclonal antibody. The preincubated cells were further incubated at 37°C for 10 minutes time intervals during 1 hour, the cells being rechilled and pelleted at each time. Radiolabel released to the culture medium was measured from the supernatant (LKB gamma counter, Pharmacia AB, Uppsala, Sweden). The supernatant was also subjected to TCA precipitation and the radioactivity of the precipitate was measured. Surface bound ¹²⁵I-C242 was removed by acid wash with 0.2M glycine-HCl buffer, pH 1.5, containing 2.5 mg/ml papain, and the radioactivity, representing internalized ¹²⁵I-C242, was counted. The amount of surface bound antibody after each time, incubated at 37°C, was calculated by subtracting released and internalized monoclonal antibody from total surface bound monoclonal antibody. Degradation of released antibody was measured from TCA precipitation of medium supernatant.

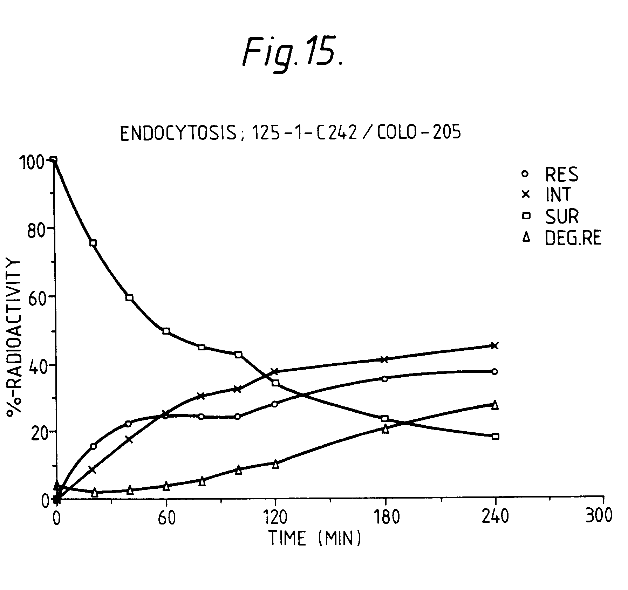

The results are presented in Fig. 15 in the accompanying drawing showing the endocytosis of ¹²⁵I-C242:II by COLO 205 cells, the radioactivity on cell surface ("Sur"), internalized radioactivity ("Int"), released radioactivity ("Res") and degraded released radioactivity ("Deg.Re") being expressed as the percentage of total initial radioactivity on cell surface. In Fig. 15 it can be seen that a more than 20% internalization of C242:II was obtained within 1 hour when preincubated cells were incubated at 37°C. There were small amounts of acid soluble radioactivity when medium supernatant was TCA precipitated which indicates little fragmentation of released activity after 1 hour of incubation.

### Cytotoxicity of ricin A/C242 antibody immunotoxin

### In vitro cytotoxicity

This test shows the in vitro cytotoxicity of the immunotoxin against a human colo-rectal tumour cell line (Colo 205-ATCC No.CCL 222).

Colo 205 cells were grown in suspension in RPM1640 medium with 2.0 g/litre sodium bicarbonate, without glutamine, with 5% heat inactivated foetal calf serum, 2mM L-glutamine and 50µg/ml gentamicin. Cells were counted using haemocytometer blocks. For protein synthesis inhibition assays, cells were plated at 2 x 10⁴ cells/well in 96 well plates in a volume of 100µl. Immunotoxin samples were added to 3 replicate wells. Immunotoxin was added at 12 doubling dilutions from 2,000 to 0.98 ng/ml. 100µl of culture medium was added to some wells as control. Each plate was incubated for 24 hours at 37°C, prior to 2µCi of ³H-L-Leucine being added to each well. The plates were incubated at 37°C for a further 24 hours. 50µl of trypsin was added to each well and the plates incubated for a further 15-20 minutes. The cells were harvested from each well/plate onto glass fibre mats and radioactivity determined using an LKB betaplate scintillation counter. Protein synthesis inhibition curves were constructed and IC₅₀ values generated. The results obtained with the preferred immunotoxin (preparation descibed above) are illustrated in Figure 16.

### In vivo cytotoxicity

This test shows the in vivo cytotoxicity of the immunotoxin against the human tumour cell line, COLO 205, grown as subcutaneous xenografts in athymic mice. Control groups, utilising antibody alone or phosphate buffered saline were also tested.

5 X 10⁶ COLO 205 cells were injected at a single sub-cutaneous site in the flanks of athymic mice. Tumours were allowed to grow and measured every 3-4 days in two dimensions using calipers. Injection of test material was not initiated until tumours had reached 0.7-1.0 cm square. This stage is day 0 and test materials were injected intravenously into tail veins on day 0, 1 and 2. Tumour size continued to be measured in two dimensions and presented as relative tumour volume. Measurements were made every 3-4 days until the experiment was terminated.

This test compares the effects on relative tumour volume of phosphate buffered saline, C242 antibody alone (1.2 mg/kg) and the preferred immunotoxin (preparation described above) (2.0 mg/kg) on tumour growth, and the results are illustrated in the following Tables.

| GROUP 1 Phosphate buffered saline 0.1ml / 10g / i.v. / x3 daily Mouse | | | | | | | | | | |
|---|---|---|---|---|---|---|---|---|---|---|
| Day | 1 | 2 | 3 | 4 | 5 | 6 | 7 | 8 | 9 | 10 |
| 0 | 1 | 1 | 1 | 1 | 1 | 1 | 1 | 1 | 1 | 1 |
| 4 | 2.02 | 1.15 | 2.28 | 1.02 | 0.51 | 0.80 | 2.05 | 1.58 | 0.79 | 1.23 |
| 8 | 5.04 | 3.27 | 5.08 | 2.18 | 1.09 | 1.83 | 4.54 | 4.75 | 1.59 | 3.63 |
| 10 | 6.41 | 4.32 | 5.24 | 3.94 | 1.96 | 2.44 | 5.58 | 5.91 | 1.85 | 4.43 |
| 15 | | | 7.72 | 5.24 | 4.13 | 2.94 | 7.25 | 9.45 | 2.71 | 7.02 |
| 18 | | | 11.81 | 6.78 | 3.53 | 8.02 | | 14.39 | 3.30 | 11.48 |

| GROUP 2 C242 antibody 1.2 mg/kg / i.v. / x3 daily Mouse | | | | | | | | | | |
|---|---|---|---|---|---|---|---|---|---|---|
| Day | 1 | 2 | 3 | 4 | 5 | 6 | 7 | 8 | 9 | 10 |
| 0 | 1 | 1 | 1 | 1 | 1 | 1 | 1 | 1 | 1 | 1 |
| 4 | 1.64 | 2.19 | 1.85 | 1.08 | 1.48 | 2.07 | 1.65 | 1.36 | 2.14 | 2.47 |
| 8 | 3.31 | 4.30 | 3.28 | 4.07 | 3.38 | 4.78 | 6.08 | 2.96 | 3.34 | 7.31 |
| 10 | 4.46 | 4.65 | 4.88 | 4.85 | 3.44 | 5.11 | 8.82 | 4.19 | 4.12 | 7.43 |
| 15 | | | | 5.29 | | | 10.80 | 2.57 | | |
| 18 | | | | | | | 15.03 | | | |

| GROUP 3 immunotoxin 2.0 mg/kg / i.v. / x3 daily Mouse | | | | | | | | | |
|---|---|---|---|---|---|---|---|---|---|
| Day | 1 | 2 | 3 | 4 | 5 | 6 | 7 | 8 | 9 |
| 0 | 1 | 1 | 1 | 1 | 1 | 1 | 1 | 1 | 1 |
| 4 | 0.63 | 0.86 | 1.40 | 0.64 | 0.48 | 0.43 | 0.73 | 0.91 | 0.77 |
| 8 | 0.44 | 0.87 | 1.08 | 0.48 | 0.58 | 0.41 | 0.67 | 0.93 | 1.07 |
| 10 | 0.48 | 0.71 | 1.39 | 0.29 | 0.55 | 0.51 | 0.56 | 1.13 | 1.21 |

As illustrated in the above Tables, the immunotoxin gives rise to a reduction in tumour size (Tables show relative tumour volumes) in contrast to the phosphate buffered salaine (PBSA) control and antibody alone.

### Reactivity of C242.II with Human Pancreatic Tumour Tissue

This test shows the reactivity of C242.II to human pancreatic tumour tissue.

C242.II reactivity to human pancreatic tumour tissue was determined using the same immunohistological techniques as described for human normal tissue reactivity. Fresh frozen pancreatic tumour tissue was obtained from fine needle biopsied material and formalin fixed/paraffin embedded tissue was obtained from tumour resections. 13/16 of pancreatic tumours stained positively. In general, the extent and pattern of binding was better than or equal to that observed in specimens of colo-rectal tumour tissue.

### In Vitro Potency of the immunotoxin in Pancreatic Tumour Cell Lines

This test shows the in vitro cytotoxicity of the immunotoxin against a human pancreatic tumour cell line. The cell line Pan 1, derived from a pancreatic adenocarcinoma, was used to quantitate the in vitro cytotoxic potency of the immunotoxin, and by FACS analysis was shown to express the target antigen. To quantitate the potency, varying concentrations of the immunottoxin were added to Pan 1 cells in culture, to derive an IC₅₀. A MOPC 21:ricin A conjugate and ricin A alone were used as negative controls. The bioassay was performed three times and a mean potency of 40 +/- 18 ng/ml was obtained. The negative controls were not cytotoxic at concentrations up to 1,000 ng/ml. This data demonstrates that the target antigen is present on pancreatic tumour cells and that antigen positive tumour cells are susceptible to killing by the immunotoxin.

### Molecular cloning of cDNA's encoding parts of the light and heavy chains of the monoclonal antibody C242:II

### A. Preparation of total RNA

Total RNA from 10⁸ cells was prepared essentially according to the method of Cirgwin, J.M. et al. (1979; Biochemistry 18, 5294-5299) as modified by Chomezynksi, P. and Sacchi, N, (1987; Anal. Biochem. 162, 156-159). Briefly, the cell pellet was dissolved in 15 ml of cold denaturing solution consisting of 4 M guanidinium thiocyanate; 25 mM sodium citrate, pH 7; 0.5% N-lauroyl sarcosine; 0.1 M 2 mercaptoethanol. After the dissolution of the cell material 1.2 ml of 2 M sodium acetate (pH 4.0) was added and the mixture extracted with phenol-chloroform-isoamyl alcohol (250:49:1). After centrifugation the RNA was precipitated from the aqueous phase by adding an equal volume of isopropanol, and incubating at -20°C over night in order to precipitate the total RNA. After centrifugation and resuspension of the RNA, the precipitation was repeated, and after an additional centrifugation and precipitation the total RNA yield was calculated to be 960 µg based on absorbance measurements.

### B. Isolation of mRNA

In order to isolate polyadenylated mRNA from the above preparation of total RNA, the PolyATract^{TM} mRNA isolation system of Promega Corporation, Madison, Wisconsin, U.S.A., was utilised according to the manufacturer's instructions (cf..: Promega Technical Bullentin, No. 090: 1990). Briefly, 960 µg of total RNA were dissolved in water and annealed with a biotinylated oligo(dT) probe (50 pmoles) in 0.4 x SSC (1 x SSC= 8.77 g NaCl, 4.41 g sodium citrate per litre of water at pH 7.0). Streptavidin coated paramagnetic beads (Streptavidin Magnesphere^{TM}) were added and after a 10 minute incubation the magnetic particles were removed and washed several times with 0.1 x SSC. The polyadenylated mRNA was eluted from the spheres by incubation in water, yielding 5 µg of mRNA.

### C. Preparation of cDNA phage library in E.coli

cDNA was prepared from polyadenylated mRNA of the previous step essentially according to the method of Gubler, U., and Hoffman, B.J. (1983; Gene 25, 263-269), as modified for the vector Uni-ZAP^{TM} (Sratagne Inc. La Jolla, California) which permits undirectional cloning of double stranded cDNA. Briefly, 5 µg of polyadenylated mRNA was converted to single stranded cDNA by priming with the Uni-ZAP^{TM} linker primer (56 ng/ml), adding dATP, dGTP, dTTP, and 5-methyl-dCTP, at 0.6 mM and 45 U of Moloncy Murine Leukemia Virus reverse transcriptase. The mixture was incubated at 37°C for 1 hours. Second strand cDNA synthesis was performed by adding dATP, dGTP, dTTP, and dCTP to a final concentration of 0.15 mM, 3.2 U RNase H, and 6.5 U DNA Polymerase 1, and incubating for 2.5 hours at 16°C. The mixture was extracted with phenol-chloroform (1:1) and ethanol precipitated. The ends of the cDNA were rendered blunt by incubating with 0.16 mM dNTP and 10 U T4 DNA polymerase at 37°C for 30 minutes After phenol-chloroform extraction and ethanol precipitation, the resulting blunt-ended cDNA was ligated to EcoRI adaptors by adding 3 Weiss U T4 DNA ligase, 1 mM ATP, and incubation over night at 8°C. After ligation the EcoRI cohesive ends were kinased by adding 10 U T4 Polynucleotide kinase in the presence of 1 mM ATP, and incubating for 30 minutes at 37°C. The resulting cDNA with phosphorylated EcoRI cohesive ends, was digested with 90 U Xhol in order to create an Xhol cohesive end from the sequence encoded by the Uni-ZAP^{TM} linker primer. The resulting cDNA was then ligated to 1 µg of Uni-ZAP^{TM} XR EcoRI and Xhol prepared arms in the presence of 2 Weiss U T4 DNA ligase, 1 mM ATP, and incubating at 12°C overnight. The ligation mixture was packed in vitro into λ-phage particles by using the Gigapack II Gold^{R} packaging extract according to the manufacturer's instructions, and the resulting phages were used to infect E.coli PLK-F'. The resulting cDNA library of 8.5 x 10⁴ independent clones was amplified once to give a phage titer of 7.5 x 10⁸ pfu/ml. The resulting cDNA library was screened using E.coli XL1-Blue as host strain (cf.. Bullock, W. (1978) Biotechniques 5, 4) as will be described below.

### D. Preparation of hybridization probes

In order to detect cDNA clones encoding the IgG1 heavy chain and kappa chain of the C242:II antibody, hybridization probes covering the first constant domain of the heavy chain, CH1, and the constant domain of the kappa chain, CK, were prepared from mouse genomic DNA by the polymerase chain reaction according to Saiki. R.H., et al. (1988; Science 239, 487-491). Briefly, oligonucleotide primer pairs hybridizing to the 5′ and 3′ regions of the exons encoding the above immunoglobulin domains were used for amplification of mouse genomic DNA. After purification by agarose gel electrophoresis the resulting DNA probe fragments were labeled with 32p by the random primer extension method according to Feinberg, A.P., and Vogelstein, B. (1983; Anal. Biochem. 132, 6).

### E. Screening for IgG1 heavy and kappa chains encoding sequences and insertion into plasmids

The cDNA library from section C above was screened in two separate reounds using the immunoglobin IgG1 and kappa probes, respectively, from section D according to methods described by Sambrook, J. et al. (1989; Molecular Cloning, 2nd Ed, Cold Spring Harbor Laboratory Press). Positively hybridizing phage clones from the two separate hybridisations were further purified by rescreening using the same probes as in the initial screening. Positively hybridizing phage clones were expanded in cultures of E.coli CL1-Blue, and the resulting phage stocks used to prepare cDNA containing pBluescript SK(-) plasmids by phagemid excision and propagation essentially according to Short, J.M. et al. (1988; Nucleic Acids Res. 16, 7583-7600).

### F. Characterization of plasmid cDNA from hybridizing colonies

The resulting cDNA containing plasmids were characterised by restricion enzyme mapping, and plasmids containing inserts of the expected sizes were subjected to dideoxy DNA sequencng according to Sanger, F. et al. (1977; Proc. Natl. Acad. Sci. USA 74, 5463-5467). A plasmid hybridizing with the Ig kappa probe, denoted pKGE761, contained a cDNA insert whose sequence encodes an open reading frame closely homologous to previously known mouse kappa light chain sequences (c.f. Kabat, E.A. et al. (1987) Sequences of Protein of Immunological Interest, 4th Ed., U.S. Department of Health and Human Services, National Institutes of Health). A partial cDNA sequence encoding the variable region of the kappa light chain, VK, and its translated protein sequence, is depicted in Fig. 18. The three CDR sequences, designated as CDR1 to CDR3, are indicated by underlining. A signal peptide preceding the aminoterminal end of the VK segment is indicated by S. CK indicates the constant region following teh carboxyterminal end of the VK sequence.

A plasmid hybridizing with the IgG1 probe, denoted pKGE762, contained a cDNA insert whose sequence encodes and open reading frame closely homologous to previously known mosue IgG1 heavy chain sequences (c.f. Kabat, E.A., et al. vide supra). A partial cDNA sequence encoding the IgG1 heavy chain variable region, VH, and its translated protein sequence, is depicted in Fig. 19. The three CDR sequences, designated as CDR1 to CDR3, are indicated by underlining. A signal peptide preceding the aminoterminal end of the VH segment is indicated by S. CH1 indicates the constant region following the carboxyterminal end of the VH sequence.

### COMPOSITIONS

The following illustrates a representative pharmaceutical dosage form containing an immunotoxin of the present invention which may be used for therapeutic purpose in humans.

### Injectable solution

A sterile aqueous solution, for injection, containing:

| | |
|---|---|
| Ricin A/C242 antibody immunotoxin | 1.0mg |
| Sodium acetate trihydrate | 6.8mg |
| Sodium chloride | 7.2mg |
| Tween 20 | 0.05mg per ml of solution |

## Claims (Claims for the following Contracting State(s): AT, BE, CH, DE, DK, FR, GB, IT, LI, LU, MC, NL, PT, SE)

1. A conjugate which comprises a toxin moiety and a target cell binding moiety which is selective for gastrointestinal tumours and wherein the target cell binding moiety comprises C242 antibody or a target cell binding moiety which has substantially the same cell binding properties.

2. A conjugate as claimed in claim 1 wherein the target cell binding moiety comprises C242 antibody or a fragment thereof.

3. A conjugate as claimed in claim 1 or 2 wherein the toxin moiety comprises recombinant ricin A.

4. A conjugate as claimed in claim 1, 2 or 3 wherein the target cell binding moiety and the toxin moiety are coupled by way of a bifunctional linker.

5. A conjugate as claimed in any one of the preceeding claims wherein the target cell binding moiety and the toxin moiety are coupled by way of a disulphide or thioether bond.

6. A conjugate as claimed in any one of the preceeding claims wherein the target cell binding moiety and the toxin moiety are coupled by a linker of the formula -S-X-CO-, wherein X is a straight chain (1-6C)alkyl group, optionally substituted by one or two substituents selected from (1-6C)alkyl, phenyl and (1-4C)alkylphenyl; or an (1-4C)alkylphenyl group, in which the alkyl moiety is optionally substituted by one or two substituents selected from (1-6C)alkyl, phenyl and (1-4C)alkylphenyl; and wherein the phenyl ring may bear a substituent selected from halogen, (1-4C)alkyl and (1-4C)alkoxy.

7. A conjugate as claimed in claim 6 wherein X comprises a methylene, ethylene, propylene or butylene group substituted by one or two groups selected from methyl, ethyl, propyl and butyl.

8. A conjugate as claimed in claim 6 wherein the linker comprises the radical -S-CH(CH₃)CH₂CO-.

9. A conjugate as claimed in any one of the preceding claims wherein the target cell binding moiety has CDR regions with sequences substantially as follows:
light chain 
CDR1: ArgSerSerLysSerLeuLeuHisSerAsnGlyAsnThrTyrLeuTyr
CDR2: ArgMetSerAsnLeuValSer
CDR3: LeuGlnHisLeuGluTyrProPheThr
 Heavy Chain 
CDR1: TyrThrGlyMetAsn
CDR2: TrpIleAspThrThrThrGlyGluProThrTyrAlaGluAspPheLysGly
CDR3: ArgGlyProTyrAsnTrpTyrPheAspVal
 the CDR regions being arranged such that the target cell binding moiety has substantially the same cell binding properties as C242 antibody.

10. A conjugate which is of the formula: 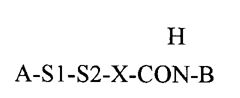 wherein
A comprises recombinant ricin A, and B comprises a target cell binding moiety which comprises C242 antibody;
S1 is a sulphur atom present on recombinant ricin A;
the NH is on the C242 antibody;
S2 is a sulphur atom group and X a straight chain (1-6C)alkyl group, optionally substituted by one or two substituents selected from (1-6C)alkyl, phenyl and (1-4C)alkylphenyl; or an
(1-4C)alkylphenyl group, in which the alkyl moiety is optionally substituted by one or two substituents selected from (1-6C)alkyl, phenyl and (1-4C)alkylphenyl; and wherein the phenyl ring may bear a substituent selected from halogen, (1-4C)alkyl and (1-4C)alkoxy.

11. A conjugate which comprises C242 antibody and recombinant ricin A, linked from an amino group on C242 to a thiol of a cysteine residue in ricin A by a 3-mercaptobutyric acid diradical (-CO.CH2.CH(CH3)-S-).

12. A conjugate which comprises recombinant ricin A and a target cell binding moiety having at least one of the CDR regions with sequences substantially as follows:
light chain 
CDR1: ArgSerSerLysSerLeuLeuHisSerAsnGlyAsnThrTyrLeuTyr
CDR2: ArgMetSerAsnLeuValSer
CDR3: LeuGlnHisLeuGluTyrProPheThr
 Heavy Chain 
CDR1: TyrThrGlyMetAsn
CDR2: TrpIleAspThrThrThrGlyGluProThrTyrAlaGluAspPheLysGly
CDR3: ArgGlyProTyrAsnTrpTyrPheAspVal
 the number of such CDR's and their arrangement being such that the target cell binding moiety has substantially the same cell binding properties as C242 antibody.

13. A conjugate which comprises a toxin moiety and a target cell binding moiety which is specific for a gastrointestinal tumour epitope.

14. A conjugate as claimed in claim 13 wherein the target cell binding moiety is specific for the epitope to which C242 antibody binds.

15. A process for the preparation of a conjugate as claimed in claim 1, which process comprises:
(a) for those conjugates in which the target cell binding moiety is directly coupled to the toxin moiety, reacting the toxin moiety with the target cell binding moiety; or
(b) for those conjugates in which the target cell binding moiety is coupled to the toxin moiety by a linker, reacting the target cell binding moiety derivatised with linker with the toxin moiety, or reacting toxin moiety derivatised with linker with the target cell binding moiety.

16. A pharmaceutial composition which comprises a conjugate as defined in any one of the preceding claims in association with a pharmaceutically acceptable diluent or carrier.

17. The use of a conjugate as claimed in any one of claims 1 to 14 for the manufacture of a medicament for treating gastrointestinal tumours.

18. The use of a conjugate as claimed in any one of claims 1 to 14 for the manufacture of a medicament for treating colorectal tumours.

## Claims (Claims for the following Contracting State(s): ES, GR)

1. A process for the preparation of a conjugate, wherein the conjugate comprises a toxin moiety and a target cell binding moiety which is selective for gastrointestinal tumours and wherein the target cell binding moiety comprises C242 antibody or a target cell binding moiety which has substantially the same cell binding properties; which process comprises:
(a) for those conjugates in which the target cell binding moiety is directly coupled to the toxin moiety, reacting the toxin moiety with the target cell binding moiety; or
(b) for those conjugates in which the target cell binding moiety is coupled to the toxin moiety by a linker, reacting the target cell binding moiety derivatised with linker with the toxin moiety, or reacting toxin moiety derivatised with linker with the target cell binding moiety.

2. A process as claimed in claim 1 wherein the target cell binding moiety comprises C242 antibody or a fragment thereof.

3. A process as claimed in claim 1 or 2 wherein the toxin moiety comprises recombinant ricin A.

4. A process as claimed in claim 1, 2 or 3 wherein the target cell binding moiety and the toxin moiety are coupled by way of a bifunctional linker.

5. A process as claimed in any one of the preceeding claims wherein the target cell binding moiety and the toxin moiety are coupled by way of a disulphide or thioether bond.

6. A process as claimed in any one of the preceeding claims wherein the target cell binding moiety and the toxin moiety are coupled by a linker of the formula -S-X-CO-, wherein X is a straight chain (1-6C)alkyl group, optionally substituted by one or two substituents selected from (1-6C)alkyl, phenyl and (1-4C)alkylphenyl; or an (1-4C)alkylphenyl group, in which the alkyl moiety is optionally substituted by one or two substituents selected from (1-6C)alkyl, phenyl and (1-4C)alkylphenyl; and wherein the phenyl ring may bear a substituent selected from halogen, (1-4C)alkyl and (1-4C)alkoxy.

7. A process as claimed in claim 6 wherein X comprises a methylene, ethylene, propylene or butylene group substituted by one or two groups selected from methyl, ethyl, propyl and butyl.

8. A process as claimed in claim 6 wherein the linker comprises the radical -S-CH(CH₃)CH₂CO-.

9. A process as claimed in any one of the preceding claims wherein the target cell binding moiety has CDR regions with sequences substantially as follows:
light chain 
CDR1: ArgSerSerLysSerLeuLeuHisSerAsnGlyAsnThrTyrLeuTyr
CDR2: ArgMetSerAsnLeuValSer
CDR3: LeuGlnHisLeuGluTyrProPheThr
 Heavy Chain 
CDR1: TyrThrGlyMetAsn
CDR2: TrpIleAspThrThrThrGlyGluProThrTyrAlaGluAspPheLysGly
CDR3: ArgGlyProTyrAsnTrpTyrPheAspVal
 the CDR regions being arranged such that the target cell binding moiety has substantially the same cell binding properties as C242 antibody.

10. A process for the preparation of a conjugate, wherein the conjugate is of the formula: 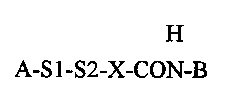 wherein
A comprises recombinant ricin A, and B comprises a target cell binding moiety which comprises C242 antibody;
S1 is a sulphur atom present on recombinant ricin A;
the NH is on the C242 antibody;
S2 is a sulphur atom group and X a straight chain (1-6C)alkyl group, optionally substituted by one or two substituents selected from (1-6C)alkyl, phenyl and (1-4C)alkylphenyl; or an
(1-4C)alkylphenyl group, in which the alkyl moiety is optionally substituted by one or two substituents selected from (1-6C)alkyl, phenyl and (1-4C)alkylphenyl; and wherein the phenyl ring may bear a substituent selected from halogen, (1-4C)alkyl and (1-4C)alkoxy;
which process comprises:
reacting a derivative of the target cell binding moiety of formula 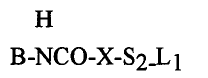 wherein B, X and S₂ are as defined above and L₁ is a displaceable group, with recombinant ricin A.

11. A process for preparing a conjugate which comprises C242 antibody and recombinant ricin A, linked from an amino group on C242 to a thiol of a cysteine residue in ricin A by a 3-mercaptobutyric acid diradical (-CO.CH2.CH(CH3)-S-), which process comprises reacting a derivative of formula 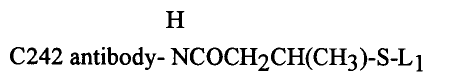 wherein the NH is on the C242 antibody and L₁ represents a pyridyl group, with ricin A.

12. A process for the preparation of a conjugate, wherein the conjugate comprises recombinant ricin A and a target cell binding moiety having at least one of the CDR regions with sequences substantially as follows:
light chain 
CDR1: ArgSerSerLysSerLeuLeuHisSerAsnGlyAsnThrTyrLeuTyr
CDR2: ArgMetSerAsnLeuValSer
CDR3: LeuGlnHisLeuGluTyrProPheThr
 Heavy Chain 
CDR1: TyrThrGlyMetAsn
CDR2: TrpIleAspThrThrThrGlyGluProThrTyrAlaGluAspPheLysGly
CDR3: ArgGlyProTyrAsnTrpTyrPheAspVal
 the number of such CDR's and their arrangement being such that the target cell binding moiety has substantially the same cell binding properties as C242 antibody; which process comprises:
(a) for those conjugates in which the target cell binding moiety is directly coupled to the toxin moiety, reacting the toxin moiety with the target cell binding moiety; or
(b) for those conjugates in which the target cell binding moiety is coupled to the toxin moiety by a linker, reacting the target cell binding moiety derivatised with linker with the toxin moiety, or reacting toxin moiety derivatised with linker with the target cell binding moiety.

13. A process for the preparation of a conjugate, wherein the conjugate comprises a toxin moiety and a target cell binding moiety which is specific for a gastrointestinal tumour epitope, which process comprises:
(a) for those conjugates in which the target cell binding moiety is directly coupled to the toxin moiety, reacting the toxin moiety with the target cell binding moiety; or
(b) for those conjugates in which the target cell binding moiety is coupled to the toxin moiety by a linker, reacting the target cell binding moiety derivatised with linker with the toxin moiety, or reacting toxin moiety derivatised with linker with the target cell binding moiety.

14. A process as claimed in claim 13 wherein the target cell binding moiety is specific for the epitope to which C242 antibody binds.

15. The use of a conjugate as defined in any one of claims 1 to 14 for the manufacture of a medicament for treating gastrointestinal tumours.

16. The use of a conjugate as defined in any one of claims 1 to 14 for the manufacture of a medicament for treating colorectal tumours.

17. A conjugate which comprises a toxin moiety and a target cell binding moiety which is selective for gastrointestinal tumours and wherein the target cell binding moiety comprises C242 antibody or a target cell binding moiety which has substantially the same cell binding properties.

18. A conjugate as claimed in claim 17 wherein the target cell binding moiety comprises C242 antibody or a fragment thereof.

19. A conjugate as claimed in claim 17 wherein the toxin moiety comprises recombinant ricin A.

20. A conjugate as claimed in claim 17, 18 or 19 wherein the target cell binding moiety and the toxin moiety are coupled by a linker of the formula -S-X-CO-, wherein X is a straight chain (1-6C)alkyl group, optionally substituted by one or two substituents selected from (1-6C)alkyl, phenyl and (1-4C)alkylphenyl; or an (1-4C)alkylphenyl group, in which the alkyl moiety is optionally substituted by one or two substituents selected from (1-6C)alkyl, phenyl and (1-4C)alkylphenyl; and wherein the phenyl ring may bear a substituent selected from halogen, (1-4C)alkyl and (1-4)alkoxy.

21. A conjugate as claimed in claim 20 wherein the linker comprises the radical -S-CH(CH₃)CH₂CO-.

22. A conjugate as claimed in any one of claims 17 to 21 wherein the target cell binding moiety has CDR regions with sequences substantially as follows:
light chain 
CDR1: ArgSerSerLysSerLeuLeuHisSerAsnGlyAsnThrTyrLeuTyr
CDR2: ArgMetSerAsnLeuValSer
 CDR3: LeuGlnHisLeuGluTyrProPheThr Heavy Chain 
CDR1: TyrThrGlyMetAsn
CDR2: TrpIleAspThrThrThrGlyGluProThrTyrAlaGluAspPheLysGly
CDR3: ArgGlyProTyrAsnTrpTyrPheAspVal
 the CDR regions being arranged such that the target cell binding moiety has substantially the same cell binding properties as C242 antibody.

23. A conjugate as claimed in claim 17 which comprises C242 antibody and recombinant ricin A, linked from an amino group on C242 to a thiol of a cysteine residue in ricin A by a 3-mercaptobutyric acid diradical (-CO.CH₂.CH(CH₃)-S-).

24. A conjugate which comprises a toxin moiety and a target cell binding moiety which is specific for a gastrointestinal tumour epitope.

25. A conjugate as claimed in claim 24 which is specific for the epitope to which C242 antibody binds.

26. A pharmaceutical composition for treating gastrointestinal tumours which comprises a conjugate as defined in any one of claims 17 to 25 in association with a pharmaceutically acceptable diluent or carrier.

## Patentansprüche (Patentansprüche für folgende(n) Vertragsstaat(en): AT, BE, CH, DE, DK, FR, GB, IT, LI, LU, MC, NL, PT, SE)

1. Konjugat, das eine Toxin-Komponente und eine Zielzell-Bindungskomponente aufweist, die selektiv für gastrointestinale Tumoren ist, wobei die Zielzell-Bindungskomponente den C242-Antikörper oder eine Zielzell-Bindungskomponente mit im wesentlichen den gleichen Zellbindungseigenschaften umfaßt.

2. Konjugat nach Anspruch 1, wobei die Zielzell-Bindungskomponente den C242-Antikörper oder ein Fragment davon umfaßt.

3. Konjugat nach Anspruch 1 oder 2, wobei die Toxin-Komponente rekombiniertes Ricin A umfaßt.

4. Konjugat nach Anspruch 1, 2 oder 3, wobei die Zielzell-Bindungskomponente und die Toxin-Komponente über einen bifunktionellen Linker gekuppelt sind.

5. Konjugat nach einem der vorhergehenden Ansprüche, wobei die Zielzell-Bindungskomponente und die Toxin-Komponente über eine Disulfid- oder Thioether-Bindung gekuppelt sind.

6. Konjugat nach einem der vorhergehenden Ansprüche, wobei die Zielzell-Bindungskomponente und die Toxin-Komponente über einen Linker der Formel -S-X-CO-gekuppelt sind, wobei bedeuten: X eine geradkettige (1-6C)Alkyl-Gruppe, die gegebenenfalls mit einem oder zwei Substituenten substituiert ist, die unter (1-6C)Alkyl, Phenyl und (1-4C)Alkylphenyl ausgewählt sind, oder eine (1-4C)Alkylphenyl-Gruppe, wobei der Alkyl-Rest gegebenenfalls mit einem oder zwei Substituenten substituiert ist, die unter (1-6C)Alkyl, Phenyl und (1-4C)Alkylphenyl ausgewählt sind, und wobei der Phenyl-Ring einen unter Halogen, (1-4C)Alkyl und (1-4C)Alkoxy ausgewählten Substituenten tragen kann.

7. Konjugat nach Anspruch 6, wobei X eine Methylen-, Ethylen-, Propylen- oder Butylen-Gruppe umfaßt, die mit einer oder zwei unter Methyl, Ethyl, Propyl und Butyl ausgewählten Gruppen substituiert ist.

8. Konjugat nach Anspruch 6, wobei der Linker den Rest -S-CH(CH₃)CH₂CO- umfaßt.

9. Konjugat nach einem der vorhergehenden Ansprüche, wobei die Zielzell-Bindungskomponente CDR-Regionen mit im wesentlichen den folgenden Sequenzen aufweist:
leichte Kette 
CDR1: ArgSerSerLysSerLeuLeuHisSerAsnGlyAsnThrTyrLeuTyr
CDR2: ArgMetSerAsnLeuValSer
CDR3: LeuGlnHisLeuGluTyrProPheThr
 schwere Kette 
CDR1: TyrThrGlyMetAsn
CDR2: TrpIleAspThrThrThrGlyGluProThrTyrAlaGluAspPhe
LysGly
CDR3: ArgGlyProTyrAsnTrpTyrPheAspVal,
 wobei die CDR-Regionen so angeordnet sind, daß die Zielzell-Bindungskomponente im wesentlichen die gleichen Zellbindungseigenschaften wie der C242-Antikörper hat.

10. Konjugat der Formel: 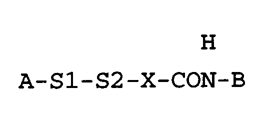 wobei:
A rekombiniertes Ricin A umfaßt und B eine Zielzell-Bindungskomponente umfaßt, die den C242-Antikörper umfaßt,
S1 ein an dem rekombinierten Ricin A vorhandenes Schwefelatom ist,
das NH sich am C242-Antikörper befindet,
S2 eine Schwefelatom-Gruppe ist und
X eine geradkettige (1-6C)Alkyl-Gruppe, die gegebenenfalls mit einem oder zwei Substituenten substituiert ist, die unter (1-6C)Alkyl, Phenyl und (1-4C)Alkylphenyl ausgewählt sind, oder eine (1-4C)Alkylphenyl-Gruppe, wobei der Alkyl-Rest gegebenenfalls mit einem oder zwei Substituenten substituiert ist, die unter (1-6C)Alkyl, Phenyl und (1-4C)Alkylphenyl ausgewählt sind, und wobei der Phenyl-Ring einen unter Halogen, (1-4C)Alkyl und (1-4C)Alkoxy ausgewählten Substituenten tragen kann, ist.

11. Konjugat, das den C242-Antikörper und rekombiniertes Ricin A aufweist, die durch eine Amino-Gruppe an C242 an ein Thiol eines Cystein-Restes in Ricin A durch einen 3-Mercaptobuttersäure-Direst (-CO.CH₂.CH(CH₃)-S-) gebunden sind.

12. Konjugat, das rekombiniertes Ricin A und eine Zielzell-Bindungskomponente mit mindestens einer der CDR-Regionen mit im wesentlichen den folgenden Sequenzen aufweist:
leichte Kette 
CDR1: ArgSerSerLysSerLeuLeuHisSerAsnGlyAsnThrTyrLeuTyr
CDR2: ArgMetSerAsnLeuValSer
CDR3: LeuGlnHisLeuGluTyrProPheThr
 schwere Kette 
CDR1: TyrThrGlyMetAsn
CDR2: TrpIleAspThrThrThrGlyGluProThrTyrAlaGluAspPhe
LysGly
CDR3: ArgGlyProTyrAsnTrpTyrPheAspVal,
 wobei die Anzahl derartiger CDRs und ihre Anordnung derart ist, daß die Zielzell-Bindungskomponente im wesentlichen die gleichen Zellbindungseigenschaften wie der C242-Antikörper hat.

13. Konjugat, das eine Toxin-Komponente und eine Zielzell-Bindungskomponente, die spezifisch für das Epitop eines gastrointestinalen Tumors ist, aufweist.

14. Konjugat nach Anspruch 13, wobei die Zielzell-Bindungskomponente spezifisch für das Epitop ist, an das der C242-Antikörper bindet.

15. Verfahren zur Herstellung eines Konjugats nach Anspruch 1, bei dem:
(a) für solche Konjugate, bei denen die Zielzell-Bindungskomponente direkt an die Toxin-Komponente gekuppelt ist, die Toxin-Komponente mit der Zielzell-Bindungskomponente umgesetzt wird, oder
(b) für solche Konjugate, bei denen die Zielzell-Bindungskomponente über einen Linker an die Toxin-Komponente gekuppelt ist, die mit dem Linker derivatisierte Zielzell-Bindungskomponente mit der Toxin-Komponente umgesetzt wird, oder die mit dem Linker derivatisierte Toxin-Komponente mit der Zielzell-Bindungskomponente umgesetzt wird.

16. Pharmazeutische Zusammensetzung, die ein in einem der vorhergehenden Ansprüche definiertes Konjugat in Verbindung mit einem pharmazeutisch akzeptablen Verdünnungsmittel oder Trägermittel enthält.

17. Verwendung eines Konjugats nach einem der Ansprüche 1 bis 14 zur Herstellung eines Arzneimittels zur Behandlung von gastrointestinalen Tumoren.

18. Verwendung eines Konjugats nach einem der Ansprüche 1 bis 14 zur Herstellung eines Arzneimittels zur Behandlung von kolorektalen Tumoren.

## Patentansprüche (Patentansprüche für folgende(n) Vertragsstaat(en): ES, GR)

1. Verfahren zur Herstellung eines Konjugats, wobei das Konjugat eine Toxin-Komponente und eine Zielzell-Bindungskomponente aufweist, die selektiv für gastrointestinale Tumoren ist, wobei die Zielzell-Bindungskomponente den C242-Antikörper oder eine Zielzell-Bindungskomponente, die im wesentlichen die gleichen Zellbindungseigenschaften hat, umfaßt, bei dem:
(a) für solche Konjugate, bei denen die Zielzell-Bindungskomponente direkt an die Toxin-Komponente gekuppelt ist, die Toxin-Komponente mit der Zielzell-Bindungskomponente umgesetzt wird, oder
(b) für solche Konjugate, bei denen die Zielzell-Bindungskomponente über einen Linker an die Toxin-Komponente gekuppelt ist, die mit dem Linker derivatisierte Zielzell-Bindungskomponente mit der Toxin-Komponente umgesetzt wird, oder die mit dem Linker derivatisierte Toxin-Komponente mit der Zielzell-Bindungskomponente umgesetzt wird.

2. Verfahren nach Anspruch 1, wobei die Zielzell-Bindungskomponente den C242-Antikörper oder ein Fragment davon umfaßt.

3. Verfahren nach Anspruch 1 oder 2, wobei die Toxin-Komponente rekombiniertes Ricin A umfaßt.

4. Verfahren nach Anspruch 1, 2 oder 3, wobei die Zielzell-Bindungskomponente und die Toxin-Komponente über einen bifunktionellen Linker gekuppelt sind.

5. Verfahren nach einem der vorhergehenden Ansprüche, wobei die Zielzell-Bindungskomponente und die Toxin-Komponente über eine Disulfid- oder Thioether-Bindung gekuppelt sind.

6. Verfahren nach einem der vorhergehenden Ansprüche, wobei die Zielzell-Bindungskomponente und die Toxin-Komponente über einen Linker der Formel -S-X-CO-gekuppelt sind, wobei X eine geradkettige (1-6C)Alkyl-Gruppe, die gegebenenfalls mit einem oder zwei Substituenten substituiert ist, die unter (1-6C)Alkyl, Phenyl und (1-4C)Alkylphenyl ausgewählt sind, oder eine (1-4C)Alkylphenyl-Gruppe, wobei der Alkyl-Rest gegebenenfalls mit einem oder zwei Substituenten substituiert ist, die unter (1-6C)Alkyl, Phenyl und (1-4C)Alkylphenyl ausgewählt sind, und wobei der Phenyl-Ring einen unter Halogen, (1-4C)Alkyl und (1-4C)Alkoxy ausgewählten Substituenten tragen kann, ist.

7. Verfahren nach Anspruch 6, wobei X eine Methylen-, Ethylen-, Propylen- oder Butylen-Gruppe umfaßt, die mit einer oder zwei unter Methyl, Ethyl, Propyl und Butyl ausgewählten Gruppen substituiert ist.

8. Verfahren nach Anspruch 6, wobei der Linker den Rest -S-CH(CH₃)CH₂CO- umfaßt.

9. Verfahren nach einem der vorhergehenden Ansprüche, wobei die Zielzell-Bindungskomponente CDR-Regionen mit im wesentlichen den folgenden Sequenzen aufweist:
leichte Kette 
CDR1: ArgSerSerLysSerLeuLeuHisSerAsnGlyAsnThrTyrLeuTyr
CDR2: ArgMetSerAsnLeuValSer
CDR3: LeuGlnHisLeuGluTyrProPheThr
 schwere Kette 
CDR1: TyrThrGlyMetAsn
CDR2: TrpIleAspThrThrThrGlyGluProThrTyrAlaGluAspPhe
LysGly
CDR3: ArgGlyProTyrAsnTrpTyrPheAspVal,
 wobei die CDR-Regionen so angeordnet sind, daß die Zielzell-Bindungskomponente im wesentlichen die gleichen Zellbindungseigenschaften wie der C242-Antikörper hat.

10. Verfahren zur Herstellung eines Konjugats, wobei das Konjugat die folgende Formel hat: 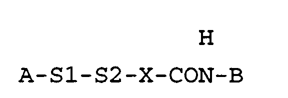 wobei:
A rekombiniertes Ricin A umfaßt und B eine Zielzell-Bindungskomponente umfaßt, die den C242-Antikörper umfaßt,
S1 ein an dem rekombinierten Ricin A vorhandenes Schwefelatom ist,
das NH sich am C242-Antikörper befindet,
S2 eine Schwefelatom-Gruppe ist und
X eine geradkettige (1-6C)Alkyl-Gruppe, die gegebenenfalls mit einem oder zwei Substituenten substituiert ist, die unter (1-6C)Alkyl, Phenyl und (1-4C)Alkylphenyl ausgewählt sind, oder eine (1-4C)Alkylphenyl-Gruppe, wobei der Alkyl-Rest gegebenenfalls mit einem oder zwei Substituenten substituiert ist, die unter (1-6C)Alkyl, Phenyl und (1-4C)Alkylphenyl ausgewählt sind, und wobei der Phenyl-Ring einen unter Halogen, (1-4C)Alkyl und (1-4C)Alkoxy ausgewählten Substituenten tragen kann, ist, bei dem:
ein Derivat der Zielzell-Bindungskomponente der Formel: 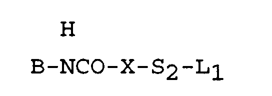 wobei B, X und S₂ wie oben definiert sind und L₁ eine austauschbare Gruppe ist, mit rekombiniertem Ricin A untersetzt wird.

11. Verfahren zur Herstellung eines Konjugats, das den C242-Antikörper und rekombiniertes Ricin A aufweist, die über eine Amino-Gruppe an C242 an ein Thiol eines Cystein-Restes in Ricin A durch einen 3-Mercaptobuttersäure-Direst (-CO.CH₂.CH(CH₃)-S-) gebunden sind, bei dem ein Derivat der Formel: 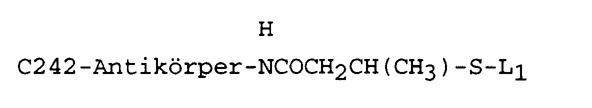 wobei das NH sich an dem C242-Antikörper befindet und L₁ eine Pyridyl-Gruppe darstellt, mit Ricin A umgesetzt wird.

12. Verfahren zur Herstellung eines Konjugats, wobei das Konjugat rekombiniertes Ricin A und eine Zielzell-Bindungskomponente mit mindestens einer der CDR-Regionen mit im wesentlichen den folgenden Sequenzen aufweist:
leichte Kette 
CDR1: ArgSerSerLysSerLeuLeuHisSerAsnGlyAsnThrTyrLeuTyr
CDR2: ArgMetSerAsnLeuValSer
CDR3: LeuGlnHisLeuGluTyrProPheThr
 schwere Kette 
CDR1: TyrThrGlyMetAsn
CDR2: TrpIleAspThrThrThrGlyGluProThrTyrAlaGluAspPhe
LysGly
CDR3: ArgGlyProTyrAsnTrpTyrPheAspVal,
 wobei die Anzahl derartiger CDRs und ihre Anordnung derart ist, daß die Zielzell-Bindungskomponente im wesentlichen die gleichen Zellbindungseigenschaften wie der C242-Antikörper hat, bei dem:
(a) für solche Konjugate, bei denen die Zielzell-Bindungskomponente direkt an die Toxin-Komponente gekuppelt ist, die Toxin-Komponente mit der Zielzell-Bindungskomponente umgesetzt wird, oder
(b) für solche Konjugate, bei denen die Zielzell-Bindungskomponente über einen Linker an die Toxin-Komponente gekuppelt ist, die mit dem Linker derivatisierte Zielzell-Bindungskomponente mit der Toxin-Komponente umgesetzt wird, oder die mit dem Linker derivatisierte Toxin-Komponente mit der Zielzell-Bindungskomponente umgesetzt wird.

13. Verfahren zur Herstellung eines Konjugats, wobei das Konjugat eine Toxin-Komponente und eine Zielzell-Bindungskomponente, die für das Epitop eines gastrointestinalen Tumors spezifisch ist, aufweist, bei dem:
(a) für solche Konjugate, bei denen die Zielzell-Bindungskomponente direkt an die Toxin-Komponente gekuppelt ist, die Toxin-Komponente mit der Zielzell-Bindungskomponente umgesetzt wird, oder
(b) für solche Konjugate, bei denen die Zielzell-Bindungskomponente über einen Linker an die Toxin-Komponente gekuppelt ist, die mit dem Linker derivatisierte Zielzell-Bindungskomponente mit der Toxin-Komponente umgesetzt wird, oder die mit dem Linker derivatisierte Toxin-Komponente mit der Zielzell-Bindungskomponente umgesetzt wird.

14. Verfahren nach Anspruch 13, wobei die Zielzell-Bindungskomponente spezifisch für das Epitop ist, an das der C242-Antikörper bindet.

15. Verwendung eines in einem der Ansprüche 1 bis 14 definierten Konjugats zur Herstellung eines Arzneimittels zur Behandlung von gastrointestinalen Tumoren.

16. Verwendung eines in einem der Ansprüche 1 bis 14 definierten Konjugats zur Herstellung eines Arzneimittels zur Behandlung von kolorektalen Tumoren.

17. Konjugat, das eine Toxin-Komponente und eine Zielzell-Bindungskomponente aufweist, die selektiv für gastrointestinale Tumoren ist, wobei die Zielzell-Bindungskomponente den C242-Antikörper oder eine Zielzell-Bindungskomponente mit im wesentlichen den gleichen Zellbindungseigenschaften umfaßt.

18. Konjugat nach Anspruch 17, wobei die Zielzell-Bindungskomponente den C242-Antikörper oder ein Fragment davon umfaßt.

19. Konjugat nach Anspruch 17, wobei die Toxin-Komponente rekombiniertes Ricin A umfaßt.

20. Konjugat nach Anspruch 17, 18 oder 19, wobei die Zielzell-Bindungskomponente und die Toxin-Komponente über einen Linker der Formel -S-X-CO- gekuppelt sind, wobei bedeuten: X eine geradkettige (1-6C)Alkyl-Gruppe, die gegebenenfalls mit einem oder zwei Substituenten substituiert ist, die unter (1-6C)Alkyl, Phenyl und (1-4C)Alkylphenyl ausgewählt sind, oder eine (1-4C)Alkylphenyl-Gruppe, wobei der Alkyl-Rest gegebenenfalls mit einem oder zwei Substituenten substituiert ist, die unter (1-6C)Alkyl, Phenyl und (1-4)Alkylphenyl ausgewählt sind, und wobei der Phenyl-Ring einen unter Halogen, (1-4C)Alkyl und (1-4C)Alkoxy ausgewählten Substituenten tragen kann.

21. Konjugat nach Anspruch 20, wobei der Linker den Rest -S-CH(CH₃)CH₂CO- umfaßt.

22. Konjugat nach einem der Ansprüche 17 bis 21, wobei die Zielzell-Bindungskomponente CDR-Regionen mit im wesentlichen den folgenden Sequenzen aufweist:
leichte Kette: 
CDR1: ArgSerSerLysSerLeuLeuHisSerAsnGlyAsnThrTyrLeuTyr
CDR2: ArgMetSerAsnLeuValSer
CDR3: LeuGlnHisLeuGluTyrProPheThr
 schwere Kette: 
CDR1: TyrThrGlyMetAsn
CDR2: TrpIleAspThrThrThrGlyGluProThrTyrAlaGluAspPhe-
LysGly
CDR3: ArgGlyProTyrAsnTrpTyrPheAspVal
 wobei die CDR-Regionen so angeordnet sind, daß die Zielzell-Bindungskomponente im wesentlichen die gleichen Zellbindungseigenschaften wie der C242-Antikörper hat.

23. Konjugat nach Anspruch 17, das den C242-Antikörper und rekombinierte Ricin A aufweist, die durch eine Amino-Gruppe an C242 an ein Thiol eines Cystein-Restes in Ricin A durch einen 3-Mercaptobuttersäure-Direst (-CO.CH₂.CH(CH₃)-S-) gebunden sind.

24. Konjugat, das eine Toxin-Komponente und eine Zielzell-Bindungskomponente, die spezifische für das Epitop eines gastrointestinalen Tumors ist, aufweist.

25. Konjugat nach Anspruch 24, das spezifisch für das Epitop ist, an das der C242-Antikörper bindet.

26. Pharmazeutische Zusammensetzung zu Behandlung gastrointestinaler Tumoren, die ein in einem der Ansprüche 17 bis 25 definiertes Konjugat in Verbindung mit einem pharmazeutisch akzeptablen Verdünnungsmittel und Trägermittel enthält.

## Revendications (Revendications pour l'(les) Etat(s) contractant(s) suivant(s): AT, BE, CH, DE, DK, FR, GB, IT, LI, LU, MC, NL, PT, SE)

1. Conjugué qui comprend un groupement consistant en une toxine et un groupement de liaison à une cellule cible qui présente une sélectivité vis-à-vis de tumeurs gastro-intestinales, et dans lequel le groupement de liaison à la cellule cible comprend un anticorps C242 ou un groupement de liaison à une cellule cible qui présente des propriétés pratiquement identiques de liaison aux cellules.

2. Conjugué suivant la revendication 1, dans lequel le groupement de liaison à une cellule cible comprend l'anticorps C242 ou un de ses fragments.

3. Conjugué suivant la revendication 1 ou 2, dans lequel le groupement consistant en une toxine comprend la ricine A recombinante.

4. Conjugué suivant la revendication 1, 2 ou 3, dans lequel le groupement de liaison à une cellule cible et le groupement consistant en une toxine sont couplés par l'intermédiaire d'un agent de liaision bifonctionnel.

5. Conjugué suivant l'une quelconque des revendications précédentes, dans lequel le groupement de liaison à une cellule cible et le groupement consistant en une toxine sont couplés par l'intermédiaire d'une liaison disulfure ou thioéther.

6. Conjugué suivant l'une quelconque des revendications précédentes, dans lequel le groupement de liaison à une cellule cible et le groupement consistant en une toxine sont couplés par un agent de liaison de formule -S-X-CO-, dans laquelle X représente un groupe alkyle en C₁ à C₆ à chaîne droite, facultativement substitué par un ou deux substituants choisis entre des substituants alkyle en C₁ à C₆, phényle et (alkyle en C₁ à C₄)phényle ; ou un groupe (alkyle en C₁ à C₄)phényle dans lequel le groupement alkyle est facultativement substitué par un ou deux substituants choisis entre des substituants alkyle en C₁ à C₆, phényle et (alkyle en C₁ à C₄)phényle ; et dans lequel le noyau phényle peut porter un substituant choisi entre des substituants halogéno, alkyle en C₁ à C₄ et alkoxy en C₁ à C₄.

7. Conjugué suivant la revendication 6, dans lequel X comprend un groupe méthylène, éthylène, propylène ou butylène substitué par un ou deux groupes choisis entre les groupes méthyle, éthyle, propyle et butyle.

8. Conjugué suivant la revendication 6, dans lequel l'agent de liaison comprend le radical -S-CH(CH₃)CH₂CO-.

9. Conjugué suivant l'une quelconque des revendications précédentes, dans lequel le groupement de liaison à une cellule cible comporte des régions CDR avec des séquences consistant essentiellement en les suivantes :
**Chaîne légère** 
CDR1: ArgSerSerLysSerLeuLeuHisSerAsnGlyAsnThrTyrLeuTyr
CDR2: ArgMetSerAsnLeuValSer
CDR3: LeuGlnHisLeuGluTyrProPheThr
 **Chaîne lourde** 
CDR1: TyrThrGlyMetAsn
CDR2: TrpIleAspThrThrThrGlyGluProThrTyrAlaGluAspPheLysGly
CDR3: ArgGlyProTyrAsnTrpTyrPheAspVal
 les régions CDR étant disposées de telle sorte que le groupement de liaison à une cellule cible présente des propriétés de liaison aux cellules pratiquement identiques à celles de l'anticorps C242.

10. Conjugué qui répond à la formule : 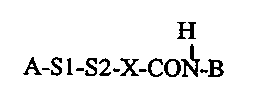 dans laquelle
A comprend la ricine A recombinante et B comprend un groupement de liaison à une cellule cible, qui comprend l'anticorps C242 ;
S1 représente un atome de soufre présent sur la ricine A recombinante ;
le groupe NH est présent sur l'anticorps C242 ;
S2 représente un atome de soufre et X représente un groupe alkyle en C₁ à C₆ à chaîne droite, facultativement substitué par un ou deux substituants choisis entre des substituants alkyle en C₁ à C₆, phényle et (alkyle en C₁ à C₄)phényle ; ou un groupe (alkyle en C₁ à C₄)phényle, dans lequel le groupement alkyle est facultativement substitué par un ou deux substituants choisis entre des substituants alkyle en C₁ à C₆, phényle et (alkyle en C₁ à C₄)phényle ; et dans lequel le noyau phényle peut porter un substituant choisi entre des substituants halogéno, alkyle en C₁ à C₄ et alkoxy en C₁ à C₄.

11. Conjugué qui comprend l'anticorps C242 et la ricine A recombinante, liés d'un groupe amino sur l'anticorps C242 à un groupe thiol d'un résidu cystéine dans la ricine A par un diradical acide 3-mercaptobutyrique (-CO.CH2.CH(CH3)-S-).

12. Conjugué qui comprend la ricine A recombinante et un groupement de liaison à une cellule cible comportant au moins une des régions CDR avec des séquences consistant essentiellement en les suivantes :
**Chaîne légère** 
CDR1: ArgSerSerLysSerLeuLeuHisSerAsnGlyAsnThrTyrLeuTyr
CDR2: ArgMetSerAsnLeu ValSer
CDR3: LeuGlnHisLeuGluTyrProPheThr
 **Chaîne lourde** 
CDR1: TyrThrGlyMetAsn
CDR2: TrpIleAspThrThrThrGlyGluProThrTyrAlaGluAspPheLysGly
CDR3: ArgGlyProTyrAsnTrpTyrPheAspVal
 le nombre de ces régions CDR et leur disposition étant tels que le groupement de liaison à une cellule cible ait des propriétés de liaison aux cellules pratiquement identiques à celles de l'anticorps C242.

13. Conjugué qui comprend un groupement consistant en une toxine et un groupement de liaison à une cellule cible qui est spécifique d'un épitope de tumeur gastro-intestinale.

14. Conjugué suivant la revendication 13, dans lequel le groupement de liaison à une cellule cible est spécifique de l'épitope auquel se lie l'anticorps C242.

15. Procédé pour la préparation d'un conjugué suivant la revendication 1, procédé qui comprend :
(a) pour les conjugués dans lesquels le groupement de liaison à une cellule cible est couplé directement au groupement consistant en une toxine, la réaction du groupement consistant en une toxine avec le groupement de liaison à une cellule cible ; ou
(b) pour les conjugués dans lesquels le groupement de liaison à une cellule cible est couplé au groupement consistant en une toxine par un agent de liaison , la réaction du groupement de liaison à une cellule cible transformé en dérivé au moyen de l'agent de liaison avec le groupement consistant en une toxine, ou la réaction du groupement consistant en une toxine transformé en dérivé au moyen de l'agent de liaison avec le groupement de liaison à une cellule cible.

16. Composition pharmaceutique qui comprend un conjugué répondant à la définition suivant l'une quelconque des revendications précédentes, en association avec un diluant ou support pharmaceutiquement acceptable.

17. Utilisation d'un conjugué suivant l'une quelconque des revendications 1 à 14 pour la production d'un médicament destiné au traitement de tumeurs gastro-intestinales.

18. Utilisation d'un conjugué suivant l'une quelconque des revendications 1 à 14, pour la production d'un médicament destiné au traitement de tumeurs colorectales.

## Revendications (Revendications pour l'(les) Etat(s) contractant(s) suivant(s): ES, GR)

1. Procédé pour la préparation d'un conjugué, dans lequel le conjugué comprend un groupement consistant en une toxine et un groupement de liaison à une cellule cible qui est sélectif vis-à-vis de tumeurs gastro-intestinales, et dans lequel le groupement de liaison à une cellule cible comprend l'anticorps C242 ou un groupement de liaison à une cellule cible qui possède des propriétés de liaison aux cellules pratiquement identiques ;
procédé qui comprend :
(a) pour les conjugués dans lesquels le groupement de liaison à une cellule cible est couplé directement au groupement consistant en une toxine, la réaction du groupement consistant en une toxine avec le groupement de liaison à une cellule cible ; ou
(b) pour les conjugués dans lesquels le groupement de liaison à une cellule cible est couplé au groupement consistant en une toxine par un agent de liaison , la réaction du groupement de liaison à une cellule cible transformé en dérivé au moyen de l'agent de liaison avec le groupement consistant en une toxine, ou la réaction du groupement consistant en une toxine transformé en dérivé au moyen de l'agent de liaison avec le groupement de liaison à une cellule cible.

2. Procédé suivant la revendication 1, dans lequel le groupement de liaison à une cellule cible comprend l'anticorps C242 ou un de ses fragments.

3. Procédé suivant la revendication 1 ou 2, dans lequel le groupement consistant en une toxine comprend la ricine A recombinante.

4. Procédé suivant la revendication 1, 2 ou 3, dans lequel le groupement de liaison à une cellule cible et le groupement consistant en une toxine sont couplés par l'intermédiaire d'un agent de liaison bifonctionnel.

5. Procédé suivant l'une quelconque des revendications précédentes, dans lequel le groupement de liaison à une cellule cible et le groupement consistant en une toxine sont couplés par l'intermédiaire d'une liaison disulfure ou thioéther.

6. Procédé suivant l'une quelconque des revendications précédentes, dans lequel le groupement de liaison à une cellule cible et le groupement consistant en une toxine sont couplés par un agent de liaison de formule -S-X-CO-, dans laquelle X représente un groupe alkyle en C₁ à C₆ à chaîne droite, facultativement substitué par un ou deux substituants choisis entre des substituants alkyle en C₁ à C₆, phényle et (alkyle en C₁ à C₄)phényle ; ou un groupe (alkyle en C₁ à C₄)phényle dans lequel le groupement alkyle est facultativement substitué par un ou deux substituants choisis entre des substituants alkyle en C₁ à C₆, phényle et (alkyle en C₁ à C₄)phényle ; et dans lequel le noyau phényle peut porter un substituant choisi entre des substituants halogéno, alkyle en C₁ à C₄ et alkoxy en C₁ à C₄.

7. Procédé suivant la revendication 6, dans lequel X comprend un groupe méthylène, éthylène, propylène ou butylène substitué par un ou deux groupes choisis entre les groupes méthyle, éthyle, propyle et butyle.

8. Procédé suivant la revendication 6, dans lequel l'agent de liaison comprend le radical -S-CH(CH₃)CH₂CO-.

9. Procédé suivant l'une quelconque des revendications précédentes, dans lequel le groupement de liaison à une cellule cible comporte des régions CDR avec des séquences consistant essentiellement en les séquences suivantes :
**Chaîne légère** 
CDR1: ArgSerSerLysSerLeuLeuHisSerAsnGlyAsnThrTyrLeuTyr
CDR2: ArgMetSerAsnLeuValSer
CDR3: LeuGlnHisLeuGluTyrProPheThr
 **Chaîne lourde** 
CDR1: TyrThrGlyMetAsn
CDR2: TrpIleAspThrThrThrGlyGluProThrTyrAlaGluAspPheLysGly
CDR3: ArgGlyProTyrAsnTrpTyrPheAspVal
 les régions CDR étant disposées de telle sorte que le groupement de liaison à une cellule cible ait des propriétés de liaison aux cellules pratiquement identiques à celles de l'anticorps C242.

10. Procédé pour la préparation d'un conjugué, dans lequel le conjugué répond à la formule : 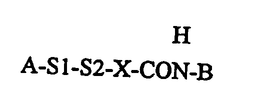 dans laquelle
A comprend la ricine A recombinante et B comprend un groupement de liaison à une cellule cible, qui comprend l'anticorps C242 ;
S1 représente un atome de soufre présent sur la ricine A recombinante ;
le groupe NH est présent sur l'anticorps C242 ;
S2 représente un atome de soufre et X représente un groupe alkyle en C₁ à C₆ à chaîne droite, facultativement substitué par un ou deux substituants choisis entre des substituants alkyle en C₁ à C₆, phényle et (alkyle en C₁ à C₄)phényle ; ou un groupe (alkyle en C₁ à C₄)phényle, dans lequel le groupement alkyle est facultativement substitué par un ou deux substituants choisis entre des substituants alkyle en C₁ à C₆, phényle et (alkyle en C₁ à C₄)phényle ; et dans lequel le noyau phényle peut porter un substituant choisi entre des substituants halogéno, alkyle en C₁ à C₄ et alkoxy en C₁ à C₄ ; procédé qui comprend :
la réaction d'un dérivé du groupement de liaison à une cellule cible, de formule 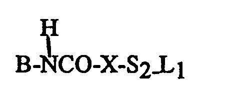 dans laquelle B, X et S₂ répondent aux définitions précitées et L₁ représente un groupe déplaçable, avec la ricine A recombinante.

11. Procédé pour la préparation d'un conjugué qui comprend l'anticorps C242 et la ricine A recombinante, liés d'un groupe amino sur l'anticorps C242 à un groupe thiol d'un résidu cystéine dans la ricine A par un diradical acide 3-mercaptobutyrique (-CO.CH2.CH(CH3)-S-), procédé qui comprend la réaction d'un dérivé de formule 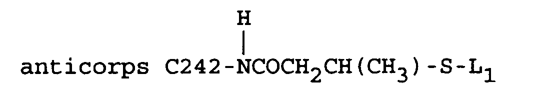 dans laquelle le groupe NH est présent sur l'anticorps C242 et L₁ représente un groupe pyridyle, avec la ricine A.

12. Procédé pour la préparation d'un conjugué, dans lequel le conjugué comprend la ricine A recombinante et un groupement de liaison à une cellule cible comportant au moins une des régions CDR avec des séquences consistant essentiellement en les séquences suivantes :
**Chaîne légère** 
CDR1: ArgSerSerLysSerLeuLeuHisSerAsnGlyAsnThrTyrLeuTyr
CDR2: ArgMetSerAsnLeuValSer
CDR3: LeuGlnHisLeuGluTyrProPheThr
 **Chaîne lourde** 
CDR1: TyrThrGlyMetAsn
CDR2: TrpIleAspThrThrThrGlyGluProThrTyrAlaGluAspPheLysGly
CDR3: ArgGlyProTyrAsnTrpTyrPheAspVal
 le nombre de ces régions CDR et leur disposition étant tels que le groupement de liaison à une cellule cible ait des propriétés de liaison aux cellules pratiquement identiques à celles de l'anticorps C242 ;
procédé qui comprend :
(a) pour les conjugués dans lesquels le groupement de liaison à une cellule cible est couplé directement au groupement consistant en une toxine, la réaction du groupement consistant en une toxine avec le groupement de liaison à une cellule cible ; ou
(b) pour les conjugués dans lesquels le groupement de liaison à une cellule cible est couplé au groupement consistant en une toxine par un agent de liaison , la réaction du groupement de liaison à une cellule cible transformé en dérivé au moyen de l'agent de liaison avec le groupement consistant en une toxine, ou la réaction du groupement consistant en une toxine transformé en dérivé au moyen de l'agent de liaison avec le groupement de liaison à une cellule cible.

13. Procédé pour la préparation d'un conjugué, dans lequel le conjugué comprend un groupement consistant en une toxine et un groupement de liaison à une cellule cible qui est spécifique d'un épitope de tumeurs gastro-intestinales, procédé qui comprend :
(a) pour les conjugués dans lesquels le groupement de liaison à une cellule cible est couplé directement au groupement consistant en une toxine, la réaction du groupement consistant en une toxine avec le groupement de liaison à une cellule cible ; ou
(b) pour les conjugués dans lesquels le groupement de liaison à une cellule cible est couplé au groupement consistant en une toxine par un agent de liaison , la réaction du groupement de liaison à une cellule cible transformé en dérivé au moyen de l'agent de liaison avec le groupement consistant en une toxine, ou la réaction du groupement consistant en une toxine transformé en dérivé au moyen de l'agent de liaison avec le groupement de liaison à une cellule cible.

14. Procédé suivant la revendication 13, dans lequel le groupement de liaison à une cellule cible est spécifique de l'épitope auquel se lie l'anticorps C242.

15. Utilisation d'un conjugué répondant à la définition suivant l'une quelconque des revendications 1 à 14 pour la production d'un médicament destiné au traitement de tumeurs gastro-intestinales.

16. Utilisation d'un conjugué répondant à la définition suivant l'une quelconque des revendications 1 à 14 pour la production d'un médicament destiné au traitement de tumeurs colorectales.

17. Conjugué qui comprend un groupement consistant en une toxine et un groupement de liaison à une cellule cible qui présente une sélectivité vis-à-vis de tumeurs gastro-intestinales et dans lequel le groupement de liaison à la cellule cible comprend un anticorps C242 ou un groupement de liaison à une cellule cible qui présente des propriétés pratiquement identiques de liaison aux cellules.

18. Conjugué suivant la revendication 17, dans lequel le groupement de liaison à une cellule cible comprend l'anticorps C242 ou un de ses fragments.

19. Conjugué suivant la revendication 17, dans lequel le groupement consistant en une toxine comprend la ricine A recombinante.

20. Conjugué suivant la revendication 17, 18 ou 19, dans lequel le groupement de liaison à une cellule cible et le groupement consistant en une toxine sont couplés par un agent de liaison de formule -S-X-CO-, dans laquelle X repésente un groupe alkyle en C₁ à C₆ à chaîne droite, facultativement substitué par un ou deux substituants choisis entre des substituants alkyle en C₁ à C₆, phényle et (alkyle en C₁ à C₄)phényle ; ou un groupe (alkyle en C₁ à C₄)phényle, dans lequel le groupe alkyle est facultativement substitué par un ou deux substituants choisis entre des substituants alkyle en C₁ à C₆, phényle et (alkyle en C₁ à C₄)phényle ; et dans lequel le noyau phényle peut porter un substituant choisi entre des substituants halogéno, alkyle en C₁ à C₄ et alkoxy en C₁ à C₄.

21. Conjugué suivant la revendication 20, dans lequel l'agent de liaison comprend le radical -S-CH(CH₃)CH₂CO-.

22. Conjugué suivant l'une quelconque des revendications 17 à 21, dans lequel le groupement de liaison à une cellule cible comporte des régions CDR et des séquences consistant essentiellement en les suivantes :
**Chaîne légère** 
CDR1: ArgSerSerLysSerLeuLeuHisSerAsnGlyAsnThrTyrLeuTyr

CDR2: ArgMetSerAsnLeuValSer

CDR3: LeuGlnHisLeuGluTyrProPheThr
 **Chaîne lourde** 
CDR1: TyrThrGlyMetAsn
CDR2: TrpIleAspThrThrThrGlyGluProThrTyrAlaGluAspPheLysGly
CDR3: ArgGlyProTyrAsnTrpTyrPheAspVal
 les régions CDR étant disposées de telle sorte que le groupement de liaison à une séquence cible présente des propriétés de liaisons aux cellules pratiquement identiques à celles de l'anticorps C242.

23. Conjugué suivant la revendication 17, qui comprend l'anticorps C242 et la ricine A recombinante, liés d'un groupe amino sur l'anticorps C242 à un groupe thiol d'un résidu cystéine de ricine A par undit radical acide 3-mercapto-butyrique (-CO.CH₂.CH(CH₃)-S-).

24. Conjugué qui comprend un groupement consistant en une toxine et un groupement de liaison à une cellule cible qui est spécifique d'un épitope de tumeur gastro-intestinale.

25. Conjugué suivant la revendication 24, qui est spécifique de l'épitope auquel se lie l'anticorps C242.

26. Composition pharmaceutique destinée au traitement de tumeurs gastro-intestinales, qui comprend un conjugué répondant à la définition suivant l'une quelconque des revendications 17 à 25 en association avec un diluant ou support pharmaceutiquement acceptable.
